# EUROPEAN PATENT APPLICATION

(11) **EP 4 628 507 A1**
(43) Date of publication of application: **08.10.2025**
(21) Application number: 23896820.0
(22) Date of filing: 29.11.2023
(51) Int. Cl.: C07K 16/28, C07K 16/46, C12N 15/62, C12N 5/10, C12N 15/63, A61P 35/00, A61K 39/395, A61K 39/00

(54) **CLDN18.2/4-1BB BINDING PROTEIN AND MEDICAL USE THEREOF**

(30) Priority: 29.11.2022 CN 202211513673
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: HU, Rongting, Shanghai 201210 (CN); GUO, Ne, Shanghai 201210 (CN); LI, Dan, Shanghai 201210 (CN); CHEN, Simeng, Shanghai 201210 (CN); LIN, Yuan, Shanghai 201210 (CN); LIAO, Cheng, Shanghai 201210 (CN)
(74) Representative: Dragotti & Associati S.R.L.
(86) International application number: PCT/CN2023/134972
(87) International publication number: WO 2024/114676

(57) **Abstract**

The present disclosure relates to a CLDN18.2/4-1BB binding protein and a medical use thereof. Specifically, the present disclosure relates to a CLDN18.2/4-1BB binding protein, a 4-1BB binding protein, a CD16A binding protein, methods for treating cancer by using same, and related pharmaceutical uses.

## Description

The present application claims priority to Chinese Patent Application No. CN202211513673.8, filed on November 29, 2022, which is incorporated herein by reference in its entirety.

### TECHNICAL FIELD

The present disclosure relates to the field of biopharmaceutics and particularly to a CLDN18.2/4-1BB-binding protein, a 4-1BB-binding protein, and a CD16A-binding protein, as well as a method for treating a cancer by using same and related pharmaceutical use thereof.

### BACKGROUND

The Claudin protein family is a class of important intercellular tight junction proteins that typically consist of four transmembrane domains and two extracellular loops. Their primary function is to maintain normal molecular exchange between cells. Claudin18, a member of the Claudin protein family, has two splice variants: Claudin18 splice variant 1 (CLDN18.1) and Claudin18 splice variant 2 (CLDN18.2). The two variants differ by only eight amino acids in the first extracellular segment. The two variants differ significantly in physiological distribution: CLDN18.1 is predominantly expressed in normal lung tissue, while CLDN18.2 is primarily expressed in the stomach. In healthy tissue, CLDN18.2 is usually embedded within the gastric mucosa, making it largely inaccessible to antibodies. When cells are malignantly transformed, the disruption of intercellular junctions exposes the antibody-binding epitopes of CLDN18.2, rendering it a tumor-specific target. CLDN18.2 exhibits significant ectopic expression in various epithelial tumors, including gastric cancer, esophageal cancer, pancreatic cancer, etc., providing a biological basis for CLDN18.2-targeting tumor targeted therapies. IMAB362, an anti-CLDN18.2 antibody of the IgG1 subtype developed by Astellas, demonstrated a response rate of 63.2% when used in combination with chemotherapy in patients with advanced metastatic gastric cancer and gastroesophageal cancer, which is superior to historical data on monochemotherapy (2021 ASCO). This demonstrates the clinical value of CLDN18.2 as a tumor-specific antigen.

4-1BB (CD137, TNFRSF9) is a transmembrane protein belonging to the tumor necrosis factor receptor superfamily. 4-1BB is a co-stimulatory molecule expressed on the surface of cells such as CD8⁺ and CD4⁺ T cells, regulatory T cells (Tregs), NK cells, NKT cells, B cells, and neutrophils. On T cells, 4-1BB is not constitutively expressed but is inducibly expressed after T-cell receptor (TCR) activation. 4-1BB is expressed on the cell surface in the form of a monomer or dimer, forms a trimer after binding to its natural ligand 4-1BBL, and signals through the TNFR-associated factors (TRAF)-2 and TRAF-1. Early signaling of 4-1BB involves K-63 polyubiquitination, which activates the nuclear factor (NF-κB) and mitogen-activated protein kinase (MAPK) signaling pathways, resulting in T cell proliferation, maturation, and prolonged survival, cytokine production, etc. Studies have shown that antibody agonists targeting 4-1BB can enhance the antitumor function of T cells in mice (Murillo et al., Clin Cancer Res. 2008; 14(21):6895-906). In many models, 4-1BB-activating antibodies increase the expression of co-stimulatory molecules and induce T cell survival and proliferation, thereby enhancing antitumor immune responses and triggering killing of tumors by immune cells. Existing 4-1BB-activating antibodies are, for example, urelumab, a human IgG4 antibody developed by Bristol Myers Squibb (BMS) (WO2005035584); utomilumab, a human IgG2 antibody developed by Pfizer (Fisher et al., Cancer Immunol. 2012; 61:1721-1733); and ADG106, a human IgG4 antibody developed by Adagene (WO2019037711A1). However, the development of 4-1BB-targeting antitumor antibodies is a challenge. The primary reason is that 4-1BB activates not only T cells within tumors but also T cells in the periphery (e.g., the liver), leading to hepatic inflammation and further to severe hepatic impairment (Todd Bartkowiak et al., Clin Cancer Res; 24(5) March 1, 2018). This makes 4-1BB have a narrow therapeutic window.

Given the excellent tumor specificity and targeting capacity of CLDN18.2, coupled with the limitations of existing 4-1BB-targeting agonistic antibodies in the prior art, the present disclosure provides an anti-4-1BB single-domain antibody of a new structure, and a CLDN18.2/4-1BB bispecific antibody was designed and developed. The bispecific antibody needs to bind to the CLDN18.2 on tumor tissues to activate 4-1BB; therefore, 4-1BB-induced hepatotoxicity is avoided, which allows the antibody to have a larger therapeutic window. When an Fc with an enhanced effector function (e.g., ADCC or ADCP) is used in the anti-CLDN18.2/4-1BB bispecific antibody, the effect is further improved. In addition, the present disclosure further provides an anti-CD16A single-domain antibody of a new structure that forms an anti-CLDN18.2/4-1BB/CD16A trispecific antibody with an anti-CLDN18.2 antibody and the anti-4-1BB single-domain antibody. The anti-CD16A single-domain antibody functions to enhance the ADCC function of the antibody. Both the anti-CLDN18.2/4-1BB bispecific antibody and the anti-CLDN18.2/4-1BB/CD16A trispecific antibody of the present disclosure can avoid ADCC-mediated killing of 4-1BB-positive T cells, which helps improve antitumor effects; therefore, they have good safety profiles and effectiveness. Furthermore, the antibodies of the present disclosure have good pharmacological activity, druggability, and expression levels. In sum, the antibodies of the present disclosure have the potential to become excellent candidate drugs for clinical treatment of tumors.

### SUMMARY

The present disclosure provides a 4-1BB-binding protein, a CD16A-binding protein, a CLDN18.2/4-1BB-binding protein, a CLDN18.2/4-1BB/CD16A-binding protein, a coding nucleic acid therefor, a vector thereof, a host cell thereof, and a pharmaceutical composition thereof, as well as a method for treating or preventing a cancer by using same and related pharmaceutical use thereof.

### 4-1BB-Binding Protein

The present disclosure provides a 4-1BB-binding protein comprising an immunoglobulin single variable domain, wherein the immunoglobulin single variable domain comprises a CDR1, a CDR2, and a CDR3 from the amino acid sequence set forth in any one of SEQ ID NOs: 10 and 18-21, wherein the CDR1, CDR2, and CDR3 are defined according to the Kabat, IMGT, Chothia, AbM, or Contact numbering scheme. The immunoglobulin single variable structure specifically binds to a 4-1BB antigen or a fragment thereof.

In some embodiments, provided is a 4-1BB-binding protein comprising any one of or any combination of the CDR1, CDR2, and CDR3 described above.

In some embodiments, provided is a 4-1BB-binding protein, wherein the amino acid sequences of the CDR1, CDR2, and CDR3 of the immunoglobulin single variable domain are set forth in SEQ ID NOs: 11, 12, and 13 or SEQ ID NOs: 11, 12, and 22, respectively. The CDRs are defined according to the Kabat numbering scheme.

In some embodiments, the immunoglobulin single variable domain in the aforementioned 4-1BB-binding protein is engineered by humanization, affinity maturation, T cell epitope (TCE) removal/decreasing the number of TCEs, reduction of antibody deamidation, and/or reduction of antibody isomerization.

In some embodiments, the immunoglobulin single variable domain is obtained by TCE removal/decreasing the number of TCEs and has one or more changes in one or more CDRs, and the changes cause a reduction in the immunogenicity of the 4-1BB-binding protein.

In some embodiments, the immunoglobulin single variable domain is engineered by humanization. Heavy chain framework regions (FRs) of a human germline template used in the humanization are derived from IGHV3-64*04, IGHV3-23*03, and/or IGHV3-74*01. In some embodiments, FR1 is derived from IGHV3-64*04, FR2 is derived from IGHV3-23*03, and FR3 is derived from IGHV3-74*01.

In some embodiments, the amino acid sequence of the immunoglobulin single variable domain in the aforementioned 4-1BB-binding protein is set forth in any one of SEQ ID NOs: 10 and 18-21 or has at least 80% or at least 90% sequence identity to any one of SEQ ID NOs: 10 and 18-21.

In the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the aforementioned 4-1BB-binding protein comprises or is an antibody that specifically binds to 4-1BB or a fragment thereof, or an antigen-binding fragment thereof. In some specific embodiments, the antibody or the antigen-binding fragment thereof is, for example, a camelid antibody, a chimeric antibody, a humanized antibody, or a fully human antibody or an antigen-binding fragment thereof. In some specific embodiments, the antibody or the antigen-binding fragment thereof is, for example, a recombinant antibody or a fragment thereof.

In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the immunoglobulin single variable domain in the aforementioned 4-1BB-binding protein is a single-domain antibody or VHH.

In some embodiments, the present disclosure provides a 4-1BB-binding protein comprising one or more (e.g., 2, 3, 4, 5, 6, 7, or 8) of the aforementioned immunoglobulin single variable domains, wherein the immunoglobulin single variable domains may be identical or different and may form a dimeric or multimeric molecule.

In some embodiments, the aforementioned 4-1BB-binding protein further comprises an Fc region of a human immunoglobulin; for example, the Fc region is an Fc region of human IgG1, IgG2, or IgG4.

In some embodiments, the Fc comprises a C220 mutation, e.g., C220A.

In some embodiments, the Fc region is an Fc region that increases an effector function, for example, increases antibody-dependent cell-mediated cytotoxicity (ADCC), antibody-dependent cellular phagocytosis (ADCP), and/or complement-dependent cytotoxicity (CDC).

Exemplary IgG1 Fc regions comprise the following substitutions: 239D; 239E; 239K, 241A; 262A; 264D; 264L; 264A; 264S; 265A; 265S; 265V; 296A; 301A; 332E; 239D/332E; 239D/330S/332E; 239D/330L/332E; 298A/333A/334A; 2471/339D; 2471/339Q; 280H/290S; 280H/290S/298D; 280H/290S/298V; 243L/292P/300L; 243L/292P/300L/396L; 243L/292P/300L/305I/396L; 236A/239D/332E; 326A/333A; 326W/333S; 290E/298G/299A; 290N/298G/299A; 290E/298G/299A/326E; or 290N/298G/299A/326E; or any combination of any of the above positions. The mutations are defined according to the EU numbering scheme.

Exemplary IgG1 Fc regions comprise the following substitutions: S239D; S239E; S239K, F241A; V262A; V264D; V264L; V264A; V264S; D265A; D265S; D265V; F296A; Y296A; R301A; I332E; S239D/I332E; S239D/A330S/I332E; S239D/A330L/I332E; S298A/D333A/K334A; P2471/A339D; P247I/A339Q; D280H/K290S; D280H/K290S/S298D; D280H/K290S/S298V; F243L/R292P/Y300L; F243L/R292P/Y300L/P396L; F243L/R292P/Y300/V305I/P396L; G236A/S239D/I332E; K326A/E333A; K326W/E333S; K290E/S298G/T299A; K290N/S298G/T299A; K290E/S298G/T299A/K326E; or K290N/S298G/T299A/K326E, or any combination of any of the above positions.

In some embodiments, the Fc region is an Fc region that weakens an effector function, for example, weakens ADCC, ADCP, and/or CDC. Exemplary Fc regions with a decreased effector function include those that comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

In some specific embodiments, the aforementioned 4-1BB-binding protein comprises an Fc region of human IgG1, wherein the Fc comprises mutations C220A/S267E/L328F, C220A/L234A/L235A/N297A, S267E/L328F, or L234A/L235A/N297A. In some specific embodiments, the aforementioned 4-1BB-binding protein comprises an Fc region of human IgG4, and the Fc region of human IgG4 comprises mutation S228P.

In some embodiments, the aforementioned 4-1BB-binding protein further comprises an Fc region of a human immunoglobulin, wherein the Fc region is set forth in any one of SEQ ID NOs: 14-16 or has at least 80% or at least 90% sequence identity to any one of SEQ ID NOs: 14-16.

In some embodiments, provided is a 4-1BB-binding protein comprising the amino acid sequence set forth in SEQ ID NO: 17 or having at least 80% or at least 90% sequence identity thereto.

In the context of the mutations contained in the Fc region of the present disclosure, "/" represents "and"; for example, "L234A/L235A" represents "L234A and L235A"; that is, the Fc comprises mutations L234A and L235A; the amino acid positions of the mutations in the Fc region of the present disclosure are defined according to the EU numbering scheme.

In some embodiments, the Fc region contained in the aforementioned 4-1BB-binding protein can cause the binding protein to form a dimeric molecule.

In some embodiments, the Fc region contained in the aforementioned 4-1BB-binding protein may extend the *in vivo* half-life of the binding protein.

In some embodiments, in the aforementioned 4-1BB-binding protein, the immunoglobulin single variable domain and the Fc region are linked, either directly or by a linker. The linker may be a non-functional amino acid sequence of 1-20 or more amino acids without a secondary or higher structure. For example, the linker is a flexible linker such as G₄S (SEQ ID NO: 102), GS, GAP, (G₄S)₂ (SEQ ID NO: 103), (G₄S)₃ (SEQ ID NO: 104), (G₄S)₄ (SEQ ID NO: 105), (G₄S)₅ (SEQ ID NO: 106), or ASGS (SEQ ID NO: 107). For example, the linker is (G₄S)₂.

In some embodiments, the 4-1BB-binding protein of the present disclosure is an anti-4-1BB antibody or an antigen-binding fragment thereof, or a conjugate or fusion protein comprising the antibody or antigen-binding fragment.

In some embodiments, the aforementioned 4-1BB-binding protein has an activity selected from the group consisting of at least one of the following:
(a) binding to human 4-1BB or an epitope thereof with a K_{D} value of ≤10⁻⁷;
(b) when not cross-linked by FcyRIIb (i.e., CD32b), exhibiting weak or no activation of the 4-1BB signaling pathway; for example, when at a 100 n antibody concentration and not cross-linked by FcyRIIb, exhibiting a degree of activation that is no more than 10% of the activity when cross-linked by FcγRIIb and at a saturated antibody concentration;
(c) when cross-linked by FcγRIIb, exhibiting relatively strong or strong activation of the 4-1BB signaling pathway, for example, with an EC₅₀ of less than 1 nM;
(d) activating T cells and/or promoting T cell proliferation; and
(e) inhibiting tumor growth;
the activation of the 4-1BB signaling pathway in (b) and (c) is measured, for example, using the 4-1BB/NF-κB luciferase reporter gene assay of Example 2.

In some embodiments, the aforementioned 4-1BB-binding protein of the present disclosure may bind to 4-1BB with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the aforementioned 4-1BB-binding protein of the present disclosure is capable of inhibiting tumor growth by at least about 10%, e.g., at least about 20%, about 30%, about 40%, about 50%, about 60%, about 70%, or about 80%.

In some embodiments, the aforementioned 4-1BB-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 10 and 18-21; the amino acid mutations may be conservative replacements, substitutions, or modifications, and/or deletions or additions that do not affect function; the amino acid mutations may occur in the CDRs and/or FRs.

In some embodiments, provided is an anti-4-1BB antibody or an antigen-binding fragment thereof that binds to or competes with the immunoglobulin single variable domain in the aforementioned 4-1BB-binding protein of the present disclosure for binding to the same epitope.

In some embodiments, provided is an anti-4-1BB antibody or an antigen-binding fragment thereof that blocks the binding of the immunoglobulin single variable domain in the aforementioned 4-1BB-binding protein of the present disclosure to 4-1BB (e.g., human 4-1BB). In some specific embodiments, the anti-4-1BB antibody or the antigen-binding fragment thereof can also activate T cells and/or promote T cell proliferation.

In some embodiments, provided is an anti-4-1BB antibody or an antigen-binding fragment thereof whose binding to 4-1BB (e.g., human 4-1BB) is blocked by the immunoglobulin single variable domain in the aforementioned 4-1BB-binding protein of the present disclosure.

In some embodiments, provided is a protein or molecule comprising any one or more (e.g., 1, 2, 3, 4, 5, 6, 7, 8, 9, or 10) of the immunoglobulin single variable domains in the aforementioned 4-1BB-binding proteins of the present disclosure, wherein the immunoglobulin single variable domains are identical or different. For example, the protein or molecule is a conjugate, and the conjugate may, for example, comprise any detectable label.

### CD 16A-Binding Protein

The present disclosure provides a CD16A-binding protein comprising an immunoglobulin single variable domain, wherein the immunoglobulin single variable domain comprises:
a CDR1, a CDR2, and a CDR3 from the amino acid sequence set forth in any one of SEQ ID NOs: 23 and 35-39, or a CDR1, a CDR2, and a CDR3 from the amino acid sequence set forth in any one of SEQ ID NOs: 24 and 40-43,
wherein the CDR1, CDR2, and CDR3 are defined according to the Kabat, IMGT, Chothia, AbM, or Contact numbering scheme.

In some embodiments, provided is a CD16A-binding protein comprising an immunoglobulin single variable domain, wherein the immunoglobulin single variable domain comprises any one of or any combination of the CDR1, CDR2, and CDR3 described above.

In some embodiments, provided is a CD16A-binding protein comprising an immunoglobulin single variable domain, wherein the immunoglobulin single variable domain comprises a CDR1, a CDR2, and a CDR3, wherein:
the amino acid sequences of the CDR1, CDR2, and CDR3 are set forth in SEQ ID NOs: 25, 26, and 27, respectively,
the amino acid sequences of the CDR1, CDR2, and CDR3 are set forth in SEQ ID NOs: 28, 29, and 30, respectively,
or the amino acid sequences of the CDR1, CDR2, and CDR3 are set forth in SEQ ID NOs: 28, 44, and 30, respectively, wherein the CDR1, CDR2, and CDR3 are CDRs defined according to the Kabat numbering scheme.

In some embodiments, the immunoglobulin single variable domain in the aforementioned CD16A-binding protein is engineered by humanization, affinity maturation, T cell epitope (TCE) removal/decreasing the number of TCEs, reduction of antibody deamidation, and/or reduction of antibody isomerization.

In some embodiments, the immunoglobulin single variable domain is obtained by TCE removal/decreasing the number of TCEs and has one or more changes in one or more CDRs, and the changes cause a reduction in the immunogenicity of the CD16A-binding protein.

In some embodiments, the immunoglobulin single variable domain is engineered by humanization. Heavy chain framework regions (FRs) of a human germline template used in the humanization are derived from IGHV3-23*04 or IGHV3-20*04. In some embodiments, when the parent immunoglobulin single variable domain is SEQ ID NO: 23, the heavy chain framework regions (FRs) of the human germline template used in the humanization are derived from IGHV3-23*04; when the parent immunoglobulin single variable domain is SEQ ID NO: 24, the heavy chain framework regions (FRs) of the human germline template used in the humanization are derived from IGHV3-20*04.

In some embodiments, the amino acid sequence of the immunoglobulin single variable domain in the aforementioned CD16A-binding protein is set forth in any one of SEQ ID NOs: 23 and 35-39 or has at least 80% or at least 90% sequence identity to any one of SEQ ID NOs: 23 and 35-39, or is set forth in any one of SEQ ID NOs: 24 and 40-43 or has at least 80% or at least 90% sequence identity to any one of SEQ ID NOs: 24 and 40-43.

In some embodiments, the immunoglobulin single variable domain in the aforementioned CD16A-binding protein specifically binds to CD16A and does not specifically bind to CD16B.

In some embodiments, the aforementioned CD16A-binding protein comprises or is an antibody that specifically binds to CD16A or an antigen-binding fragment thereof. In some specific embodiments, the antibody or the antigen-binding fragment thereof is, for example, a camelid antibody, a chimeric antibody, a humanized antibody, or a fully human antibody or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the immunoglobulin single variable domain in the aforementioned CD16A-binding protein is a single-domain antibody or VHH.

In some embodiments, the present disclosure provides a CD 16A-binding protein comprising one or more (e.g., 2, 3, 4, 5, 6, 7, or 8) of the aforementioned immunoglobulin single variable domains, wherein the immunoglobulin single variable domains may be identical or different and may form a dimeric or multimeric molecule.

In some embodiments, the aforementioned CD16A-binding protein further comprises an Fc region of a human immunoglobulin; for example, the Fc region is an Fc region of human IgG1, IgG2, or IgG4. In some embodiments, the Fc region may be an Fc region with an enhanced effector function, wherein the effector function is ADCC, ADCP, and/or CDC. For example, the Fc region may comprise a mutation, and exemplary IgG1 Fc regions with an increased effector function comprise the following substitutions or any combination thereof: S239D, S239E, S239K, F241A, V262A, V264D, V264L, V264A, V264S, D265A, D265S, D265V, F296A, Y296A, R301A, I332E, S239D/I332E, S239D/A330S/I332E, S239D/A330L/I332E, S298A/D333A/K334A, P2471/A339D, P247I/A339Q, D280H/K290S, D280H/K290S/S298D, D280H/K290S/S298V, F243L/R292P/Y300L, F243L/R292P/Y300L/P396L, F243L/R292P/Y300L/V305I/P396L, G236A/S239D/I332E, K326A/E333A, K326W/E333S, K290E/S298G/T299A, K290N/S298G/T299A, K290E/S298G/T299A/K326E, or K290N/S298G/T299A/K326E, or any combination of any of the above positions.

In some other embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

The mutations used in the present disclosure to increase or reduce an effector function of the Fc region are well known in the art and are disclosed in, for example, WO2019220369A, WO2021027850A, and WO2020180712A, which are incorporated herein by reference in their entirety.

In some specific embodiments, the Fc region is set forth in any one of SEQ ID NOs: 14-16 or has at least 80% or at least 90% sequence identity to any one of SEQ ID NOs: 14-16.

In some embodiments, the Fc region contained in the aforementioned CD16A-binding protein can cause the binding protein to form a dimeric molecule and extend the *in vivo* half-life of the binding protein.

In some embodiments, in the aforementioned CD16A-binding protein, the immunoglobulin single variable domain and the Fc region are linked, either directly or by a linker. The linker may be a non-functional amino acid sequence of 1-20 or more amino acids without a secondary or higher structure. For example, the linker is a flexible linker such as G₄S, GS, GAP, (G₄S)₂, (G₄S)₃, (G₄S)₄, (G₄S)₅, or ASGS. For example, the linker is (G₄S)₂.

In some embodiments, the CD16A-binding protein of the present disclosure is an anti-CD 16A antibody or an antigen-binding fragment thereof, or a conjugate or fusion protein comprising the antibody or antigen-binding fragment.

In some embodiments, the aforementioned CD16A-binding protein has an activity selected from the group consisting of at least one of the following:
(a) specifically binding to human CD16A or an epitope thereof with a K_{D} value of ≤10⁻⁷;
(b) no binding or weak binding to human CD16B or an epitope thereof, wherein the weak binding is 10% or lower of the specific binding affinity for human CD16A or an epitope thereof;
(c) binding to a CD16A 158V (i.e., CD16A 176V) variant and CD16A 158F (i.e., CD16A 176F) with the same affinity or similar affinities;
(d) activating or enhancing ADCC via NK cells;
(e) activating or enhancing ADCP via macrophages; and
(f) inhibiting tumor growth.

In some embodiments, the aforementioned CD16A-binding protein of the present disclosure may bind to CD16A with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the aforementioned CD16A-binding protein of the present disclosure is capable of inhibiting tumor growth by at least about 10%, e.g., at least about 20%, about 30%, about 40%, about 50%, about 60%, about 70%, or about 80%.

In some embodiments, the aforementioned CD16A-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 23-24 and 35-43; the amino acid mutations may be conservative replacements, substitutions, or modifications, and/or deletions or additions that do not affect function; the amino acid mutations may occur in the CDRs and/or FRs.

In some embodiments, provided is an anti-CD16A antibody or an antigen-binding fragment thereof that binds to or competes with the immunoglobulin single variable domain in the aforementioned CD16A-binding protein of the present disclosure for binding to the same epitope.

In some embodiments, provided is an anti-CD16A antibody or an antigen-binding fragment thereof that blocks the binding of the immunoglobulin single variable domain in the aforementioned CD16A-binding protein of the present disclosure to CD16A (e.g., human CD16A).

In some embodiments, provided is an anti-CD16A antibody or an antigen-binding fragment thereof whose binding to CD16A (e.g., human CD16A) is blocked by the immunoglobulin single variable domain in the aforementioned CD16A-binding protein of the present disclosure.

In some embodiments, provided is a protein or molecule comprising any one or more (e.g., 1, 2, 3, 4, 5, 6, 7, 8, 9, or 10) of the immunoglobulin single variable domains in the aforementioned CD16A-binding proteins of the present disclosure, wherein the immunoglobulin single variable domains are identical or different. For example, the protein or molecule is a conjugate, and the conjugate may, for example, comprise any detectable label.

### CLDN 18.2/4-1BB-Binding Protein

In a first aspect, the present disclosure provides a CLDN18.2/4-1BB-binding protein comprising a first antigen-binding domain that specifically binds to 4-1BB and a second antigen-binding domain that specifically binds to CLDN18.2, and capable of specifically binding to 4-1BB and CLDN18.2 simultaneously or separately.

In some embodiments, when the second antigen-binding domain that specifically binds to CLDN18.2 does not bind to CLDN18.2, the first antigen-binding domain that specifically binds to 4-1BB does not activate 4-1BB signaling, or binds to a 4-1BB antigen but does not activate 4-1BB signaling.

In some embodiments, the CLDN18.2/4-1BB-binding protein has an enhanced effector function; for example, the CLDN18.2/4-1BB-binding protein comprises an Fc region with an enhanced effector function and/or a third antigen-binding domain that specifically binds to CD16A.

In some embodiments, the Fc region with an enhanced effector function has increased binding to FcyR, for example, increased binding to FcyRIIB (CD32B) or increased binding to FcyRIIIA (CD16A). In some embodiments, the Fc region has decreased, reduced, or no binding to FcyRIIIB (CD16B).

In some embodiments, the Fc region with an enhanced effector function has increased binding to C1q.

In some embodiments, the Fc region with an enhanced effector function has a normal or higher glycosylation level than a wild-type Fc region.

In some embodiments, the effector function is antibody-dependent cell-mediated cytotoxicity (ADCC), antibody-dependent cellular phagocytosis (ADCP), or complement-dependent cytotoxicity (CDC).

In a second aspect, the present disclosure provides a CLDN18.2/4-1BB-binding protein comprising a first antigen-binding domain that specifically binds to 4-1BB, a second antigen-binding domain that specifically binds to CLDN18.2, and a third antigen-binding domain that specifically binds to CD16A, and capable of specifically binding to 4-1BB, CLDN18.2, and CD16A simultaneously or separately.

In some embodiments, when the second antigen-binding domain that specifically binds to CLDN18.2 does not bind to CLDN18.2, the first antigen-binding domain that specifically binds to 4-1BB does not activate 4-1BB signaling, or binds to a 4-1BB antigen but does not activate 4-1BB signaling.

In the above embodiments, the first antigen-binding domain that specifically binds to 4-1BB, the second antigen-binding domain that specifically binds to CLDN18.2, and the third antigen-binding domain that specifically binds to CD16A may be independently selected from the group consisting of, for example, a Fab, a Fab', a F(ab')2, an Fv, a Fab-Fv, a Fab-dsFv, a single-domain antibody (e.g., a VH or VL or VHH), and an scFv. For example, the first antigen-binding domain that specifically binds to 4-1BB and the third antigen-binding domain that specifically binds to CD16A are VHHs, and the second antigen-binding domain that specifically binds to CLDN18.2 is a Fab.

### Regarding the first antigen-binding domain that specifically binds to 4-1BB:

In some embodiments, in the CLDN18.2/4-1BB-binding protein, the first antigen-binding domain that specifically binds to 4-1BB comprises an immunoglobulin single variable domain, wherein the immunoglobulin single variable domain comprises a CDR1, a CDR2, and a CDR3 from the amino acid sequence set forth in any one of SEQ ID NOs: 10 and 18-21, wherein the CDR1, CDR2, and CDR3 are defined according to the Kabat, IMGT, Chothia, AbM, or Contact numbering scheme. The immunoglobulin single variable structure specifically binds to a 4-1BB antigen or a fragment thereof.

In some embodiments, the immunoglobulin single variable domain comprises any one of or any combination of the CDR1, CDR2, and CDR3 described above.

In some embodiments, the amino acid sequences of the CDR1, CDR2, and CDR3 of the immunoglobulin single variable domain are set forth in SEQ ID NOs: 11, 12, and 13 or SEQ ID NOs: 11, 12, and 22, respectively. The CDRs are defined according to the Kabat numbering scheme.

In some embodiments, the amino acid sequences of the CDR1, CDR2, and CDR3 of the immunoglobulin single variable domain are GFTFSSYA (SEQ ID NO: 66), INSGGEST (SEQ ID NO: 67), and AKHPLTFTIATMNDYDY (SEQ ID NO: 68), or SEQ ID NOs: 66 and 67 and AKHPLTYTIATMNDYDY (SEQ ID NO: 69), respectively. The CDRs are defined according to the IMGT numbering scheme.

In some embodiments, the amino acid sequences of the CDR1, CDR2, and CDR3 of the immunoglobulin single variable domain are GFTFSSY (SEQ ID NO: 70), NSGGES (SEQ ID NO: 71), and SEQ ID NO: 13, or SEQ ID NOs: 70, 71, and 22, respectively. The CDRs are defined according to the Chothia numbering scheme.

In some embodiments, the amino acid sequences of the CDR1, CDR2, and CDR3 of the immunoglobulin single variable domain are GFTFSSYAMS (SEQ ID NO: 72), DINSGGESTF (SEQ ID NO: 73), and SEQ ID NO: 13, or SEQ ID NOs: 72, 73, and 22, respectively. The CDRs are defined according to the AbM numbering scheme.

In some embodiments, the amino acid sequences of the CDR1, CDR2, and CDR3 of the immunoglobulin single variable domain are SSYAMS (SEQ ID NO: 74), WVSDINSGGESTF (SEQ ID NO: 75), and AKHPLTFTIATMNDYD (SEQ ID NO: 76), or SEQ ID NOs: 74 and 75 and AKHPLTYTIATMNDYD (SEQ ID NO: 77), respectively. The CDRs are defined according to the Contact numbering scheme.

In some embodiments, the immunoglobulin single variable domain is engineered by humanization, affinity maturation, T cell epitope removal/decreasing the number of T cell epitopes, reduction of antibody deamidation, and/or reduction of antibody isomerization. In some embodiments, the immunoglobulin single variable domain is obtained by T cell epitope removal/decreasing the number of T cell epitopes and has one or more changes in one or more CDRs, and the changes cause a reduction in the immunogenicity of the binding protein.

In some embodiments, the immunoglobulin single variable domain is engineered by humanization. Heavy chain framework regions (FRs) of a human germline template used in the humanization are derived from IGHV3-64*04, IGHV3-23*03, and/or IGHV3-74*01. In some embodiments, FR1 is derived from IGHV3-64*04, FR2 is derived from IGHV3-23*03, and FR3 is derived from IGHV3-74*01.

In some embodiments, the amino acid sequence of the immunoglobulin single variable domain is set forth in any one of SEQ ID NOs: 10 and 18-21 or has at least 80% sequence identity to any one of SEQ ID NOs: 10 and 18-21.

In some embodiments, in the CLDN18.2/4-1BB-binding protein, the first antigen-binding domain that specifically binds to 4-1BB comprises the aforementioned 4-1BB-binding protein of the present disclosure or comprises urelumab, utomilumab, ADG106, and the anti-4-1BB antibodies in WO2005035584A, WO2019037711A, US20190055314A, WO2019014328A3, and US20210206867A or antigen-binding fragments thereof. The patents described above are incorporated herein by reference in their entirety.

### Regarding the second antigen-binding domain that specifically binds to CLDN18-2:

In some embodiments, in the CLDN18.2/4-1BB-binding protein, the second antigen-binding domain that specifically binds to CLDN18.2 comprises a heavy chain variable region (VH) and a light chain variable region (VL).

In some embodiments, the VH of the second antigen-binding domain that specifically binds to CLDN18.2 comprises a HCDR1, a HCDR2, and a HCDR3 from the amino acid sequence set forth in SEQ ID NO: 63, and the VL comprises a LCDR1, a LCDR2, and a LCDR3 from the amino acid sequence set forth in SEQ ID NO: 64. The CDRs are defined according to the Kabat, IMGT, Chothia, AbM, or Contact numbering scheme, for example, according to the Kabat numbering scheme.

In some embodiments, the VH of the second antigen-binding domain that specifically binds to CLDN18.2 comprises a HCDR1, a HCDR2, and a HCDR3 set forth in SEQ ID NOs: 57, 58, and 59, and the VL comprises a LCDR1, a LCDR2, and a LCDR3 set forth in SEQ ID NOs: 60, 61, and 62. The CDRs are defined according to the Kabat numbering scheme.

In some embodiments, the VH of the second antigen-binding domain that specifically binds to CLDN18.2 comprises a HCDR1, a HCDR2, and a HCDR3 as follows: GYTFTSYW (SEQ ID NO: 78), IHPNSGST (SEQ ID NO: 79), and ARLKTGNSFDY (SEQ ID NO: 80), and the VL comprises a LCDR1, a LCDR2, and a LCDR3 as follows: QSLLNSGNQKNY (SEQ ID NO: 81), WA, and SEQ ID NO: 62. The CDRs are defined according to the IMGT numbering scheme.

In some embodiments, the VH of the second antigen-binding domain that specifically binds to CLDN18.2 comprises a HCDR1, a HCDR2, and a HCDR3 as follows: GYTFTSY (SEQ ID NO: 82), HPNSGS (SEQ ID NO: 83), and SEQ ID NO: 59, and the VL comprises a LCDR1, a LCDR2, and a LCDR3 set forth in SEQ ID NOs: 60, 61, and 62. The CDRs are defined according to the Chothia numbering scheme.

In some embodiments, the VH of the second antigen-binding domain that specifically binds to CLDN18.2 comprises a HCDR1, a HCDR2, and a HCDR3 as follows: GYTFTSYWMH (SEQ ID NO: 84), MIHPNSGSTN (SEQ ID NO: 85), and SEQ ID NO: 59, and the VL comprises a LCDR1, a LCDR2, and a LCDR3 set forth in SEQ ID NOs: 60, 61, and 62. The CDRs are defined according to the AbM numbering scheme.

In some embodiments, the VH of the second antigen-binding domain that specifically binds to CLDN18.2 comprises a HCDR1, a HCDR2, and a HCDR3 as follows: TSYWMH (SEQ ID NO: 86), WMGMIHPNSGSTN (SEQ ID NO: 87), and ARLKTGNSFD (SEQ ID NO: 88), and the VL comprises a LCDR1, a LCDR2, and a LCDR3 as follows: LNSGNQKNYLTWY (SEQ ID NO: 89), LLIYWASTRE (SEQ ID NO: 90), and QNAYTYPF (SEQ ID NO: 91). The CDRs are defined according to the Contact numbering scheme.

In some embodiments, the VH of the second antigen-binding domain that specifically binds to CLDN18.2 comprises the amino acid sequence set forth in SEQ ID NO: 63 or an amino acid sequence having at least 80% or at least 90% identity thereto, and the VL comprises the amino acid sequence set forth in SEQ ID NO: 64 or an amino acid sequence having at least 80% or at least 90% identity thereto.

In some embodiments, the second antigen-binding domain that specifically binds to CLDN18.2 further comprises an Fc region of a human immunoglobulin; for example, the Fc region is an Fc region of human IgG1, IgG2, or IgG4.

In some embodiments, the second antigen-binding domain that specifically binds to CLDN18.2 comprises a heavy chain and a light chain, wherein the amino acid sequence of the heavy chain is set forth in SEQ ID NO: 45 or has at least 80% or at least 90% identity thereto, and the amino acid sequence of the light chain is set forth in SEQ ID NO: 46 or has at least 80% or at least 90% identity thereto.

The anti-CLDN18.2 antibodies in WO2020200196A are incorporated herein by reference in their entirety, and the antibodies or antigen-binding fragments thereof can all be used as the second antigen-binding domain that specifically binds to CLDN18.2 in the present disclosure. In some other embodiments, the second antigen-binding domain that specifically binds to CLDN18.2 comprises the anti-CLDN18.2 antibodies in WO2021027850A, WO2014146672A, WO2021025177A, WO2016180782A, and WO2021254481A or antigen-binding fragments thereof, and the patents described above are also incorporated herein by reference in their entirety.

### Regarding the third antigen-binding domain that specifically binds to CD16A:

In some embodiments, in the CLDN18.2/4-1BB-binding protein, the third antigen-binding domain that specifically binds to CD16A comprises an immunoglobulin single variable domain, wherein the immunoglobulin single variable domain comprises a CDR1, a CDR2, and a CDR3 from the amino acid sequence set forth in any one of SEQ ID NOs: 23 and 35-39, or a CDR1, a CDR2, and a CDR3 from the amino acid sequence set forth in any one of SEQ ID NOs: 24 and 40-43, wherein the CDR1, CDR2, and CDR3 are defined according to the Kabat, IMGT, Chothia, AbM, or Contact numbering scheme.

In some embodiments, the immunoglobulin single variable domain comprises any one of or any combination of the CDR1, CDR2, and CDR3 described above.

In some embodiments, the amino acid sequences of the CDR1, CDR2, and CDR3 of the immunoglobulin single variable domain are set forth in SEQ ID NOs: 25, 26, and 27, respectively. The CDRs are defined according to the Kabat numbering scheme.

In some embodiments, the amino acid sequences of the CDR1, CDR2, and CDR3 of the immunoglobulin single variable domain are GFAFSTYA (SEQ ID NO: 92), INSDGSST (SEQ ID NO: 93), and AKGWISSPVWGDYVPPV (SEQ ID NO: 94), respectively. The CDRs are defined according to the IMGT numbering scheme.

In some embodiments, the amino acid sequences of the CDR1, CDR2, and CDR3 of the immunoglobulin single variable domain are GFAFSTY (SEQ ID NO: 95), NSDGSS (SEQ ID NO: 96), and SEQ ID NO: 27, respectively. The CDRs are defined according to the Chothia numbering scheme.

In some embodiments, the amino acid sequences of the CDR1, CDR2, and CDR3 of the immunoglobulin single variable domain are GFTFSTYAMY (SEQ ID NO: 97), TINSDGSSTR (SEQ ID NO: 98), and SEQ ID NO: 27, respectively. The CDRs are defined according to the AbM numbering scheme.

In some embodiments, the amino acid sequences of the CDR1, CDR2, and CDR3 of the immunoglobulin single variable domain are STYAMY (SEQ ID NO: 99), WVSTINSDGSSTR (SEQ ID NO: 100), and AKGWISSPVWGDYVPP (SEQ ID NO: 101), respectively. The CDRs are defined according to the Contact numbering scheme.

In some embodiments, the amino acid sequences of the CDR1, CDR2, and CDR3 of the immunoglobulin single variable domain are set forth in SEQ ID NOs: 28, 29, and 30, respectively. The CDRs are defined according to the Kabat numbering scheme. The CDRs are defined according to the Kabat numbering scheme.

In some embodiments, the immunoglobulin single variable domain is engineered by humanization, affinity maturation, T cell epitope removal/decreasing the number of T cell epitopes, reduction of antibody deamidation, and/or reduction of antibody isomerization. In some embodiments, the immunoglobulin single variable domain is obtained by T cell epitope removal/decreasing the number of T cell epitopes and has one or more changes in one or more CDRs, and the changes cause a reduction in the immunogenicity of the binding protein.

In some embodiments, the immunoglobulin single variable domain is engineered by humanization. Heavy chain framework regions (FRs) of a human germline template used in the humanization are derived from IGHV3-23*04 or IGHV3-20*04. In some embodiments, when the parent immunoglobulin single variable domain is SEQ ID NO: 23, the heavy chain framework regions (FRs) of the human germline template used in the humanization are derived from IGHV3-23*04; when the parent immunoglobulin single variable domain is SEQ ID NO: 24, the heavy chain framework regions (FRs) of the human germline template used in the humanization are derived from IGHV3-20*04.

In some embodiments, the amino acid sequence of the immunoglobulin single variable domain is set forth in any one of SEQ ID NOs: 23 and 35-39 or has at least 80% or at least 90% sequence identity thereto, or is set forth in any one of SEQ ID NOs: 24 and 40-43 or has at least 80% or at least 90% sequence identity thereto.

In some embodiments, the third antigen-binding domain that specifically binds to CD16A comprises the aforementioned CD16A-binding protein of the present disclosure or the anti-CD16A antibodies in WO2006125668A, WO2007009065A, and WO2016177846A or antigen-binding fragments thereof. The patents described above are incorporated herein by reference in their entirety.

The CLDN18.2/4-1BB-binding proteins of the present disclosure are provided below for illustrative purposes:
In some embodiments, in the CLDN18.2/4-1BB-binding protein, the first antigen-binding domain that specifically binds to 4-1BB comprises an immunoglobulin single variable domain, wherein the immunoglobulin single variable domain comprises a CDR1, a CDR2, and a CDR3 from the amino acid sequence set forth in any one of SEQ ID NOs: 10 and 18-21, wherein the CDR1, CDR2, and CDR3 are defined according to the Kabat, IMGT, Chothia, AbM, or Contact numbering scheme. In some specific embodiments, according to the Kabat numbering scheme, the amino acid sequences of the CDR1, CDR2, and CDR3 of the immunoglobulin single variable domain are set forth in SEQ ID NOs: 11, 12, and 13 or SEQ ID NOs: 11, 12, and 22, respectively. In some specific embodiments, the amino acid sequence of the immunoglobulin single variable domain is set forth in any one of SEQ ID NOs: 10 and 18-21 or has at least 80% or at least 90% sequence identity to any one of SEQ ID NOs: 10 and 18-21. In some specific embodiments, the immunoglobulin single variable domain is a single-domain antibody or VHH.

In some embodiments, in the CLDN18.2/4-1BB-binding protein, the second antigen-binding domain that specifically binds to CLDN18.2 comprises a VH and a VL. In some specific embodiments, the VH comprises a HCDR1, a HCDR2, and a HCDR3 from the amino acid sequence set forth in SEQ ID NO: 63, and the VL comprises a LCDR1, a LCDR2, and a LCDR3 from the amino acid sequence set forth in SEQ ID NO: 64. The CDRs are defined according to the Kabat, IMGT, Chothia, AbM, or Contact numbering scheme, for example, according to the Kabat numbering scheme. In some specific embodiments, the VH comprises a HCDR1, a HCDR2, and a HCDR3 set forth in SEQ ID NOs: 57, 58, and 59, and the VL comprises a LCDR1, a LCDR2, and a LCDR3 set forth in SEQ ID NOs: 60, 61, and 62. In some specific embodiments, the VH comprises the amino acid sequence set forth in SEQ ID NO: 63 or an amino acid sequence having at least 80% or at least 90% identity thereto, and the VL comprises the amino acid sequence set forth in SEQ ID NO: 64 or an amino acid sequence having at least 80% or at least 90% identity thereto. In some specific embodiments, in the CLDN18.2/4-1BB-binding protein, the second antigen-binding domain that specifically binds to CLDN18.2 comprises a heavy chain and a light chain, wherein the heavy chain comprises the amino acid sequence set forth in SEQ ID NO: 45 or an amino acid sequence having at least 80% or at least 90% identity thereto, and the light chain comprises the amino acid sequence set forth in SEQ ID NO: 46 or an amino acid sequence having at least 80% or at least 90% identity thereto.

Optionally, in some embodiments, the CLDN18.2/4-1BB-binding protein further comprises a third antigen-binding domain that specifically binds to CD16A. In some specific embodiments, the third antigen-binding domain that specifically binds to CD16A comprises an immunoglobulin single variable domain, wherein the immunoglobulin single variable domain comprises a CDR1, a CDR2, and a CDR3 from the amino acid sequence set forth in any one of SEQ ID NOs: 23 and 35-39, or a CDR1, a CDR2, and a CDR3 from the amino acid sequence set forth in any one of SEQ ID NOs: 24 and 40-43, wherein the CDR1, CDR2, and CDR3 are defined according to the Kabat, IMGT, Chothia, AbM, or Contact numbering scheme. In some specific embodiments, according to the Kabat numbering scheme, the amino acid sequences of the CDR1, CDR2, and CDR3 of the immunoglobulin single variable domain are set forth in SEQ ID NOs: 25, 26, and 27 or SEQ ID NOs: 28, 29, and 30, respectively. In some specific embodiments, the amino acid sequence of the immunoglobulin single variable domain is set forth in any one of SEQ ID NOs: 23 and 35-39 or has at least 80% or at least 90% sequence identity thereto, or is set forth in any one of SEQ ID NOs: 24 and 40-43 or has at least 80% or at least 90% sequence identity thereto. In some specific embodiments, the immunoglobulin single variable domain is a single-domain antibody or VHH.

In some embodiments, the CLDN18.2/4-1BB-binding protein comprises one or more (e.g., 2, 3, 4, 5, or 6) first antigen-binding domains that specifically bind to 4-1BB and/or one or more (e.g., 2, 3, or 4) second antigen-binding domains that specifically bind to CLDN18.2; optionally, the CLDN18.2/4-1BB-binding protein further comprises one or more (e.g., 2, 3, 4, 5, or 6) third antigen-binding domains that specifically bind to CD16A. In some specific embodiments, the CLDN18.2/4-1BB-binding protein comprises two first antigen-binding domains that specifically bind to 4-1BB and two second antigen-binding domains that specifically bind to CLDN18.2. In some specific embodiments, the CLDN18.2/4-1BB-binding protein comprises two first antigen-binding domains that specifically bind to 4-1BB, two second antigen-binding domains that specifically bind to CLDN18.2, and two third antigen-binding domains that specifically bind to CD16A.

In some embodiments, in the CLDN18.2/4-1BB-binding protein, a valency ratio of the first antigen-binding domain that specifically binds to 4-1BB to the second antigen-binding domain that specifically binds to CLDN18.2 is between 6:1 and 1:3 (e.g., between 4:1 and 1:2), e.g., 1:1, 1:2, 2:1, 1:3, or 3:1. In some embodiments, in the CLDN18.2/4-1BB-binding protein, a valency ratio of the first antigen-binding domain that specifically binds to 4-1BB to the second antigen-binding domain that specifically binds to CLDN18.2 to the third antigen-binding domain that specifically binds to CD16A is between 6:3:1 and 1:3:6, e.g., 1:1:1, 1:2:2, 2:2:1, 2:1:2, 1:2:1, 3:1:3, 3:1:2, 2:1:3, 3:1:1, or 3:2:1.

In some embodiments, in the CLDN18.2/4-1BB-binding protein, the first antigen-binding domain that specifically binds to 4-1BB is located at the N-terminus and/or the C-terminus of the second antigen-binding domain that specifically binds to CLDN18.2; optionally, in some embodiments, the third antigen-binding domain that specifically binds to CD16A is located at the N-terminus and/or the C-terminus of the second antigen-binding domain that specifically binds to CLDN18.2.

In some embodiments, the CLDN18.2/4-1BB-binding protein further comprises an Fc region of a human immunoglobulin. For example, the Fc region is an Fc region of human IgG1, IgG2, or IgG4.

In some specific embodiments, the Fc region can cause the binding protein to form a dimeric molecule.

In some specific embodiments, the Fc region comprises a mutation that extends the *in vivo* half-life, which depends on the FcRn binding affinity. The extension of half-life can allow for a decrease in the amount of a drug given to a patient and/or a reduction in the frequency of administration. For example, the Fc region comprises mutations M252Y, S254T, and/or T256E.

In some specific embodiments, the Fc region is an Fc region with an enhanced effector function, e.g., an Fc region with enhanced ADCC, ADCP, and/or CDC or an Fc region with decreased fucosylation. The Fc region may comprise a mutation, and exemplary IgG1 Fc regions with an enhanced effector function comprise the following substitutions or any combination thereof: S239D, S239E, S239K, F241A, V262A, V264D, V264L, V264A, V264S, D265A, D265S, D265V, F296A, Y296A, R301A, I332E, S239D/1332E, S239D/A330S/I332E, S239D/A330L/I332E, S298A/D333A/K334A, P247I/A339D, P247I/A339Q, D280H/K290S, D280H/K290S/S298D, D280H/K290S/S298V, F243L/R292P/Y300L, F243L/R292P/Y300L/P396L, F243L/ R292P/Y300L/V305I/P396L, G236A/S239D/I332E, K326A/E333A, K326W/E333S, K290E/S298G/T299A, K290N/S298G/T299A, K290E/S298G/T299A/K326E, or K290N/S298G/T299A/K326E, or any combination of any of the above positions. In some specific embodiments, the CLDN18.2/4-1BB-binding protein comprises an Fc region of human IgG1 with mutations S239D/I332E.

In some other specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

In the present disclosure, in the CLDN18.2/4-1BB-binding protein (e.g., an anti-CLDN18.2/4-1BB bispecific antibody or an anti-CLDN18.2/4-1BB/CD16A trispecific antibody), an Fc region with an enhanced effector function is significantly superior to an Fc region with an unchanged or reduced effector function.

In some specific embodiments, the Fc comprises the amino acid sequence set forth in SEQ ID NO: 65. In some other specific embodiments, the Fc comprises the amino acid sequence set forth in any one of SEQ ID NOs: 14-16.

In some embodiments, the Fc region in the CLDN18.2/4-1BB-binding protein comprises a first subunit Fc1 and a second subunit Fc2 capable of associating with each other.

In some embodiments, Fc1 and Fc2 comprise such amino acid mutations that Fc1 preferentially pairs with Fc2 or preferentially forms a heterodimer as compared to Fc1. In some embodiments, the mutations are located in the CH3 domains of Fc1 and Fc2. In some embodiments, the amino acid mutations in Fc1 and Fc2 result in greater electrostatic complementarity than a wild type without the mutations. In some embodiments, the amino acid mutations in Fc1 and Fc2 result in greater steric complementarity than a wild type without the mutations.

In some embodiments, in Fc1 and Fc2, for example, within the CH3/CH3 interface, one or more amino acid residues in the CH3 domain of Fc1 are mutated with one or more amino acid residues having a larger side-chain volume, thereby forming protuberances (or knobs) in the surface of the CH3 domain of Fc1; one or more, preferably two or three, of the amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some embodiments, an import residue having a larger side-chain volume is phenylalanine (F), tyrosine (Y), arginine (R), or tryptophan (W). In some embodiments, an import residue having a smaller side-chain volume is serine (S), alanine (A), valine (V), or threonine (T).

In some specific embodiments, the Fc1 comprises at least one or at least two amino acid mutations selected from the group consisting of T366S, L368A, and Y407V (hole mutation modifications), and the Fc2 comprises T366W (a knob mutation modification); or the Fc1 comprises T366W (a knob mutation modification), and the Fc2 comprises at least one or at least two amino acid mutations selected from the group consisting of T366S, L368A, and Y407V (hole mutation modifications).

In some specific embodiments, Fc1 and Fc2 may comprise natural-non-cysteine-to-cysteine mutations, for example, in the CH3 domains; for example, Fc1 comprises S354C, and Fc2 comprises Y349C; or Fc1 comprises Y349C, and Fc2 comprises S354C.

In some specific embodiments, Fc1 and Fc2, for example, within the Fc1 CH3/Fc2 CH3 interface, comprise the following amino acid mutations or a combination thereof: T366Y/Y407T; T366W/Y407A; T366Y/Y407T; T394W/F405A; T366Y/F405AT394W/ Y407T; T366W/F405WT394S/Y407A; F405W/T394S; D399C/K392C; T366W/T366S/ L368A/Y407V; T366W/D399C/T366S/L368A/K392C/Y407V; T366W/K392C/T366S/ D399C/L368A/Y407V; S354C/T366W/Y349C/T366S/L368A/Y407V; Y349C/T366W/ S354C/T366S/L368A/Y407V; E356C/T366W/Y349C/T366S/L368A/Y407V; Y349C/ T366W/E356C/T366S/L368A/Y407V; E357C/T366W/Y349C/T366S/L368A/Y407V; and Y349C/T366W/E357C/T366S/L368A/Y407V.

In some specific embodiments, Fc1 and Fc2 further comprise amino acid mutations that cause the formation of an electrostatic interaction interface between Fc1 and Fc2 (e.g., CH3 and CH3). The amino acid mutations that cause the formation of the electrostatic interaction interface are selected from the group consisting of, for example, K370E/D399K/K439D/D356K/E357K/K409D; K409D/D399K; K409E/D399K; K409E/D399R; K409D/D399R; D339K/E356K; D399K/E356K/K409D/K392D; D399K/E356K/K409D/K439D; D399K/E357K/K409D/K370D; D399K/E356K/E357K/ K409D/K392D/K370D; D399K/E357K/K409D/K392D; K392D/K409D/D399K; and K409D/K360D/D399K.

In some embodiments, Fc1 and/or Fc2 comprise(s) domains from different antibody subtypes, e.g., CH3 domains from different antibody subtypes.

Additionally, the present disclosure cites the scheme of modifying the CH3 region of the Fc region to enhance heterodimerization in WO96/27011, WO98/050431, EP1870459, WO2007/110205, WO2007/147901, WO2009/089004, WO2010/129304, WO2011/90754, WO2011/143545, WO2012058768, WO2013157954, and WO2013096291.

In some embodiments, in the CLDN18.2/4-1BB-binding protein, the first antigen-binding domain that specifically binds to 4-1BB and the second antigen-binding domain that specifically binds to CLDN18.2 are linked, either directly or by a linker. In some embodiments, in the CLDN18.2/4-1 BB-binding protein, the first antigen-binding domain that specifically binds to 4-1BB, the second antigen-binding domain that specifically binds to CLDN18.2, and the third antigen-binding domain that specifically binds to CD16A are linked, either directly or by a linker. In some embodiments, the first antigen-binding domain that specifically binds to 4-1BB and the Fc region are linked, either directly or by a linker. In some embodiments, the third antigen-binding domain that specifically binds to CD16A is linked to a light chain constant region of the second antigen-binding domain that specifically binds to CLDN18.2, either directly or by a linker.

In some specific embodiments, the linker includes, but is not limited to, amino acid sequences represented by (GₘSₙ)ₕ or (GGNGT)ₙ or (YGNGT)ₕ or (EPKSS)ₕ, wherein m and n are each independently selected from the group consisting of an integer of 1-8 (e.g., 1, 2, 3, 4, 5, 6, 7, or 8), and h is independently selected from the group consisting of an integer of 1-20 (e.g., 1, 2, 3, 4, 5, 6, 7, 8, 9, 10, 11, 12, 13, 14, 15, 16, 17, 18, 19, or 20). In some specific embodiments, the linker may be a non-functional amino acid sequence of 1-20 or more amino acids without a secondary or higher structure. In some specific embodiments, the linker is a flexible linker. In some specific embodiments, the linker is selected from the group consisting of G₄S, GS, GAP, (G₄S)₂, (G₄S)₃, (G₄S)₄, (G₄S)₅, and ASGS; for example, the linker is (G₄S)₂ or (G₄S)₃.

In some specific embodiments, the CLDN18.2/4-1BB-binding protein comprises a first polypeptide chain and a second polypeptide chain, wherein the first and second polypeptide chains are as follows, from the N-terminus to the C-terminus:
(1) the first polypeptide chain: [the VH of the second antigen-binding domain that specifically binds to CLDN18.2]-CH1-Fc region-[linker 1]a-[the first antigen-binding domain that specifically binds to 4-1BB], and
   the second polypeptide chain: [the VL of the second antigen-binding domain that specifically binds to CLDN18.2]-CL,
(2) the first polypeptide chain: [the first antigen-binding domain that specifically binds to 4-1BB]-[linker 1]a-[the VH of the second antigen-binding domain that specifically binds to CLDN18.2]-CH1-Fc region, and
   the second polypeptide chain: [the VL of the second antigen-binding domain that specifically binds to CLDN18.2]-CL,
(3) the first polypeptide chain: [the VH of the second antigen-binding domain that specifically binds to CLDN18.2]-CH1-Fc region, and
   the second polypeptide chain: [the first antigen-binding domain that specifically binds to 4-1BB]-[linker 1]a-[the VL of the second antigen-binding domain that specifically binds to CLDN18.2]-CL,
(4) the first polypeptide chain: [the VH of the second antigen-binding domain that specifically binds to CLDN18.2]-CH1-Fc region, and
   the second polypeptide chain: [the VL of the second antigen-binding domain that specifically binds to CLDN18.2]-[linker 1]a-CL[the first antigen-binding domain that specifically binds to 4-1BB],
(5) the first polypeptide chain: [the VH of the second antigen-binding domain that specifically binds to CLDN18.2]-CH1-Fc region-[linker 2]b-[the first antigen-binding domain that specifically binds to 4-1BB], and
   the second polypeptide chain: [the VL of the second antigen-binding domain that specifically binds to CLDN18.2]-[linker 1]a-CL[the first antigen-binding domain that specifically binds to 4-1BB],
(6) the first polypeptide chain: [the VH of the second antigen-binding domain that specifically binds to CLDN18.2]-CH1-Fc region-[linker 3]c-[the first antigen-binding domain that specifically binds to 4-1BB], and
   the second polypeptide chain: [the VL of the second antigen-binding domain that specifically binds to CLDN18.2]-CL-[linker 4]d-[the third antigen-binding domain that specifically binds to CD16A], or
(7) the first polypeptide chain: [the VH of the second antigen-binding domain that specifically binds to CLDN18.2]-CH1-Fc region-[linker 3]c-[the third antigen-binding domain that specifically binds to CD16A], and
   the second polypeptide chain: [the VL of the second antigen-binding domain that specifically binds to CLDN18.2]-CL-[linker 4]d-[the first antigen-binding domain that specifically binds to 4-1BB],
wherein - represents a peptide bond; the linkers are polypeptides capable of fulfilling the function of linking; linker 1, linker 2, linker 3, and linker 4 may be identical or different; a, b, c, and d may be independently selected from the group consisting of 0 and 1; for example, a, b, c, and d are all 1. The linkers are, for example, independently selected from the group consisting of G₄S, GS, GAP, (G₄S)₂, (G₄S)₃, (G₄S)₄, (G₄S)₅, and ASGS; for example, the linkers are (G₄S)₂ or (G₄S)₃.

In some embodiments, provided is a CLDN18.2/4-1BB-binding protein comprising a first polypeptide chain and a second polypeptide chain. In some specific embodiments, the amino acid sequence of the first polypeptide chain is set forth in any one of SEQ ID NOs: 47-49 or has at least 80% or at least 90% sequence identity thereto, and the amino acid sequence of the second polypeptide chain is set forth in SEQ ID NO: 46 or has at least 80% or at least 90% sequence identity thereto.

In some specific embodiments, the amino acid sequence of the first polypeptide chain is set forth in any one of SEQ ID NOs: 50, 52, and 53 or has at least 80% or at least 90% sequence identity thereto, and the amino acid sequence of the second polypeptide chain is set forth in SEQ ID NO: 51 or 54 or has at least 80% or at least 90% sequence identity thereto; for example, the amino acid sequence of the first polypeptide chain is set forth in SEQ ID NO: 50 or 52 or has at least 80% or at least 90% sequence identity thereto, and the amino acid sequence of the second polypeptide chain is set forth in SEQ ID NO: 51 or has at least 80% or at least 90% sequence identity thereto; for example, the amino acid sequence of the first polypeptide chain is set forth in SEQ ID NO: 53 or has at least 80% or at least 90% sequence identity thereto, and the amino acid sequence of the second polypeptide chain is set forth in SEQ ID NO: 54 or has at least 80% or at least 90% sequence identity thereto.

In some specific embodiments, the amino acid sequence of the first polypeptide chain is set forth in SEQ ID NO: 55 or 56 or has at least 80% or at least 90% sequence identity thereto, and the amino acid sequence of the second polypeptide chain is set forth in SEQ ID NO: 46 or has at least 80% or at least 90% sequence identity thereto.

In some embodiments, provided is a CLDN18.2/4-1BB-binding protein comprising a first polypeptide chain and a second polypeptide chain, wherein the first polypeptide chain and the second polypeptide chain are selected from the group consisting of:
(1) the amino acid sequence of the first polypeptide chain is set forth in any one of SEQ ID NOs: 47-49, and the amino acid sequence of the second polypeptide chain is set forth in SEQ ID NO: 46;
(2) the amino acid sequence of the first polypeptide chain is set forth in SEQ ID NO: 50 or 52, and the amino acid sequence of the second polypeptide chain is set forth in SEQ ID NO: 51;
(3) the amino acid sequence of the first polypeptide chain is set forth in SEQ ID NO: 53, and the amino acid sequence of the second polypeptide chain is set forth in SEQ ID NO: 54; and
(4) the amino acid sequence of the first polypeptide chain is set forth in SEQ ID NO: 55 or 56, and the amino acid sequence of the second polypeptide chain is set forth in SEQ ID NO: 46.

In some embodiments, the CLDN18.2/4-1BB-binding protein of the present disclosure has an activity selected from the group consisting of at least one of the following:
(a) binding to human 4-1BB or an epitope thereof with a K_{D} value of ≤10⁻⁷;
(b) when not cross-linked by CLDN18.2 (or not binding to CLDN18.2), exhibiting weak or no activation of the 4-1BB signaling pathway; for example, when at a 100 n antibody concentration and not cross-linked by CLDN18.2 (or not binding to CLDN18.2), exhibiting a degree of activation that is no more than 10% of the activity when cross-linked by CLDN18.2 (or binding to CLDN18.2) and at a saturated antibody concentration;
(c) when cross-linked by CLDN18.2 (or not binding to CLDN18.2), exhibiting relatively strong or strong activation of the 4-1BB signaling pathway, for example, with an EC₅₀ of less than 1 nM;
(d) activating T cells and/or promoting T cell proliferation;
(e) inhibiting tumor growth; and
(f) having an enhanced ADCC function and/or an enhanced ADCP function;
the activation of the 4-1BB signaling pathway in (b) and (c) is measured, for example, using the 4-1BB/NF-κB luciferase reporter gene assay of Example 2.

In some embodiments, the CLDN18.2/4-1BB-binding protein of the present disclosure is capable of inhibiting tumor growth by at least about 10%, e.g., at least about 20%, about 30%, about 40%, about 50%, about 60%, about 70%, or about 80%.

In some embodiments, the CLDN18.2/4-1BB-binding protein of the present disclosure encompasses a variant, wherein the variant comprises one or more (e.g., 1, 2, 3, 4, 5, 6, 7, 8, 9, or 10) amino acid mutations as compared to the first and second polypeptide chains in any one of the aforementioned combinations (1)-(4); the amino acid mutations may be conservative replacements, substitutions, or modifications, and/or deletions or additions that do not affect function.

In some embodiments, provided is a CLDN18.2/4-1BB-binding protein that binds to or competes with the aforementioned CLDN18.2/4-1BB-binding protein of the present disclosure for binding to CLDN18.2 and/or 4-1BB, or the same epitope of CLDN18.2 and/or 4-1BB. When the CLDN18.2/4-1BB-binding protein further binds to CD16A, provided is a CLDN18.2/4-1BB-binding protein that binds to or competes with the aforementioned CLDN18.2/4-1BB-binding protein of the present disclosure for binding to CLDN18.2 and/or 4-1BB and/or CD16A, or the same epitope of CLDN18.2 and/or 4-1BB and/or CD16A.

In some embodiments, provided is a CLDN18.2/4-1BB-binding protein that blocks the binding of the aforementioned CLDN18.2/4-1BB-binding protein of the present disclosure to CLDN18.2 and/or 4-1BB and optionally blocks the binding of the aforementioned CLDN18.2/4-1BB-binding protein of the present disclosure to CD16A. In some embodiments, provided is a protein or molecule comprising any of the aforementioned CLDN18.2/4-1BB-binding proteins of the present disclosure. For example, the protein or molecule is a conjugate, and the conjugate may, for example, comprise any detectable label.

### Polynucleotide and Vector

The present disclosure provides a polynucleotide encoding the 4-1BB-binding protein, the CD16A-binding protein, or the CLDN18.2/4-1BB-binding protein of the present disclosure. The nucleic acid of the present disclosure may be an RNA, DNA, or cDNA. According to some embodiments of the present disclosure, the nucleic acid of the present disclosure is substantially an isolated nucleic acid.

The nucleic acid of the present disclosure may also be in the form of a vector and may be present in the vector and/or may be part of the vector. The vector is, for example, a plasmid, cosmid, YAC, or viral vector. The vector may especially be an expression vector, i.e., a vector that provides for *in vitro* and/or *in vivo* (i.e., in suitable host cells, host organisms, and/or expression systems) expression of the 4-1BB-binding protein, the CD16A-binding protein, or the CLDN18.2/4-1BB-binding protein. The expression vector generally comprises at least one of the nucleic acids of the present disclosure, which is operatively linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expression of the 4-1BB-binding protein, the CD16A-binding protein, and the CLDN18.2/4-1BB-binding protein of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell that expresses or is capable of expressing one or more of the 4-1BB-binding proteins, CD16A-binding proteins, and CLDN18.2/4-1BB-binding proteins of the present disclosure and/or comprises the polynucleotide or vector of the present disclosure. In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus*; or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

### Preparation Method

The present disclosure provides a method for preparing a 4-1BB-binding protein, a CD16A-binding protein, or a CLDN18.2/4-1BB-binding protein, the method comprising: expressing the target protein in the aforementioned host cell and isolating the target protein from the host cell. Optionally, the method may further comprise a purification step, for example, purifying using an A or G Sepharose FF column containing an adjusted buffer to wash off non-specifically bound components, then eluting bound antibodies using a pH gradient method, detecting using SDS-PAGE, and collecting. Optionally, filtration and concentration are performed by conventional methods. Soluble mixtures and polymers can also be removed by conventional methods, such as molecular sieves and ion exchange. The resulting products need to be immediately frozen, such as at - 70 °C, or lyophilized.

Methods for producing and purifying antibodies are well known in the prior art and can be found in, for example, "Antibodies: A Laboratory Manual", Cold Spring Harbor Press (chapters 5-8 and 15).

The engineered antibodies or antigen-binding fragments of the present disclosure can be prepared and purified by conventional methods. For example, cDNA sequences encoding heavy and light chains can be cloned and recombined into an expression vector. CHO cells can be stably transfected with recombinant immunoglobulin expression vectors. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange.

### Composition

The present disclosure provides a composition comprising the aforementioned 4-1BB-binding protein, CD16A-binding protein, or CLDN18.2/4-1BB-binding protein of the present disclosure. For example, provided is a pharmaceutical composition comprising an amount of the aforementioned 4-1BB-binding protein, CD16A-binding protein, or CLDN18.2/4-1BB-binding protein that is effective in treating, alleviating, or preventing a cancer, and at least one pharmaceutically acceptable excipient, diluent, or carrier.

In some specific embodiments, a unit dose of the pharmaceutical composition may comprise 0.01 to 99 weight% of the 4-1BB-binding protein, the CD 16A-binding protein, or the CLDN18.2/4-1BB-binding protein, or a unit dose of the pharmaceutical composition comprises 0.1-2000 mg of the 4-1BB-binding protein, the CD16A-binding protein, or the CLDN18.2/4-1BB-binding protein. In some specific embodiments, a unit dose of the pharmaceutical composition comprises 1-1000 mg of the 4-1BB-binding protein, the CD16A-binding protein, or the CLDN18.2/4-1 BB-binding protein.

In some embodiments, provided is a product (e.g., a kit) comprising at least one container, wherein the container independently comprises the aforementioned 4-1BB-binding protein, CD16A-binding protein, or CLDN18.2/4-1BB-binding protein. Optionally, the product comprises a container and a label. Examples of containers are bottles, syringes, and test tubes. The container accommodates a composition effective in treating a disorder. The label on or associated with the container indicates that the composition is used for treating the disorder of choice.

### Treatment Method and Pharmaceutical Use

The present disclosure provides the aforementioned 4-1BB-binding protein, CD16A-binding protein, CLDN18.2/4-1BB-binding protein, polynucleotide, or composition (including the pharmaceutical composition) for use in treating, alleviating, preventing, or diagnosing a disease or disorder.

In some embodiments, provided is a method for ameliorating, alleviating, treating, or preventing a disease, the method comprising administering to a subject an amount of the aforementioned 4-1BB-binding protein, CD16A-binding protein, CLDN18.2/4-1BB-binding protein, polynucleotide, or composition (including the pharmaceutical composition) that is effective in ameliorating, alleviating, treating, or preventing the disease.

In some embodiments, provided is use of the 4-1BB-binding protein, the CD16A-binding protein, the CLDN18.2/4-1BB-binding protein, the polynucleotide, or the composition (including the pharmaceutical composition) of the present disclosure for the manufacture of a medicament for ameliorating, alleviating, treating, or preventing a disease.

In some embodiments, the aforementioned disease is a proliferative disorder or any other disease or disorder characterized by uncontrolled cell growth, such as a cancer. In the present disclosure, "cancer" and "tumor" are used interchangeably.

In some embodiments, the aforementioned cancer is a solid tumor or hematological tumor.

In some embodiments, the aforementioned cancer is advanced or metastatic.

In some embodiments, the aforementioned disease is CLDN18.2-associated or CLDN18.2-positive, e.g., a CLDN18.2-positive cancer. In some embodiments, Claudin18.2-associated or CLDN18.2-positive diseases can be diagnosed by detecting or assaying cells expressing Claudin18.2 with the antibodies or antibody fragments of the present disclosure. In order to detect cells expressing polypeptides, known immunodetection methods can be used, and immunoprecipitation, fluorescent cell staining, immunohistochemical staining, etc. are preferred. In addition, a fluorescent antibody staining method utilizing the FMAT8100HTS system (Applied Biosystem) can be used. In the present disclosure, there is no particular limitation on a test sample used for detection or determination of the target antigen (e.g., Claudin18.2), as long as it has the possibility of comprising cells expressing the target antigen (e.g., Claudin18.2), such as histiocytes, blood, plasma, serum, pancreatic juice, urine, feces, tissue fluid, or culture solution.

In some embodiments, the aforementioned cancer is selected from the group consisting of the following and combinations thereof: lung cancer, prostate cancer, breast cancer, head and neck cancer, esophageal cancer, gastric cancer, colon cancer, colorectal cancer, bladder cancer, cervical cancer, uterine cancer, ovarian cancer, liver cancer, melanoma, renal cancer, squamous cell carcinoma, hematological cancer, and any other diseases or disorders characterized by uncontrolled cell growth.

### Detection

The present disclosure provides use of the 4-1BB-binding protein, the CD16A-binding protein, the CLDN18.2/4-1BB-binding protein, the polynucleotide, or the composition for detection. The present disclosure further provides a method, system, or device for detecting 4-1BB, CLDN18.2, or CD16A *in vivo* or *in vitro,* comprising treating a sample with the aforementioned binding protein, polynucleotide, or composition of the present disclosure.

In some embodiments, the method, system, or device for *in vitro* detection may comprise, for example,
(1) contacting the sample with the 4-1BB-binding protein, the CD16A-binding protein, the CLDN18.2/4-1BB-binding protein, the polynucleotide, or the composition of the present disclosure;
(2) detecting a complex formed between the aforementioned binding protein or polynucleotide and the sample; and/or
(3) contacting a reference sample (e.g., a control sample) with the binding protein or nucleic acid; and
(4) determining the extent of formation of the complex by comparison with the reference sample. A change (e.g., a statistically significant change) in complex formation in the sample as compared to the control sample indicates the presence of 4-1BB, CLDN18.2, or CD16A in the sample.

In some embodiments, further provided is a kit comprising the aforementioned 4-1BB-binding protein, CD16A-binding protein, CLDN18.2/4-1BB-binding protein, or polynucleotide, wherein the kit may further comprise instructions for diagnostic use. The kit may further comprise at least one additional reagent, such as a label or an additional diagnostic agent. For *in vivo* use, the 4-1BB-binding protein, the CD 16A-binding protein, or the CLDN18.2/4-1BB-binding protein may be formulated into a pharmaceutical composition.

### Definitions of Terms

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558 (1968).

"CLDN18 protein" or "CLDN18" is a protein encoded by the Claudin18 gene in humans and belongs to the cellular tight junction protein family. Claudin-18 can control the flow of molecules between layer cells. The Claudin-18 protein structure comprises four transmembrane regions and two extracellular loops, with its N-terminus and C-terminus in the cytoplasm. There are two splice variants of Claudin-18, Claudin 18.1 and Claudin 18.2, which differ in sequence by eight amino acids in the first extracellular loops. Claudin 18.1 and Claudin 18.2 differ in expression distribution. Claudin 18.1 is selectively expressed in normal lung cells, while the expression of Claudin 18.2 is highly restricted in normal cells, but it is frequently ectopically activated and overexpressed in a variety of tumors (gastric cancer, lung cancer, pancreatic cancer, etc.).

"CD16", also known as FcγRIII, is a low-affinity receptor for the Fc fragments of IgGs associated with antibody-dependent cell-mediated cytotoxicity (ADCC). Human FcyRIII has two subtypes: FcyRIIIA (i.e., CD16A) and FcyRIIIB (i.e., CD16B), which share 96% sequence identity in their extracellular immunoglobulin-binding region sequences (van de Winkel and Capel, 1993, Immunol Today 14(5):215-221). CD16A is a transmembrane receptor expressed on macrophages, mast cells, and NK cells. On NK cells, the α chain of CD16A binds to an immunoreceptor tyrosine-based activation motif (ITAM) containing FcεRIγ chains and/or T-cell receptor (TCR)/CD3ζ-chains (Wirthmueller et al., 1992, J. Exp. Med. 175:1381-1390), inducing signaling, which leads to cytokine production and cytotoxic effects. CD16B exists as a glycosylphosphatidylinositol (GPI)-anchored receptor (FcyRIIIB subtype) on polymorphonuclear neutrophils (PMNs). This receptor cannot trigger killing of tumor cells (van de Winkel and Capel, 1993, supra). In addition, CD16B exists as a soluble receptor in serum. Upon binding to an antibody *in vivo,* it may cause side effects by forming an immune complex.

"4-1BB protein" or "4-1BB", also known as CD137 or tumor necrosis factor receptor superfamily 9, is a member of the TNF receptor superfamily (TNFRSF) and a costimulatory molecule expressed on the surface of CD8⁺ and CD4⁺ T cells, regulatory T cells (Tregs), NK cells and NKT cells, B cells, neutrophils, etc. 4-1BB is a costimulatory molecule expressed upon activation of immune cells. Human 4-1BB protein is under the NCBI accession number NP_001552.2. In the present disclosure, "4-1BB" may optionally include any protein of this kind, or fragments or variants thereof, including (but not limited to) the known or wild-type 4-1BB described in the present disclosure, as well as any naturally occurring splice variants, amino acid variants, or isoforms, for example, the human 4-1BB set forth in SEQ ID NO: 11.

"4-1BB-binding protein" encompasses any molecule capable of specifically binding to a 4-1BB protein or an epitope thereof, including but not limited to antibodies targeting 4-1BB as defined in the present disclosure, antigen-binding fragments thereof, or conjugates thereof. The "4-1BB-binding protein" of the present disclosure may comprise at least one (e.g., 1, 2, 3, 4, 5, or 6 or more) immunoglobulin single variable domain (e.g., VHH) that binds to 4-1BB. The "4-1BB-binding protein" of the present disclosure may comprise, in addition to the immunoglobulin single variable domain of 4-1BB, a linker and/or a moiety with effector functions, such as a half-life extending moiety (e.g., an immunoglobulin single variable domain that binds to serum albumin) and/or a fusion partner (such as serum albumin) and/or a conjugated polymer (such as PEG) and/or an Fc region.

"CD16A-binding protein" is defined similarly to "4-1BB-binding protein" and encompasses any molecule capable of specifically binding to a CD16A protein or an epitope thereof, including but not limited to antibodies targeting CD16A as defined in the present disclosure, antigen-binding fragments thereof or conjugates thereof, and fusion proteins.

"CLDN18.2/4-1BB-binding protein" encompasses any molecule capable of specifically binding to a CLDN18.2 protein or an epitope thereof and a 4-1BB protein or an epitope thereof, including but not limited to antibodies, polypeptides, fusion proteins of antibodies and polypeptides, or conjugates thereof. In some embodiments, "CLDN18.2/4-1BB-binding protein" encompasses the anti-CLDN18.2/4-1BB bispecific antibodies in the examples of the present disclosure. In some embodiments, the "CLDN18.2/4-1BB-binding protein" of the present disclosure further comprises a CD16A-binding domain, so that the "CLDN18.2/4-1BB-binding protein" of the present disclosure can further bind to CD16A; for example, the "CLDN18.2/4-1BB-binding protein" of the present disclosure encompasses the anti-CLDN18.2/4-1BB/CD16A trispecific antibodies in the examples of the present disclosure.

"Antibody" encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) as long as they exhibit the desired antigen-binding activity. An antibody may refer to an immunoglobulin, which is of a four-peptide-chain structure formed by two heavy chains and two light chains linked by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the native (non-recombinant) cellular form or express native genes that are abnormally expressed, lowly expressed, or not expressed at all.

"Antigen-binding fragment" encompasses a single-chain antibody (i.e., full-length heavy and light chains); a Fab, a modified Fab, a Fab', a modified Fab', a F(ab')2, an Fv, a Fab-Fv, a Fab-dsFv, a single-domain antibody (e.g., VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9):1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

For the determination or definition of CDRs, the deterministic depiction of CDRs and the identification of residues of binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

In the present disclosure, "effector function" refers to those biological activities that are attributable to the Fc region of an antibody and vary with the antibody isotype. Examples of antibody effector functions include: C1q binding and complement-dependent cytotoxicity (CDC), antibody-dependent cell-mediated cytotoxicity (ADCC), antibody-dependent cellular phagocytosis (ADCP), Fc receptor (FcR) binding, cytokine secretion, immune complex-mediated antigen uptake by antigen-presenting cells, down-regulation of cell surface receptors (e.g., B cell receptor), and B cell activation. For example, ADCC is an immune mechanism leading to the lysis of antibody-coated target cells by immune effector cells. The target cells are cells to which Fc region-containing antibodies or derivatives thereof specifically bind. "Enhanced ADCC" is defined as an increase in the number of target cells that are lysed in a given time, at a given concentration of antibody in the medium surrounding the target cells, by the mechanism of ADCC defined above, and/or a reduction in the concentration of antibody in the medium surrounding the target cells, required to achieve the lysis of a given number of target cells in a given time, by the mechanism of ADCC. The enhancement to ADCC is relative to the ADCC mediated by the same antibody that is produced by the same type of host cells using the same standard production, purification, formulation, and storage methods known to those skilled in the art but has not been engineered. For example, the enhancement to ADCC mediated by an antibody comprising in its Fc region an amino acid substitution that enhances ADCC is relative to the ADCC mediated by the same antibody without this amino acid substitution in the Fc region. Suitable assays for measuring ADCC are well known in the art (see, e.g., WO2006/082515 or WO2012/130831).

"Domain" of a polypeptide or protein refers to a folded protein structure that is capable of maintaining its tertiary structure independently of the rest of the protein. In general, a domain is responsible for a particular functional property of a protein, and in many cases may be added, deleted, or transferred to other proteins without loss of functions of the rest of the protein and/or the domain.

"Immunoglobulin domain" refers to a globular region of an antibody chain (e.g., a chain of a conventional antibody with a four-peptide-chain structure or a heavy-chain antibody) or a polypeptide essentially consisting of such globular regions. Immunoglobulin domains are characterized by their maintenance of the immunoglobulin folding feature of the antibody molecule.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2" and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain that serves as a framework for the complementarity-determining regions (CDRs) of the variable domain. "Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH", also known as heavy-chain single-domain antibody, VHH, V_{H}H domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH" is used to distinguish the variable domain from the heavy chain variable domain (which is referred to in the present disclosure as a "VH domain" or VH) and the light chain variable domain (which is referred to in the present disclosure as a "VL domain" or VL) present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. The terms "heavy-chain single-domain antibody", "VHH domain", "VHH", "V_{H}H domain", "VHH antibody fragment", "VHH antibody", "Nanobody^{®}", and "Nanobody^{®} domain" ("Nanobody" is a trademark of Ablynx N.V., Ghent, Belgium) are used interchangeably. VHHs include, but are not limited to, natural antibodies produced by camelids, antibodies produced by camelids and then humanized, or antibodies obtained by screening using phage display techniques. The total number of amino acid residues in a VHH will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009 and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences, and in some specific embodiments, may contain the human framework region sequence of IGHV3. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the VBase human species sequence database, as well as in Kabat, E. A. et al., 1991 Sequences of Proteins of Immunological Interest, 5th edition. The humanized antibody of the present disclosure also includes humanized antibodies formed by further affinity maturation of the CDRs by phage display. Additionally, in order to avoid the decrease in activity caused by the decrease in immunogenicity, the framework region sequence in the human antibody variable region can be subjected to minimum reverse mutation or back mutation to maintain activity.

An "affinity-matured" antibody is an antibody that has one or more changes in one or more hypervariable regions (HVRs) that result in an improvement in the affinity of the antibody for the antigen, as compared to the parent antibody which does not have such changes. For example, an "affinity-matured" 4-1BB-binding protein or anti-4-1BB antibody has one or more changes in one or more CDRs that result in an increase in the affinity for the antigen as compared to its parent antibody. Affinity-matured antibodies can be prepared, for example, by methods known in the art as described below: Marks et al., 1992, Biotechnology 10:779-783 or Barbas et al., 1994, Proc. Nat. Acad. Sci, USA 91:3809-3813; Shier et al., 1995, Gene 169:147-155; Yelton et al., 1995, Immunol. 155:1994-2004; Jackson et al., 1995, J. Immunol. 154(7):3310-9; Hawkins et al., 1992, J. MoI. Biol. 226(3):889896; KS Johnson and RE Hawkins, "Affinity maturation of antibodies using phage display", Oxford University Press 1996.

Typically, the 4-1BB-binding protein, the CLDN18.2/4-1BB-binding protein, or the CD16A-binding protein of the present disclosure will bind to the antigen or target protein to be bound (i.e., 4-1BB, CLDN18.2, or CD16A) with a dissociation constant (K_{D}) of preferably 10⁻⁷ to 10⁻¹⁰ mol/L (M), more preferably 10⁻⁸ to 10⁻¹⁰ mol/L, and even more preferably 10⁻⁹ to 10⁻¹⁰ or less, and/or with an association constant (KA) of at least 10⁻⁷ M, preferably at least 10⁻⁸ M, more preferably at least 10⁻⁹ M, and more preferably at least 10⁻¹⁰ M, as measured in a Biacore or KinExA or Fortibio assay. Any K_{D} value greater than 10⁻⁴M is generally considered to indicate non-specific binding. Specific binding of an antigen-binding protein to an antigen or epitope can be measured in any suitable manner known, including, for example, surface plasmon resonance (SPR) assays, Scatchard assay, and/or competitive binding assay (e.g., radioimmunoassay (RIA), enzyme immunoassay (EIA), and sandwich competitive assay) described in the present disclosure. "Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining the binding of an epitope to a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (K_{D}). K_{D} can be determined by measuring the kinetics of complex formation and dissociation by using, e.g., the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods, and calculations can even be performed for complex mixtures by, for example, those methods described in Caceci et al (1984, Byte 9:340-362). For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to 4-1BB, CLDN18.2, or CD16A). "Conservative substitution" refers to a substitution with another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity" or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

"Nucleic acid" or "polynucleotide" are used interchangeably in the present disclosure to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Host cell" includes individual cells or cell cultures, which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental, or deliberate mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

"Inhibition" and "blocking" are used interchangeably and encompass both partial and complete inhibition/blocking. "Inhibition of growth" (e.g., involving cells) is intended to include any measurable reduction in cell growth.

"Arresting the growth of" or "growth inhibition" refers to inhibiting cell growth or proliferation.

"Proliferative disease" refers to a disorder associated with a certain degree of abnormal cell proliferation. In one embodiment, the proliferative disorder is cancer. "Tumor" refers to all neoplastic cell growth and proliferation, whether malignant or benign, and all pre-cancerous and cancerous cells and tissues. "Cancer", "proliferative disease", and "tumor" are not mutually exclusive when referred to in the present disclosure.

"Preventing a cancer" refers to delaying, inhibiting, or preventing the onset of the cancer in a subject in which the onset of oncogenesis or tumorigenesis has not been evidenced but a predisposition toward the cancer has been identified, as determined, for example, by genetic screening or otherwise. The term also encompasses treating a subject having a premalignant disorder to stop the progression of, or cause regression of, the premalignant disorder towards malignancy.

"Giving", "administering" and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs or biological fluids, e.g., therapeutic, pharmacokinetic, diagnostic, research and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any binding protein of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective to alleviate one or more disease symptoms in the subject or population being treated, whether by inducing regression of such symptoms or inhibiting the development of such symptoms into any clinically measurable degree. The amount of therapeutic agent effective to alleviate any specific disease symptom (also referred to as the "therapeutically effective amount") may vary depending on factors such as the disease state, the age and weight of the subject, and the capability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

"Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

The "subject" or "patient" in the present disclosure means a mammal, particularly a primate, and especially a human.

### BRIEF DESCRIPTION OF THE DRAWINGS

FIG. 1 shows FACS assay results for the binding of the test antibodies to the human 4-1BB antigen.
FIG. 2 shows the results of 4-1BB/NF-κB luciferase reporter gene assays of the test antibodies before and after FcyRIIb cross-linking.
FIG. 3 shows FACS assay results for the binding of the anti-4-1BB antibody C5 and its humanized antibodies C5_V1, C5_V2, and C5_V3 to human 4-1BB on the surface of HEK293 cells, with urelumab and an isotype IgG1 antibody as controls.
FIG. 4 shows results for the activation of the NF-κB signaling pathway by the anti-4-1BB antibody C5 and its humanized antibodies C5_V1, C5_V2, and C5_V3, with urelumab and an isotype IgG1 antibody as controls.
FIG. 5 shows FACS assay results for the binding of C5_V2 and C5_V2-YTI, which was obtained after engineering by TCE removal, to human 4-1BB on the surface of HEK293 cells, with urelumab and an isotype IgG1 antibody as controls.
FIG. 6 shows experiments of anti-CD16A antibodies binding to cells overexpressing human CD16A, human CD16B, and cynomolgus monkey CD16. In the experiment of binding to cells overexpressing human CD16B, a VHH-2-LALA that can bind to human CD16B was used as a control (the VHH-2 was from US20190276554). The experiments also used an isotype IgG as a control.
FIGs. 7A and 7B show the binding of the anti-CD16A antibodies 34 and 501 and the humanized anti-CD16A antibodies 34H3 and 501-V3NQ to cells overexpressing human CD16A or CD16B. In the experiment of binding to cells overexpressing human CD16B, a VHH-2-LALA that can bind to human CD16B was used as a control (the VHH-2 was from US20190276554). The experiments also used an isotype IgG as a control.
FIG. 8 shows a schematic diagram of the structures of CLDN18.2/4-1BB bispecific antibodies.
FIG. 9 shows a schematic diagram of the structures of CLDN18.2/4-1BB/CD16A trispecific antibodies.
FIG. 10 shows results for the binding of 1903 x C5_V2-YTI-LALA, an antibody obtained after engineering of C5_V2-YTI, to human 4-1BB on the surface of HEK293 cells, with 1903 x C5_V2-LALA, C5_V2, and the isotype IgG1 as controls.
FIG. 11 shows results for the activation of the NF-κB signaling pathway by 1903 x C5 V2-YTI-LALA, an antibody obtained after engineering of C5_V2-YTI, with 1903 x CS_V2-LALA, TJ-CD4B, and the isotype IgG1 as controls.

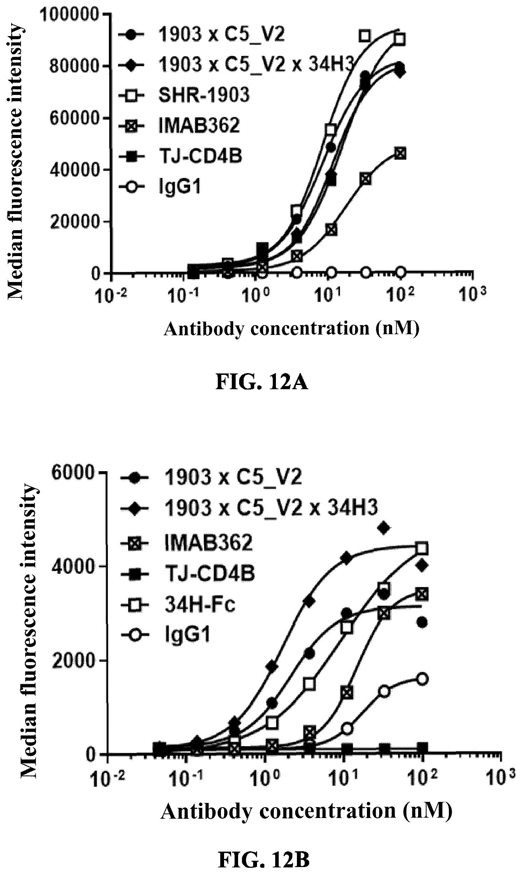

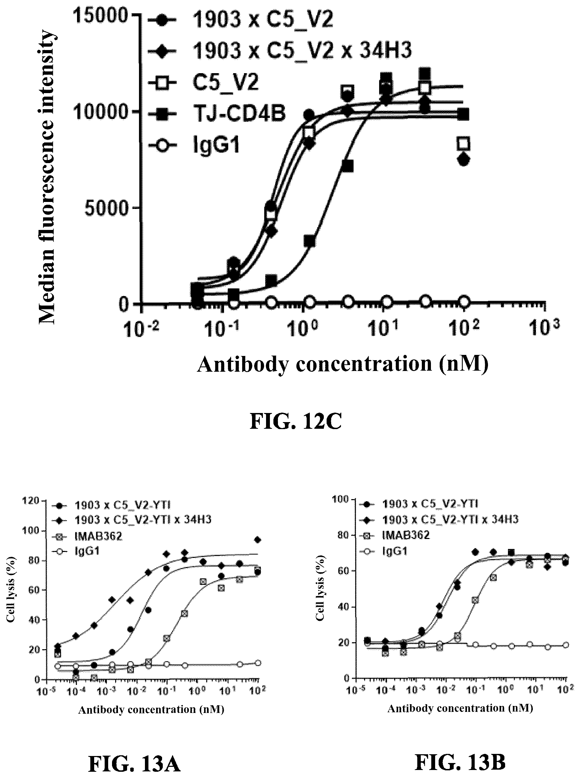

FIGs. 12A to 12C show results for the binding activity of the CLDN18.2/4-1BB multifunctional antibodies 1903 x C5_V2 x 34H3 and 1903 x C5_V2 to the CLDN18.2/CD16A V176/4-1BB antigen on the cell surface. FIG. 12A shows the results of an experiment of antibodies binding to NUGC4-hi18.2 cells, with 1903, IMAB362, TJ-CD4B, and the isotype IgG1 as controls. FIG. 12B shows the results of an experiment of antibodies binding to CHOK1-CD16A V176A cells, with IMAB362, TJ-CD4B, 34H-Fc, and the isotype IgG1 as controls. FIG. 12C shows the results of an experiment of antibodies binding to HEK293-Hu4-1BB cells, with TJ-CD4B, C5-_V2, and the isotype IgG1 as controls.
FIGs. 13A to 13D show results for the activity of the CLDN18.2/4-1BB multifunctional antibodies 1903 x C5_V2-YTI x 34H3 and 1903 x C5_V2-YTI in inducing killing of CLDN18.2-expressing target cells by NK cells, with IMAB362 and the isotype IgG1 as controls. FIG. 13A: the ADCC of PBMCs against NUGC4-hi18.2 cells (donor SC190061). FIG. 13B: the ADCC of PBMCs against NUGC4-hi18.2 cells (donor S2001102). FIG. 13C: the ADCC of PBMCs against SNU601 cells (donor SC190061). FIG. 13D: the ADCC of PBMCs against SNU601 cells (donor S2001102).
FIG. 14 shows results for the phagocytosis of tumor cells by macrophages, induced by the CLDN18.2/4-1BB multifunctional antibodies 1903 x C5_V2-YTI x 34H3 and 1903 x CS_V2-YTI, with IMAB362 and the isotype IgG1 as controls.
FIG. 15 shows the results of CLDN18.2-dependent 4-1BB/NF-κB luciferase reporter gene assays of the CLDN18.2/4-1BB multifunctional antibodies 1903 x C5_V2 x 34H3 and 1903 x C5_V2; on the left are the results before CLDN18.2 cross-linking, and on the right are the results after CLDN18.2 cross-linking; both used TJ-CD4B, ADG-106, and the isotype IgG1 as controls.
FIGs. 16A and 16B show results for the activation of T lymphocytes of donor 1 and donor 2 by the CLDN18.2/4-1BB multifunctional antibodies 1903 x C5_V2-YTI x 34H3 and 1903 x C5_V2-YTI, with TJ-CD4B and the isotype IgG1 as controls.
FIG. 17 shows results for the killing of T cells by NK cells, induced by the CLDN18.2/4-1BB multifunctional antibodies 1903 x C5_V2-YTIx 34H3 and 1903 x C5_V2-YTI, with the isotype IgG1 as a control.
FIGs. 18A and 18B show results for the inhibition of MC38-hCLDN18.2 xenograft tumors by the CLDN18.2/4-1BB multifunctional antibodies 1903 x C5_V2-YTI x 34H3 and 1903 x C5_V2-YTI, with a vehicle and TJ-CD4B as controls. FIG. 18A shows tumor volume results, and FIG. 18B shows the body weight of mice.
FIG. 19 shows the influence of the CLDN18.2/4-1BB multifunctional antibodies 1903 x C5_V2-YTI x 34H3 and 1903 x C5_V2-YTI on mouse ALT/AST, with a vehicle and TJ-CD4B as controls.
FIG. 20 shows a pharmacokinetic curve of the CLDN18.2/4-1BB multifunctional antibody 1903 x C5_V2 in h4-1BB transgenic mice, with 1903 as a control.

### DETAILED DESCRIPTION

The present disclosure is further described below with reference to examples, but these examples are not intended to limit the scope of the present disclosure. Experimental procedures without specific conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated were commercially available conventional reagents.

The proteins used in the present disclosure: human 4-1BB protein (Human 4-1BB/TNFSF9 Protein, His Tag, purchased from Acrobiosystems, Cat. No. 41B-H5227), human 4-1BB protein (biotin/His Tag) (Biotinylated Human 4-1BB/TNFRSF9 Protein, Avitag^{™}, His Tag, purchased from Acrobiosystems, Cat. No. 41B-H82E3), and monkey 4-1BB protein (Cynomolgus / Rhesus macaque 4-1BB/TNFRSF9 Protein, His Tag, purchased from Acrobiosystems, Cat. No. 41B-C52H4); the amino acid sequences all start with Leu24 and end with Gln186.

The protein reagents described above can be used in the experiments of the examples of the present disclosure, including use as immunizing and screening antigens and in activity and function identification. The amino acid sequences of the control molecules used in the present disclosure are shown in Table 2.

**Table 2. The sequences of the control molecules**

| Name | Amino acid sequence | |
|---|---|---|
| Urelumab | Heavy chain | QVQLQQWGAGLLKPSETLSLTCAVYGGSFSGYYWSWIRQ SPEKGLEWIGEINHGGYVTYNPSLESRVTISVDTSKNQFSL KLSSVTAADTAVYYCARDYGPGNYDWYFDLWGRGTLVTV SSASTKGPSVFPLAPCSRSTSESTAALGCLVKDYFPEPVTVS WNSGALTSGVHTFPAVLQSSGLYSLSSVVTVPSSSLGTKTY TCNVDHKPSNTKVDKRVESKYGPPCPPCPAPEFLGGPSVFL FPPKPKDTLMISRTPEVTCVVVDVSQEDPEVQFNWYVDGV EVHNAKTKPREEQFNSTYRVVSVLTVLHQDWLNGKEYKC KVSNKGLPSSIEKTISKAKGQPREPQVYTLPPSQEEMTKNQ VSLTCLVKGFYPSDIAVEWESNGQPENNYKTTPPVLDSDGS FFLYSRLTVDKSRWQEGNVFSCSVMHEALHNHYTQKSLSL SLGK (SEQ ID NO:1) |
| | Light chain | EIVLTQSPATLSLSPGERATLSCRASQSVSSYLAWYQQKPG QAPRLLIYDASNRATGIPARFSGSGSGTDFTLTISSLEPEDFA VYYCQQRSNWPPALTFCGGTKVEIKRTVAAPSVFIFPPSDE QLKSGTASVVCLLNNFYPREAKVQWKVDNALQSGNSQES VTEQDSKDSTYSLSSTLTLSKADYEKHKVYACEVTHQGLS SPVTKSFNRGEC        (SEQ ID NO:2) |
| IMAB362 | Heavy chain | QVQLQQPGAELVRPGASVKLSCKASGYTFTSYWINWVKQ RPGQGLEWIGNIYPSDSYTNYNQKFKDKATLTVDKSSSTAY MQLSSPTSEDSAVYYCTRSWRGNSFDYWGQGTTLTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKRVEPKSCDKTHTCPPCPAPELLGGPSVFLF PPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSREEMTKNQV SLTCLVKGFYPSDIAVEWESNGQPENNYKTTPPVLDSDGSF FLYSKLTVDKSRWQQGNVFSCSVMHEALHNHYTQKSLSLS PGK (SEQ ID NO:3) |
| | Light chain | DIVMTQSPSSLTVTAGEKVTMSCKSSQSLLNSGNQKNYLT WYQQKPGQPPKLLIYWASTRESGVPDRFTGSGSGTDFTLTI SSVQAEDLAVYYCQNDYSYPFTFGSGTKLEIKRTVAAPSVF IFPPSDEQLKSGTASVVCLLNNFYPREAKVQWKVDNALQS GNSQESVTEQDSKDSTYSLSSTLTLSKADYEKHKVYACEV THQGLSSPVTKSFNRGEC (SEQ ID NO:4) |
| TJ-CD4B | Heavy chain | QVQLVQSGAEVKKPGASVKVSCKASGYTFTTYPIEWVRQ APGQRLEWMGNFHPYNDDTKYNEKFKGRVTITRDTSAST AYMELSSLRSEDTAVYYCARRAYGYPYAMDYWGQGTLVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFNW YVDGVEVHNAKTKPREEQYASTYRVVSVLTVLHQDWLNG KEYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHY TQKSLSLSPGKGGGGSGGGGSGGGGSQSVLTQPPSASGTP GQRVTISCSGSSSNIGNNYVTWYQQLPGTAPKLLIYADSHR PSGVPDRFSGSKSGTSASLAISGLRSEDEADYYCATWDYSL SGYVFGCGTKLTVLGGGGSGGGGSGGGGSGGGGSEVQLL ESGGGLVQPGGSLRLSCAASGFTFSSYDMSWVRQAPGKCL EWVSWISYSGGSIYYADSVKGRFTISRDNSKNTLYLQMNS LRAEDTAVYYCARDAQRNSMREFDYWGQGTLVTVSS (SEQ ID NO:5) |
| | Light chain | DIVMTQSPDSLAVSLGERATINCKSSQSLLNAGNQKNYLT WYQQKPGQPPKLLIYRASSRESGVPDRFSGSGSGTDFTLTI SSLQAEDVAVYYCQNDYIYPYTFGGGTKLEIKRTVAAPSVF IFPPSDEQLKSGTASVVCLLNNFYPREAKVQWKVDNALQS GNSQESVTEQDSKDSTYSLSSTLTLSKADYEKHKVYACEV THQGLSSPVTKSFNRGEC(SEQ ID NO:6) |
| ADG106 | Heavy chain | EVQLVESGGGLVQPGGSLRLSCAASGFSLSTGGVGVGWIR QAPGKGLEWLALIDWADDKYYSPSLKSRLTISRDNSKNTL YLQLNSLRAEDTAVYYCARGGSDTVIGDWFAYWGQGTLV TVSSASTKGPSVFPLAPCSRSTSESTAALGCLVKDYFPEPVT VSWNSGALTSGVHTFPAVLQSSGLYSLSSVVTVPSSSLGTK TYTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEY KCKVSNKGLPSSIEKTISKAKGQPREPQVYTLPPSQEEMTK NQVSLTCLVKGFYPSDIAVEWESNGQPENNYKTTPPVLDS DGSFFLYSRLTVDKSRWQEGNVFSCSVMHEALHNHYTQK SLSLSLGK (SEQ ID NO:7) |
| | Light chain | DIQLTQSPSSLSASVGDRVTITCRASQSIGSYLAWYQQKPG KAPKLLIYDASNLETGVPSRFSGSGSGTDFTLTISSLQPEDF ATYYCQQGYYLWTFGQGTKVEIKRTVAAPSVFIFPPSDEQL KSGTASVVCLLNNFYPREAKVQWKVDNALQSGNSQESVT EQDSKDSTYSLSSTLTLSKADYEKHKVYACEVTHQGLSSP VTKSFNRGEC (SEQ ID NO:8) |

### Example 1. Screening and Preparation for Anti-4-1BB Single-Domain Antibodies

### 1. Alpaca immunization, titer determination, and phage library affinity panning

Alpacas were immunized with a His-tagged human 4-1BB recombinant protein (Acrobiosystems, 41B-H5227) once every two weeks, and a total of four immunizations were performed. In the first immunization, 0.5 mg of antigen and 1 mL of complete Freund's adjuvant (CFA) were mixed well and injected subcutaneously, and in the following three immunizations, 0.25 mg of antigen and 1 mL of incomplete Freund's adjuvant (IFA) were mixed well and injected subcutaneously. Blank serum was collected before the immunizations, and 50-mL samples of peripheral blood were collected one week after the third immunization and one week after the fourth immunization. PBMCs were isolated, and total RNA was extracted. The purity was tested, and reverse transcription was performed to obtain DNA. After two rounds of nested PCR, the nanobody target fragment was ligated to a phage display vector. Electroporation was performed to obtain a phage library.
> Human 4-1BB protein sequence

LQDPCSNCPAGTFCDNNRNQICSPCPPNSFSSAGGQRTCDICRQCKGVFRTRKEC
SSTSNAECDCTPGFHCLGAGCSMCEQDCKQGQELTKKGCKDCCFGTFNDQKR
GICRPWTNCSLDGKSVLVNGTKERDVVCGPSPADLSPGASSVTPPAPAREPGHSP
Q (SEQ ID NO: 9).

In order to obtain an anti-4-1BB nanobody that simultaneously recognizes humans and monkeys, a strategy of two rounds of cross-screening with human and monkey antigens was adopted. The antigens used in the first and second rounds of screening were human 4-1BB and monkey 4-1BB, or monkey 4-1BB and human 4-1BB, respectively. A Gly-HCl acid elution method was adopted in each round of screening, and phages that specifically bind to 4-1BB were eluted. Ninety-six clones (192 clones in total) were randomly picked from the plates of the first and second rounds of titer determination. A phage ELISA was used to screen for positive clones, and the optical density at 450 nm was measured. The positive clones were sequenced. Based on the sequencing results, sequence alignment and phylogenetic tree analysis were performed, and 14 distinctive sequences, including H27, H170, C3, C5, C145, etc., were selected. The C5 sequence is shown below.
> C5 
QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTQVTVSS (SEQ ID NO: 10)
 (Note: The CDRs are underlined)

**Table 3. The CDRs of the anti-4-1BB single-domain antibody C5 (Kabat numbering scheme)**

| Antibody No. | CDR sequences | | Sequence No. |
|---|---|---|---|
| C5 | CDR1 | SYAMS | SEQ ID NO:11 |
| | CDR2 | DINSGGESTFYEDSVKG | SEQ ID NO:12 |
| | CDR3 | HPLTFTIATMNDYDY | SEQ ID NO:13 |

### 2. Expression and purification of VHH-Fc fusion proteins

The sequence of C5 was linked to a human IgG1-Fc sequence (SEQ ID NO: 16; the mutations are underlined) with mutations C220A, S267E, and L328F (according to the Eu numbering scheme). The resulting VHH-Fc fusion protein sequence is shown below. Furthermore, mutations such as L234A, L235A, and N297A (according to the Eu numbering scheme) were introduced into the Fc of human IgG1 to completely remove the FcγR-mediated effector function of the antibody (e.g., set forth in SEQ ID NO: 15); S228P (according to the Eu numbering scheme) was introduced into the Fc of human IgG4 to stabilize the antibody molecule, preventing half-molecule formation (e.g., set forth in SEQ ID NO: 16). Both are alternative IgG Fc sequences. In SEQ ID NOs: 14-16, Fc mutations are underlined.
> Human IgG1-Fc (containing mutations C220A, S267E, and L328F) 
EPKSADKTHTCPPCPAPELLGGPSVFLFPPKPKDTLMISRTPEVTCVVVDVEHED
PEVKFNWYVDGVEVHNAKTKPREEQYNSTYRVVSVLTVLHQDWLNGKEYKC
KVSNKAFPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK (SEQ ID NO: 14)
> Human IgG1-Fc (containing mutations C220A, L234A, L235A, and N297A) EPKSADKTHTCPPCPAPEAAGGPSVFLFPPKPKDTLMISRTPEVTCVVVDVSHED 
PEVKFNWYVDGVEVHNAKTKPREEQYASTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK (SEQ ID NO: 15)
> Human IgG4-Fc (containing mutation S228P) 
ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLPGK (SEQ ID NO: 16)
The antibody sequence obtained by linking C5 to SEQ ID NO: 14 is set forth in SEQ ID NO: 17 by way of example.
> C5-Fc 
QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTQVTVSSEPKSADKTHTCPPCPAPELLGGPSVFLFPPKPKDTLMI
SRTPEVTCVVVDVEHEDPEVKFNWYVDGVEVHNAKTKPREEQYNSTYRVVSVLTVLH
QDWLNGKEYKCKVSNKAFPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFS
CSVMHEALHNHYTQKSLSLSPGK (SEQ ID NO: 17)
 (Note: The Fc is italicized)
Plasmids were constructed and transiently transfected into cells, and antibodies were expressed and purified. Analysis showed that the antibodies of interest were obtained.

### Example 2. Assays for Antigen-Binding Activity and Agonist Activity of Anti-4-1BB Antibodies

### 1. Assay for ability to bind to 4-1BB antigen

The binding activity of the anti-4-1BB single-domain antibodies to human 4-1BB protein was measured by flow cytometry.

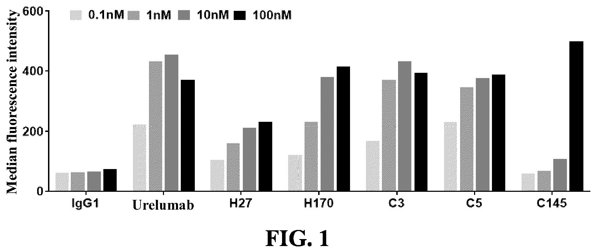

HEK293-Hu4-1BB cells were obtained by transient transfection of HEK293 cells (ATCC CRL-1573) with a gene expressing human 4-1BB protein (CD137 cDNA ORF Clone, Human, C-OFPSpark tag; purchased from Sino Biological, Cat#HG10041-ACR). The culture medium for the cells was a DMEM culture medium (Gibco, Cat#11995065) containing 10% fetal bovine serum. The experimental culture medium was a sterile PBS (phosphate-buffered saline, pH 7.40) containing 2% fetal bovine serum. The HEK293-Hu4-1BB cells were washed with the experimental culture medium twice and seeded into a 96-well U-bottom plate at 1 × 10⁵ HEK293-Hu4-1BB cells per well, and test anti-4-1BB antibody VHH-Fc samples at different concentrations were added. The cells were incubated at 4 °C for 1 h and then washed with the experimental culture medium twice. Subsequently, the goat anti-human IgG (H + L) Alexa Fluor 488 antibody (Thermo, Cat#A11013) was added. After two washes, fluorescence signal values were read using a flow cytometer. Urelumab was used as a positive control. The MFI values for the antibodies are shown in FIG. 1.

The results show that the related anti-4-1BB antibodies exhibited different abilities to bind to 4-1BB on the surface of HEK293-Hu4-1BB cells, and that C5 exhibited good cell membrane surface antigen-binding activity.

### 2. Assay for agonist activity on 4-1BB signaling pathway

The agonist activity of the anti-4-1BB antibodies was assessed using a 4-1BB/NF-κB reporter gene.

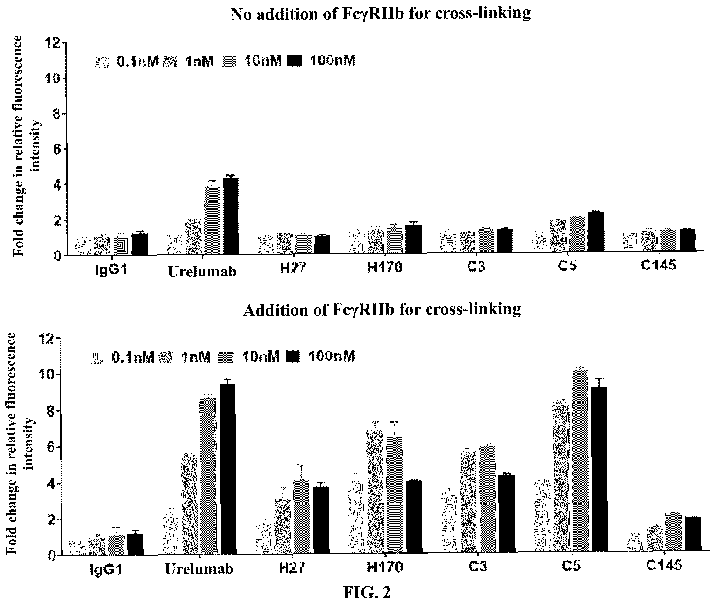

HEK293-Hu4-1BB/NF-κB double-transfected cells were obtained by transient transfection of HEK293 cells (ATCC CRL-1573) with a gene expressing human 4-1BB (CD137 cDNA ORF Clone, Human, C-OFPSpark tag; purchased from Sino Biological, Cat#HG10041-ACR) and the NF-κB reporter gene (pGL4.32[luc2P/NF-κB-RE/Hygro] Vector, purchased from Promega, Cat#E849A). 4-1BB activation can be characterized by the activation level of the NF-κB signaling pathway. HEK293 cells highly expressing FcyRIIb were obtained by transient transfection of HEK293 cells with FcyRIIb plasmids (CD32B/Fcgr2b cDNA ORF Clone, Human, N-His tag; purchased from Sino Biological, Cat#HG10259-NH). The culture medium for the cells was a DMEM culture medium (Gibco, Cat#11995065) containing 10% fetal bovine serum. HEK293-Hu4-1BB/NF-κB cells (2 × 10⁶/mL) were plated onto a 96-well cell culture plate at 50µL, 40 µL of culture medium or FcγRIIb-expressing HEK293 cells (2.5 × 10⁶/mL) was added, and 10× serially diluted test anti-4-1BB antibodies were added at 10 µL/well. The plate was incubated at 37 °C for 6 h. The cells were taken out. An equal volume of Bio-Glo Luciferase Assay System reagent (Promega, Cat#G7940) was added to each well, and the plate was incubated for 5 min in a dark place. Fluorescence signals were measured using an Envision microplate reader (PerkinElmer, 2150). EC₅₀ values and Emax values (relative to the fluorescence intensity of the antibody-free group) were calculated, and the agonist activity of the anti-4-1BB antibodies on cells *in vitro* was assessed based on the EC₅₀ values. The results are shown in FIG. 2 and Table 4.

The results show that when no FcyRIIb (i.e., CD32b) was added for positive cross-linking, the related anti-4-1BB antibodies exhibited much weaker activation of the 4-1BB/NF-κB luciferase reporter gene signaling pathway than the control urelumab, indicating that the anti-4-1BB antibodies of the present disclosure are safer; after FcγRIIb was added for cross-linking, the related anti-4-1BB antibodies exhibited significant activation of the 4-1BB/NF-κB luciferase reporter gene signaling pathway, and the activating ability of C5 was the strongest and comparable to that of the control urelumab. Based on these activity results, the sequence C5, which exhibited low background activation before FcyRIIb cross-linking and stronger activation activity after FcyRIIb cross-linking, was selected and humanized.

**Table 4. The EC₅₀ values for the agonistic activity of the anti-4-1BB antibodies on the 4-1BB/NF-κB luciferase reporter gene**

| No. | FcyRIIb cross-linking | | No FcyRIIb cross-linking | |
|---|---|---|---|---|
| | Emax | EC₅₀(nM) | Emax | EC₅₀(nM) |
| IgG | 1.12 | - | 1.10 | - |
| Urelumab | 9.39 | 0.027 | 4.30 | 2.185 |
| C5 | 10.04 | About 0.8144 | 2.26 | - |

| | | | | |
|---|---|---|---|---|
| (Note: "-" indicates that the measurement was beyond the limit of detection). | | | | |

### Example 3. Engineering of Anti-4-1BB Antibody

### 1. Engineering by humanization

The amino acids of the CDRs and FRs (human framework regions) of the C5 sequence were numbered using the Kabat numbering scheme. The FR1, FR2, and FR3 sequences were matched with an antibody germline database to obtain FR human germline templates with relatively high homology. FR1 was derived from IGHV3-64*04, FR2 from IGHV3-23*03, and FR3 from IGHV3-74*01. The above human germline FRs were inserted into the original sequences as replacements to reduce the immunogenicity produced in the human body. The key amino acids that affect the structure and functioning of the antibody were back-mutated to restore binding affinity and activity. The humanized sequences were as follows.
> C5_V1 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCARHPLTFTIAT
MNDYDYWGQGTLVTVSS (SEQ ID NO: 18)
> C5_V2 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTFTIAT
MNDYDYWGQGTLVTVSS (SEQ ID NO: 19)
>C5_V3 
QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTLVTVSS (SEQ ID NO: 20)
 (Note: The CDRs are underlined)

The above 3 humanized sequences were each linked to human IgG1-Fc (SEQ ID NO: 14). Plasmids were constructed. Transient transfection, expression, and purification were performed. The specific process was as follows: 60 µL of transfection reagent was diluted with culture solution and then mixed with 15 µg of plasmids. After 15 min of incubation at 37 °C, the mixed transfection solution was added dropwise to 30 mL of cell suspension. After one week of culture on a shaker for expression, the supernatant was collected. Subsequently, it was subjected to ProteinA affinity purification, with a citrate buffer (pH 3.4) as the eluent. Finally, the eluate was dialyzed against 1× PBS buffer and cryopreserved.

### 2. Removal of TCE sites

Subsequently, a T-cell Epitope (TCE) prediction was performed on the C5_V2 sequence, and the CDR3 sequence of C5_V2 was engineered based on the prediction results to decrease the number of TCEs. The F in the CDR3 was mutated into Y (F99Y, according to the Kabat numbering scheme) to obtain a TCE-optimized version of the C5_V2 sequence. This version was designated C5_V2-YTI, and its sequence was as follows:
> C5_V2-YTI

QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSS (SEQ ID NO: 21)

That is, according to the Kabat numbering scheme, the amino acid sequence of the CDR1 in C5_V2-YTI is set forth in SEQ ID NO: 11, the amino acid sequence of the CDR2 is set forth in SEQ ID NO: 12, and the amino acid sequence of the CDR3 is HPLTYTIATMNDYDY (SEQ ID NO: 22).

The above C5_V2-YTI sequence was linked to human IgG1-Fc (SEQ ID NO: 14). Plasmids were constructed. Transient transfection, expression, and purification were performed.

3. Amino acid engineering of the VH framework (FR) of the anti-4-1BB single-domain antibody (WO2023093899 is incorporated herein by reference in its entirety) yielded CS_V2-YTI-AA (corresponding to the C5_V2-YTI-53 set forth in the 30th sequence in WO2023093899).

### Example 4. Assays for Antigen-Binding Activity and Agonist Activity of Engineered Anti-4-1BB Antibodies

### 1. Assay for ability to bind to 4-1BB antigen

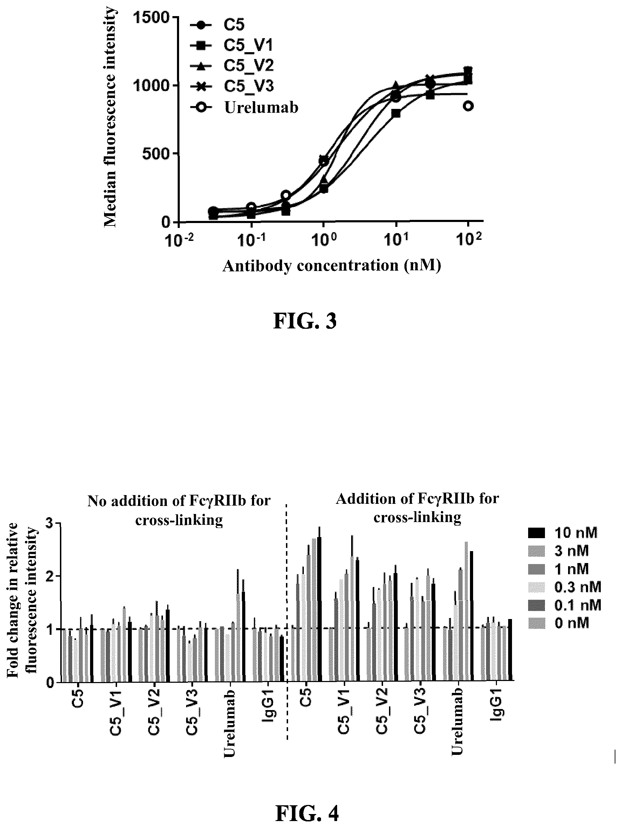

The antigen-binding activity of the humanized antibodies was measured using a FACS assay as described in Example 2. The results are shown in FIG. 3 and Table 5.

The results show that the related humanized anti-4-1BB antibodies exhibited different abilities to bind to 4-1BB on the surface of HEK293-Hu4-1BB cells, and that C5_V1, C5_V2, and C5_V3 all maintained good binding activity.

**Table 5. The EC₅₀ values for the affinities of anti-4-1BB antibodies for a cell strain highly expressing human 4-1BB before/after humanization**

| Antibody No. | EC₅₀ (nM) | Maximal fluorescence value |
|---|---|---|
| C5 | 3.196 | 1101 |
| C_V1 | 3.789 | 1036 |
| C5_V2 | 1.624 | 1031 |
| C5_V3 | 1.651 | 1096 |
| Urelumab | 1.184 | 1005 |

### 2. Assay for agonist activity on 4-1BB signaling pathway

The activation of 4-1BB/NF-κB signaling by the humanized antibodies was measured using an NF-κB luciferase reporter gene assay as described in Example 2. The results are shown in FIG. 4 and Table 6.

The results show that after FcyRIIb was added for cross-linking, the related humanized anti-4-1BB antibodies exhibited activation of the 4-1BB/NF-κB luciferase reporter gene signaling pathway, and that the activating abilities of C5, C5_V1, C5_V2, and C5_V3 were comparable to that of the control urelumab.

**Table 6. The activation of the NF-κB signaling pathway by anti-4-1BB antibodies before/after humanization**

| Antibody No. | Activation of HEK293-4-1BB cells | | FcγRIIb-mediated activation of HEK293-4-1BB cells | |
|---|---|---|---|---|
| | Activity | Fold change in Emax | Activity | Fold change in Emax |
| C5 | - | 1.08 | +++ | 2.71 |
| 5S_V1 | - | 1.14 | ++ | 2.28 |
| C5_V2 | - | 1.37 | ++ | 2.02 |
| C5_V3 | - | 1.01 | + | 1.83 |
| Urelumab | + | 1.70 | +++ | 2.61 |

| | | | | |
|---|---|---|---|---|
| (Note: For a <1.5-fold change in Emax, the activity is defined as "-"; for 1.5-2, as "+"; for 2-2.5, as "++"; for 2.5-3, as "+++"; and for >3, as "++++".) | | | | |

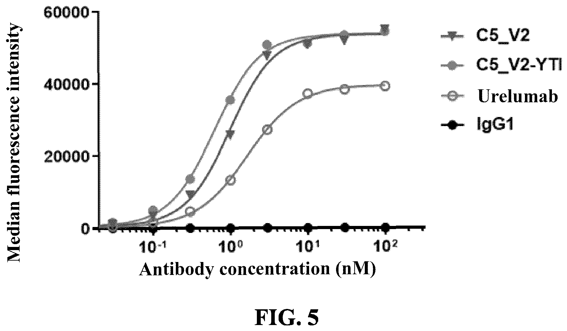

3. Assay for ability of humanized 4-1BB antibody to bind to antigen after TCE removal The humanized antibody C5_V2 was optimized by TCE removal, and the resulting antibody was C5_V2-YTI (see Example 3 for details). A comparison of the antigen-binding activities of C5_V2 and C5_V2-YTI was made using a FACS assay as described in Example 2. The results are shown in FIG. 5 and Table 7. The results show that the related antibody C5_V2-YTI maintained good binding activity to 4-1BB on the surface of HEK293-Hu4-1BB cells, and that its binding activity was comparable to that of C5_V2.

**Table 7. The EC₅₀ values for the affinities of C5_V2 and C5_V2-YTI for a cell strain highly expressing human 4-1BB**

| Antibody No. | EC₅₀ (nM) | Maximal fluorescence value |
|---|---|---|
| C5_V2 | 0.98 | 53,669 |
| C5_V2-YTI | 0.62 | 53,825 |
| Urelumab | 1.64 | 39,666 |
| IgG1 | - | 67 |

### Example 5. Process of Obtaining Anti-CD16A Nanobodies

### 1. Natural library screening of human CD16A

Research shows that in the human body, there is a gene known as FcyRIIIb (CD16B) that is very similar to the CD16A sequence, with a homology exceeding 97%. CD16B is primarily expressed on neutrophils, with a small soluble fraction present in the bloodstream. Therefore, there was a need to screen for specific antibodies that target CD16A and do not recognize CD16B. First, a first round of panning was performed on a camelid natural library using biotinylated human CD16A protein (CDA-H82E8, ACROBiosystems) at a concentration of 5 µg/mL, and elution was performed using Gly-HCL (pH 2.2) to obtain the phages of the first round. Next, the phages were amplified and subjected to a second round of panning. In the second round, negative screening was performed using 5 µg/mL biotinylated human CD16B protein (CDB-H82E4) to remove phages binding to CD16B. Subsequently, screening was performed using 3 mg/mL human CD16A protein, and elution was performed to obtain the product of the second round of panning. Finally, the product of the second round of panning was subjected to a phage ELISA, and phages that recognize human CD16A but not human CD16B were selected and sequenced to obtain the target antibody sequences.

### 2. Camelid immunization with human CD16A and screening

Camelid immunization was used in combination with phage display to screen for and prepare CD16A-specific antibodies. The immunization strategy is shown in Table 8.

**Table 8. The immunization strategy**

| **Process** | **Time** | **Procedure** |
|---|---|---|
| First blood collection | Day 0 | Collect 5 mL of blood before immunization, isolate serum, and store it at -20 °C. |
| First immunization | Day 0 | Mix 400 µg of human CD16A protein (acro, CDA-H5220) and 0.5 mL of complete Freund's adjuvant until they emulsify, evenly divide the emulsion into two parts, and immunize two camelids with the two parts. |
| Second immunization | Day 14 | Mix 400 µg of human CD16A protein (acro, CDA-H5220) and 0.5 mL of incomplete Freund's adjuvant until they emulsify, evenly divide the emulsion into two parts, and immunize two camelids with the two parts. |
| Third immunization | Day 28 | Mix 400 µg of human CD16A protein (acro, CDA-H5220) and 0.5 mL of incomplete Freund's adjuvant until they emulsify, evenly divide the emulsion into two parts, and immunize two camelids with the two parts. |
| Fourth immunization | Day 42 | Mix 400 µg of human CD16A protein (acro, CDA-H5220) and 0.5 mL of incomplete Freund's adjuvant until they emulsify, evenly divide the emulsion into two parts, and immunize two camelids with the two parts. |
| Second blood collection | Day 58 | Collect 5 mL of blood, isolate serum, and determine the titer. |
| Third blood collection | Days 59-60 | If the titer is desirable, collect 200 mL of anticoagulated blood from each animal, isolate PBMCs, and construct a library. |

PBMCs were isolated from the blood samples collected in the fourth immunization, and a library was then constructed. The screening strategy was consistent with the strategy used for the screening of the camelid natural library in Example 2.1. After screening, two candidate molecules, 34 and 501, were finally selected for subsequent development. Their sequences were as follows:
> 34 
HVQLVESGGGLVQPGGSLRLSCAASGFAFSTYAMYWVRQLPGKGLEWVSTINS
DGSSTRYAGSVKGRFTISRDNAKNTLYLQLNSLKTEDTAMYYCAKGWISSPVW
GDYVPPVRGQGTQVTVSA (SEQ ID NO: 23)
> 501

QVQLVESGGGLVQAGGSLRLSCTVSGFPVDAYPMGWFRQTPGKEREWLSCINW
NGGRTQYGDSVKGRFTISRDNAKDTLYLQMNSLKAEDTAMYYCAALYACDSA
SSPSRRDWGQGTQVTVSS (SEQ ID NO: 24)

**Table 9. The CDRs of 34 and 501 (Kabat numbering scheme)**

| Antibody No. | CDR sequences | | Sequence No. |
|---|---|---|---|
| 34 | CDR1 | TYAMY | SEQ ID NO:25 |
| | CDR2 | TINSDGSSTRYAGSVKG | SEQ ID NO:26 |
| | CDR3 | GWISSPVWGDYVPPV | SEQ ID NO:27 |
| 501 | CDR1 | AYPMG | SEQ ID NO:28 |
| | CDR2 | CINWNGGRTQYGDSVKG | SEQ ID NO:29 |
| | CDR3 | LYACDSASSPSRRD | SEQ ID NO:30 |

### 3. Cell binding verification of candidate molecules

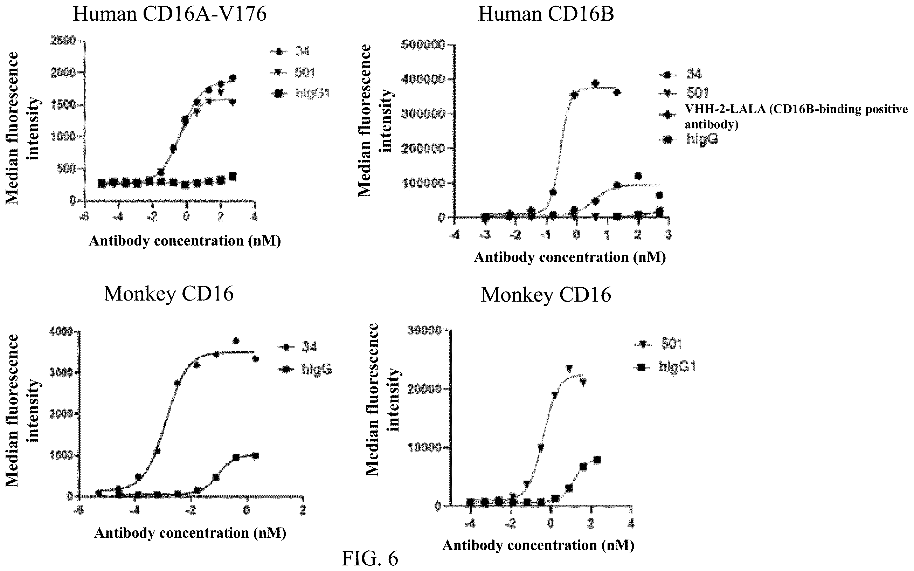

The abilities of the candidate molecules to bind to a CHO cell line overexpressing human CD16A-V176 (human CD16A-V176 protein sequence: AAH36723.1), CD16B (human CD16B protein sequence: 075015.2), or cynomolgus monkey CD16 (monkey CD16 sequence: NP_001270121.1) were assessed. A VHH-2 positive antibody with a high affinity for human CD16B was selected as a control (from Patent No. US20190276554), and the candidate molecules were found to have sub-nM-level to nM-level abilities to bind to both human CD16A and monkey CD16 but exhibit very weak binding to human CD16B (FIG. 6).

The above VHH-2 was linked to a human IgG1-Fc sequence with mutations C220A, L234A, and L235A (according to the Eu numbering scheme) (the mutations are underlined), with the VHH-2 placed at the N-terminus of the Fc, and the resulting sequence was designated VHH-2-LALA:
> Human CD16B positive antibody VHH-2-LALA 
EVQLLESGGGLVQPGGSLRLSCAASGLTFSSYNMGWVRQAPGKGLEWVASITW
SGRDTFYADSVKGRFTISRDNSKNTLYLQMNSLRAEDTAVYYCAANPWPVAAP
RSGTYWGQGTLVTVSSEPKSADKTHTCPPCPAPEAAGGPSVFLFPPKPKDTLMISR
TPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSC
SVMHEALHNHYTQKSLSLSPGK (SEQ ID NO: 31)
> The amino acid sequence of human CD16A-V176 (AAH36723.1): 
MWQLLLPTALLLLVSAGMRTEDLPKAVVFLEPQWYRVLEKDSVTLKCQGAYSP
EDNSTQWFHNESLISSQASSYFIDAATVDDSGEYRCQTNLSTLSDPVQLEVHIG
WLLLQAPRWVFKEEDPIHLRCHSWKNTALHKVTYLQNGKGRKYFHHNSDFYIP
KATLKDSGSYFCRGLVGSKNVSSETVNITITQGLAVSTISSFFPPGYQVSFCLVMV
LLFAVDTGLYFSVKTNIRSSTRDWKDHKFKWRKDPQDK (SEQ ID NO: 32)
> Human CD16B (075015.2) 
MWQLLLPTALLLLVSAGMRTEDLPKAVVFLEPQWYSVLEKDSVTLKCQGAYSP
EDNSTQWFHNESLISSQASSYFIDAATVNDSGEYRCQTNLSTLSDPVQLEVHIG
WLLLQAPRWVFKEEDPIHLRCHSWKNTALHKVTYLQNGKDRKYFHHNSDFHIP
KATLKDSGSYFCRGLVGSKNVSSETVNITITQGLAVSTISSFSPPGYQVSFCLVMV
LLFAVDTGLYFSVKTNI (SEQ ID NO: 33)
> Cynomolgus monkey CD16 (NP_001270121.1)

MWQLLLPTALLLLVSAGMRAEDLPRAVVFLEPQWYRVLEKDRVTLKCQGAYSP
EDNSTRWFHNESLISSQTSSYFIAAARVNNSGEYRCQTSLSTLSDPVQLEVHIGW
LLLQAPRWVFKEEESIHLRCHSWKNTLLHKVTYLQNGKGRKYFHQNSDFYIPK
ATLKDSGSYFCRGLIGSKNVSSETVNITITQDLAVSSISSFFPPGYQVSFCLVMVLL

FAVDTGLYFSMKKSIPSSTRDWEDHKFKWSKDPQDK (SEQ ID NO: 34)

### 4. Humanization of candidate molecules

The anti-human CD16A antibodies 34 and 501 were engineered by humanization, and their binding activity was subsequently identified on a cell line overexpressing human CD16A. The humanization templates for 34 were all IGHV3-23*04, and several back mutations were performed on the templates. The humanization templates for 501 were all IGHV3-20*04, and several back mutations were performed on the templates. The humanized amino acid sequences of 34 and 501 were as follows:
> 34H1 
EVQLVESGGGLVQPGGSLRLSCAASGFTFSTYAMYWVRQAPGKGLEWVSTINS
DGSSTRYAGSVKGRFTISRDNSKNTLYLQMNSLRAEDTAVYYCAKGWISSPVW
GDYVPPVWGQGTMVTVSS (SEQ ID NO: 35)
> 34H2 
HVQLVESGGGLVQPGGSLRLSCAASGFTFSTYAMYWVRQAPGKGLEWVSTINS
DGSSTRYAGSVKGRFTISRDNSKNTLYLQMNSLRAEDTAVYYCAKGWISSPVW
GDYVPPVWGQGTMVTVSS (SEQ ID NO: 36)
> 34H3 
EVQLVESGGGLVQPGGSLRLSCAASGFTFSTYAMYWVRQLPGKGLEWVSTINS
DGSSTRYAGSVKGRFTISRDNSKNTLYLQMNSLRAEDTAMYYCAKGWISSPVW
GDYVPPVWGQGTMVTVSS (SEQ ID NO: 37)
> 34H4 
HVQLVESGGGLVQPGGSLRLSCAASGFAFSTYAMYWVRQAPGKGLEWVSTINS
DGSSTRYAGSVKGRFTISRDNSKNTLYLQMNSLRAEDTAVYYCAKGWISSPVW
GDYVPPVWGQGTMVTVSS (SEQ ID NO: 38)
> 34H5 
HVQLVESGGGLVQPGGSLRLSCAASGFAFSTYAMYWVRQLPGKGLEWVSTINS
DGSSTRYAGSVKGRFTISRDNSKNTLYLQMNSLRAEDTAMYYCAKGWISSPVW
GDYVPPVWGQGTMVTVSS (SEQ ID NO: 39)
> 501V1 
EVQLVESGGGVVRPGGSLRLSCAASGFTFDAYPMGWFRQAPGKGLEWVSCIN
WNGGRTQYGDSVKGRFTISRDNAKNSLYLQMNSLRAEDTALYYCAALYACDSA
SSPSRRDWGQGTMVTVSS (SEQ ID NO: 40)
> 501V2 
EVQLVESGGGVVRPGGSLRLSCAASGFPVDAYPMGWFRQAPGKGLEWVSCIN
WNGGRTQYGDSVKGRFTISRDNAKNSLYLQMNSLRAEDTALYYCAALYACDSA
SSPSRRDWGQGTMVTVSS (SEQ ID NO: 41)
> 501V3 
EVQLVESGGGVVRPGGSLRLSCAASGFPVDAYPMGWFRQAPGKEREWVSCIN
WNGGRTQYGDSVKGRFTISRDNAKNSLYLQMNSLRAEDTALYYCAALYACDSA
SSPSRRDWGQGTMVTVSS (SEQ ID NO: 42)
> 501-V3NQ

EVQLVESGGGVVRPGGSLRLSCAASGFPVDAYPMGWFRQAPGKGLEWVSCIN
WQGGRTQYGDSVKGRFTISRDNAKNSLYLQMNSLRAEDTALYYCAALYACDSA
SSPSRRDWGQGTMVTVSS (SEQ ID NO: 43)

The CDR1 of 501-V3NQ is set forth in SEQ ID NO: 28, its CDR2 was CINWQGGRTQYGDSVKG (SEQ ID NO: 44), and its CDR3 is set forth in SEQ ID NO: 30.

### 5. Cell binding identification of humanized candidate molecules

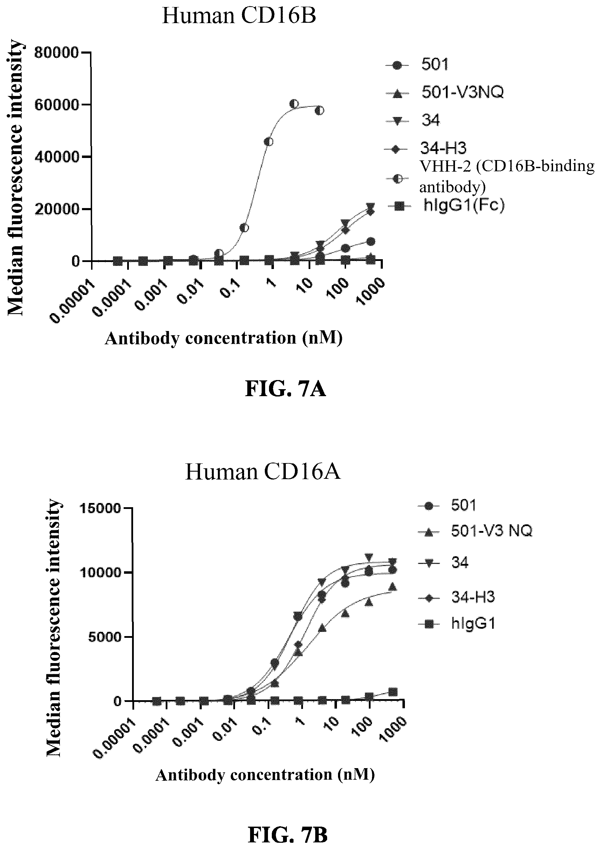

The above antibodies were each linked to human IgG1-Fc. Transient transfection and expression were performed, followed by cell binding identification. 34H3 and 501-V3NQ were selected for subsequent development and diabody combination. The cell-binding results for 34H3 and 501-V3NQ are shown in FIGs. 7A and 7B and are similar to those for the parent antibodies before humanization.

### Example 6. Preparation of CLDN18.2/4-1BB Multifunctional Antibodies

### 1. Design, expression, and purification of CLDN18.2/4-1BB bispecific antibodies (CLDN18.2 x 4-1BB)

Based on the humanized anti-4-1BB nanobody screening results, the C5_V2 clone and CLDN18.2 mAb sequence (from WO2020200196A1) were selected to construct a CLDN18.2 x 4-1BB bispecific antibody. The sequences of the CLDN18.2 mAb 1903 were as follows:
> CLDN18.2 mAb 1903 heavy chain (SEQ ID NO: 45) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
> CLDN18.2 mAb 1903 light chain (SEQ ID NO: 46)

DIVLTQSPDSLAVSLGERATINCKSSQSLLNSGNQKNYLTWYQQKPGQPPKLLIY
WASTRESGVPDRFSGSGSGTDFTLTISSLQAEDVAVYYCQNAYTYPFTFGQGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

The heavy chain constant region in the heavy chain and the light chain constant region in the light chain are italicized.

The CDRs of 1903, as defined according to the Kabat numbering scheme, were as follows:
the HCDR1 was SYWMH (SEQ ID NO: 57), the HCDR2 was MIHPNSGSTNYNEKFKG (SEQ ID NO: 58), and the HCDR3 was LKTGNSFDY (SEQ ID NO: 59);
the LCDR1 was KSSQSLLNSGNQKNYLT (SEQ ID NO: 60), the LCDR2 was WASTRES (SEQ ID NO: 61), and the LCDR3 was QNAYTYPFT (SEQ ID NO: 62).

The sequences of the heavy and light chain variable regions and the Fc segment of the antibody were as follows:
> The VH of 1903 (SEQ ID NO: 63) EVQLVQSGAEVKKPGASVKVSCKASGYTFTSYWMHWVRQAPGQRLEWMGMI 
HPNSGSTNYNEKFKGRVTITRDTSASTAYMELSSLRSEDTAVYYCARLKTGNSF
DYWGQGTTVTVSS
> The VL of 1903 (SEQ ID NO: 64) 
DIVLTQSPDSLAVSLGERATINCKSSQSLLNSGNQKNYLTWYQQKPGQPPKLLIY
WASTRESGVPDRFSGSGSGTDFTLTISSLQAEDVAVYYCQNAYTYPFTFGQGTKL
EIK
> The Fc of IgG1 (containing mutations S239D and I332E) (SEQ ID NO: 65)

EPKSCDKTHTCPPCPAPELLGGPDVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPEEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMH
EALHNHYTQKSLSLSPGK

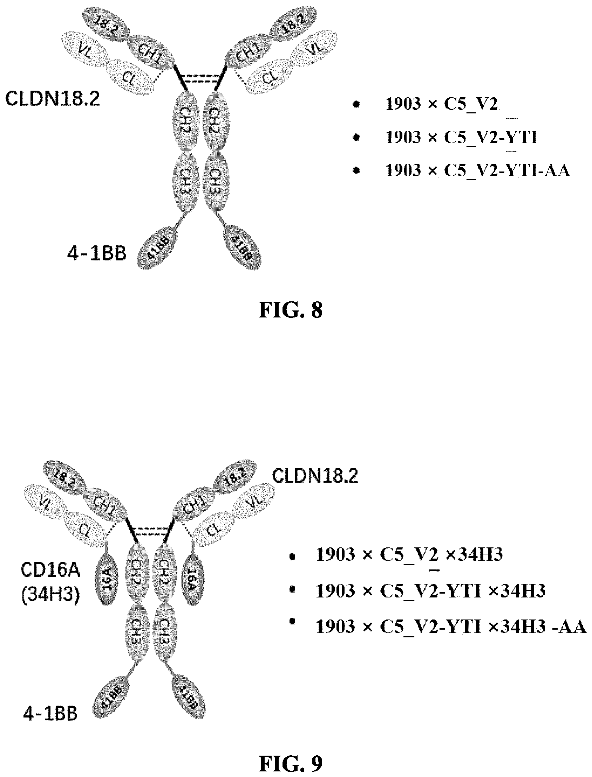

The bispecific antibody molecule had the above CLDN18.2 mAb sequence as its framework and had mutations S239D/I332E in its Fc. One C5_V2 was fused to the C-terminus of each of the two heavy chains of CLDN18.2 using a linker of (G₄S)₂ to obtain a bispecific antibody molecule, 1903 x C5_V2. Taking 1903 x C5_V2 as an example, the naming convention for the molecules in the present disclosure is as follows: 1903 represents the variable regions of the CLDN18.2 mAb, and C5_V2 is the clone number of the 4-1BB nanobody. C5_V2 was replaced with C5_V2-YTI, which was obtained by TCE removal, to obtain a bispecific antibody molecule, 1903 x CS V2-YTI. The structure of the antibody is shown in FIG. 8. The above 1903 x C5_V2-YTI bispecific antibody molecule was further optimized by removing pre-ADA by changing two amino acid residues at the C-terminus of C5_V2-YTI. The optimized bispecific antibody molecule was designated 1903 x C5 _V2-YTI-AA.

The amino acid sequences of the aforementioned bispecific antibody molecules were as follows:
> 1903 x C5_V2 heavy chain (SEQ ID NO: 47) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTFTIATMNDYDYWGQGTLVTVSS
> 1903 x C5_V2-YTI heavy chain (SEQ ID NO: 48) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
 
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTYTIATMNDYDYWGQGTLVTVSS
> 1903 x C5_V2-YTI-AA heavy chain (SEQ ID NO: 49)

EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTYTIATMNDYDYWGQGTLVTVSAA

In the above heavy chains, the heavy chain constant region is italicized, and the linker is underlined. The amino acid sequences of the light chains of 1903 x C5_V2, 1903 x C5_V2-YTI, and 1903 x C5_V2-YTI-AA are all set forth in SEQ ID NO: 46.

Transient transfection and expression: Taking a 30-mL expression system as an example, 60 µL of transfection reagent was diluted with culture solution and then mixed with 15 µg of plasmids. After 15 min of incubation at 37 °C, the mixed transfection solution was added dropwise while the cell suspension was shaken. After one week of culture on a shaker for expression, the supernatant was collected and centrifuged at 8000 rpm for 5 min.

Antibody purification: First, a Protein A affinity chromatography column was equilibrated with 20 mL of 1× PBS at a flow rate of 1 mL/min. The sample was loaded at a flow rate of 1 mL/min. 20 mL of 1× PBS was passed through the column at a flow rate of 1 mL/min to remove impurities. Elution was performed with a citrate buffer (pH 3.4) at 1 mL/min, and the eluate was collected in tubes. The absorbance at 280 nm was measured using a NanoDrop spectrophotometer. Finally, the high-concentration protein was transferred to a dialysis bag and dialyzed against 1× PBS in a beaker. Analysis showed that the antibody molecule of interest was obtained.

### 2. Design, expression, and purification of CLDN18.2/4-1BB/CD16A trispecific antibodies (CLDN18.2x4-1BBxCD16A)

Based on the humanized anti-4-1BB nanobody screening results, the C5_V2 clone was selected, and based on the humanized anti-CD16A nanobody screening results, the 34H3 clone was selected. The clones and the aforementioned CLDN18.2 mAb 1903 were used to construct a CLDN18.2 x 4-1BB x CD16A trispecific antibody.

The trispecific antibody molecule had the CLDN18.2 mAb 1903 as its framework, which is of the IgG1 subtype, and had mutations S239D/I332E in its Fc. One C5_V2 was fused to the C-terminus of each of the two heavy chains of CLDN18.2 using a linker of (G₄S)₂, and one 34H3 was fused to the C-terminus of each of the two light chains using a linker of (G₄S)₃ to construct a multispecific antibody molecule, 1903 x C5_V2 x 34H3. Taking 1903 x C5_V2 x 34H3 as an example, the naming convention for the molecule is as follows: 1903 means that the CLDN18.2 mAb uses the variable regions of 1903, C5_V2 is the clone number of the 4-1BB nanobody, and 34H3 is the clone number of the CD16A nanobody. C5_V2 was replaced with C5_V2-YTI, which was obtained by TCE removal, to construct a trispecific antibody molecule 1903 x C5_V2-YTI x 34H3. The structure of the antibody is shown in FIG. 9. The above 1903 x C5_V2-YTI x 34H3 trispecific antibody molecule was further optimized by removing pre-ADA by changing two amino acids at the C-terminus of each of C5_V2-YTI and 34H3. The optimized trispecific antibody molecule was designated 1903 x C5_V2-YTI x 34H3-AA.

The amino acid sequences of the aforementioned trispecific antibody molecules were as follows:
> 1903 x C5_V2 x 34H3 heavy chain (SEQ ID NO: 50) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTFTIATMNDYDYWGQGTLVTVSS
> 1903 x C5_V2 x 34H3 light chain (SEQ ID NO: 51) 
DIVLTQSPDSLAVSLGERATINCKSSQSLLNSGNQKNYLTWYQQKPGQPPKLLIY
WASTRESGVPDRFSGSGSGTDFTLTISSLQAEDVAVYYCQNAYTYPFTFGQGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGECGGGGS
GGGGSGGGGSEVQLVESGGGLVQPGGSLRLSCAASGFTFSTYAMYWVRQLPGK
GLEWVSTINSDGSSTRYAGSVKGRFTISRDNSKNTLYLQMNSLRAEDTAMYYC
AKGWISSPVWGDYVPPVWGQGTMVTVSS
> 1903 x C5_V2-YTI x 34H3 heavy chain (SEQ ID NO: 52) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
 KHPLTYTIATMNDYDYWGQGTLVTVSS
> 1903 x C5_V2-YTI x 34H3 light chain (SEQ ID NO: 51)
> 1903 x C5_V2-YTI x 34H3-AA heavy chain (SEQ ID NO: 53) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPDVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPE
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTYTIATMNDYDYWGQGTLVTVSAA
> 1903 x C5_V2-YTI x 34H3-AA light chain SEQ ID NO: 54)

DIVLTQSPDSLAVSLGERATINCKSSQSLLNSGNQKNYLTWYQQKPGQPPKLLIY
WASTRESGVPDRFSGSGSGTDFTLTISSLQAEDVAVYYCQNAYTYPFTFGQGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGECGGGGS
GGGGSGGGGSEVQLVESGGGLVQPGGSLRLSCAASGFTFSTYAMYWVRQLPGK
GLEWVSTINSDGSSTRYAGSVKGRFTISRDNSKNTLYLQMNSLRAEDTAMYYC
AKGWISSPVWGDYVPPVWGQGTMVTVSAA

In the above sequences, the heavy chain constant regions in the heavy chains and the light chain constant regions in the light chains are italicized, and the linkers are underlined. Transient transfection of cells and antibody expression and purification were performed using the methods in section 1 of Example 6 to obtain the antibody molecules of interest.

### Example 7. Antigen-Binding Activity of C5_V2-YTI

C5_V2, 1903 x C5_V2-LALA, and 1903 x C5_V2-YTI-LALA were compared in terms of antigen-binding activity to human 4-1BB. C5_V2 was linked to a human IgG1-Fc sequence with mutations C220A/S267E/L328F, and the resulting sequence is set forth in SEQ ID NO: 17. 1903 x C5_V2-LALA: The heavy chain was obtained by introducing mutations L234A/L235A into the Fc of the 1903 x C5_V2 heavy chain (SEQ ID NO: 47), and the Fc did not contain mutations S239D/I332E (SEQ ID NO: 55); the light chain is still set forth in SEQ ID NO: 46. 1903 x C5_V2-YTI-LALA: The heavy chain was obtained by introducing mutations L234A/L235A into the Fc of the 1903 x C5_V2-YTI heavy chain (SEQ ID NO: 48), and the Fc did not contain mutations S239D/I332E (SEQ ID NO: 56); the light chain is still set forth in SEQ ID NO: 46. The sequences were as follows:
> 1903 x C5_V2-LALA heavy chain (SEQ ID NO: 55) 
EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
 
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTFTIATMNDYDYWGQGTLVTVSS
> 1903 x C5_V2-YTI-LALA heavy chain (SEQ ID NO: 56)

EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
GGGGSGGGGSQVQLVESGGGLVQPGGSLRLSCSASGFTFSSYAMSWVRQAPGK
GLEWVSDINSGGESTFYEDSVKGRFTISRDNAKNTLYLQMNSLRAEDTAVYYCA
KHPLTYTIATMNDYDYWGQGTLVTVSS

In the above heavy chains, the heavy chain constant region is italicized, and the linker is underlined. The sequences of the light chains of 1903 x C5_V2-LALA and 1903 x C5_V2-YTI-LALA are both set forth in SEQ ID NO: 46.

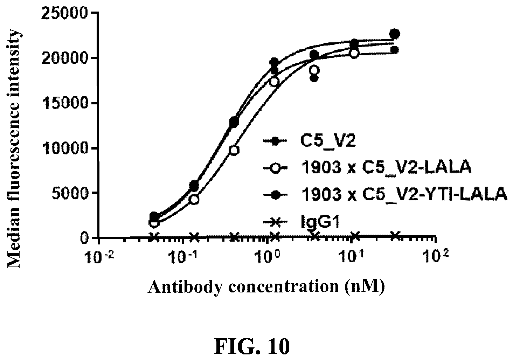

The binding activity of the antibodies obtained after the engineering of C5_V2-YTI to human 4-1BB protein was measured using a FACS assay. HEK293-Hu4-1BB cells were HEK293 cells overexpressing human 4-1BB protein. The culture medium for the cells was a DMEM culture medium (Gibco, Cat#11965092) containing 10% fetal bovine serum and 100 µg/mL hygromycin B. After being thawed, the cells were passaged to adjust their state. The experimental culture medium was a sterile PBS (phosphate-buffered saline, pH 7.40) containing 2% fetal bovine serum. The HEK293-Hu4-1BB cells were washed with the experimental culture medium twice and seeded into a 96-well plate at 1 × 10⁵ cells/well, and test samples at different concentrations were added. The cells were incubated at 4 °C for 1 h and then washed with the experimental culture medium twice. Subsequently, the Alexa Fluor 647-mouse anti-human (IgG, Fcy fragment specific) antibody (Jackson, Cat#209-605-098) was added. After two washes, fluorescence signal values were read using a flow cytometer. The results are shown in FIG. 10 and Table 10. The FACS assay results show that the related 1903 x C5_V2-YTI-LALA antibody exhibited a strong ability to bind to 4-1BB on the surface of HEK293-Hu4-1BB cells, and that the ability was comparable to those of 1903 x C5_V2-LALA and C5V2, indicating that C5_V2 and C5_V2-YTI have the same ability to bind to 4-1BB.

**Table 10. The results of the binding of the antibodies obtained after the engineering of C5_V2-YTI to HEK293-Hu4-1BB cells**

| Antibody name | EC₅₀ (nM) | Maximal fluorescence value |
|---|---|---|
| C5_V2 | 0.30 | 21244 |
| 1903 x C5_V2-LALA | 0.47 | 22546 |
| 1903 x C5_V2-YTI-LALA | 0.33 | 22561 |
| IgG1 | - | 123 |

| | | |
|---|---|---|
| (Note: "-" indicates that the measurement was beyond the limit of detection) | | |

### Example 8. 4-1BB/NF-κB Luciferase Reporter Gene Assay of C5_V2-YTI

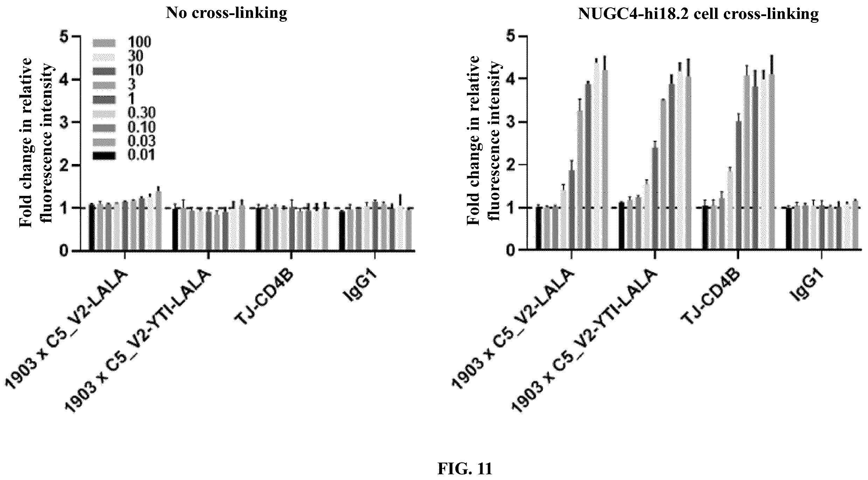

The agonist activity of the antibodies was assessed using a 4-1BB/NF-κB reporter gene. HEK293-Hu4-1BB/NF-xB double-transfected cells were obtained by transient transfection of HEK293 cells (ATCC CRL-1573) with a gene expressing human 4-1BB (CD137 cDNA ORF Clone, Human, C-OFPSpark tag; Sino Biological, Cat#HG10041-ACR) and the NF-κB genome (pGL4.32[luc2P/NF-κB-RE/Hygro] Vector, Promega, Cat#E849A). Hu4-1BB activation can be characterized by the activation level of the NF-κB signaling pathway. NUGC4-hi18.2 cells were obtained by stably transfecting NUGC4 cells with a gene expressing human CLDN18.2 protein. The culture medium for the cells was an RPMI1640 culture medium (Gibco, Cat#10491A-01) containing 10% inactivated fetal bovine serum and 10 µg/mL puromycin. The HEK293-Hu4-1BB/NF-κB cells (2 × 10⁶/mL) were plated onto a 96-well cell culture plate at 50 µL, 40 µL of culture medium or CLDN18.2-expressing NUGC4 cells (2.5 × 10⁶/mL) was added, and 10× serially diluted test antibodies were added at 10 µL/well. The plate was incubated at 37 °C for 6 h. The cells were taken out. An equal volume of Bio-Glo Luciferase Assay System reagent (Promega, Cat#G7940) was added to each well, and the plate was incubated for 5 min in a dark place. Fluorescence signals were measured using an Envision microplate reader. EC₅₀ values and Emax values (relative to the fluorescence intensity of the antibody-free group) were calculated, and the agonist activity of the anti-4-1BB antibodies on cells *in vitro* was assessed. The results are shown in FIG. 11 and Table 11. The results show that the related 1903 x C5_V2-YTI-LALA antibody did not exhibit background activation, but after cross-linking through CLDN18.2, its agonistic activity on 4-1BB was comparable to that of 1903 x C5_V2-LALA, indicating that C5_V2 and C5_V2-YTI have the same ability to activate 4-1BB.

**Table 11. The results of the CLDN18.2-dependent 4-1BB/NF-κB luciferase reporter gene assay of the antibodies obtained after the engineering of C5_V2-YTI**

| Antibody name | No CLDN18.2 cross-linking | CLDN18.2 cross-linking | |
|---|---|---|---|
| | Fold change in Emax | EC₅₀ (nM) | Fold change in Emax |
| 1903 x C5_V2-LALA | 1.38 | 1.88 | 4.38 |
| 1903 x C5_V2-YTI-LALA | 1.07 | 1.21 | 4.18 |
| TJ-CD4B | 1.03 | 0.60 | 4.11 |
| IgG1 | 1.16 | - | 1.17 |

### Example 9. Assay for Antigen-Binding Affinities of CLDN18.2/4-1BB Multifunctional Antibodies

The affinities of the CLDN18.2/4-1BB multifunctional antibodies for antigens were measured using surface plasmon resonance (SPR). A CM5 sensor chip was used for the assay, and HBS-EP + buffer (10 mM HEPES, 150 mM NaCl, 3 mM EDTA, 0.05% surfactant P20) was used as the mobile phase. A 30 µg/mL solution of anti-human IgG (Fc) antibody was prepared in a 10 mM sodium acetate buffer (pH 5.0), and the Immobilization program was selected to automatically perform anti-human IgG (Fc) antibody channel amino coupling immobilization. The test antibodies were separately diluted in HBS-EP + buffer as ligands and captured using the anti-human IgG (Fc) antibody on the chip channels. Solutions of human 4-1BB protein (Acro Biosystems, 41B-H522a) and cynomolgus monkey 4-1BB protein (Acro Biosystems, 41B-C52H4) as antigens (i.e., analytes) were prepared in HBS-EP + buffer and serially diluted 2-fold, and the diluted antibodies were left to flow through the experimental and reference channels at a flow rate of 30 µL/min, with 1 min of binding and 15 min of dissociation. 10 mM Glycine pH1.5 (GE Healthcare, BR-1003-54) was selected as the regeneration buffer, and the chip was regenerated with the buffer for 30 s at a flow rate of 10 µL/min. The instrument used was Zeba Spin Desalting Columns (Thermo, 89882), and data were analyzed using Biacore 8K evaluation software.

The results are shown in Table 12 and show that the antigen-binding affinities of the related CLDN18.2 x 4-1BB multifunctional antibodies for human and cynomolgus monkey 4-1BB proteins were comparable to that of the parent 4-1BB VHH antibody C5_V2.

**Table 12. The Biacore affinities of the CLDN18.2/4-1BB multifunctional antibodies for 4-1BB (1:1 binding)**

| Antibody name | Antigen | ka (1/Ms) | kd (1/s) | KD (M) |
|---|---|---|---|---|
| 1903 x C5_V2-YTI | Human 4-1BB | 2.32E+05 | 4.52E-03 | 1.95E-08 |
| 1903 x C5_V2-YTI x 34H3 | | 2.30E+05 | 4.19E-03 | 1.82E-08 |
| C5_V2 | | 6.45E+05 | 7.82E-03 | 1.21E-08 |
| 1903 x C5_V2-YTI | Cynomolgus monkey 4-1BB | 1.07E+05 | 3.08E-03 | 2.89E-08 |
| 1903 x C5_V2-YTI x 34H3 | | 1.10E+05 | 3.14E-03 | 2.86E-08 |
| C5_V2 | | NA | NA | 4.69E-07 |

### Example 10. Assay for Antigen-Binding Activity of CLDN18.2/4-1BB Multifunctional Antibodies

The binding activity of the CLDN18.2/4-1BB multifunctional antibodies to human CLDN18.2/CD16A/4-1BB protein was measured using a FACS assay. NUGC4-hi18.2 cells were obtained by stably transfecting NUGC4 cells with a gene expressing human CLDN18.2 protein. The culture medium for the cells was an RPMI1640 culture medium (Gibco, Cat#10491A-01) containing 10% inactivated fetal bovine serum and 10 µg/mL puromycin. CHO-K1-CD16A V176 cells were CHO-K1 cells overexpressing human CD16A V176 protein. The culture medium for the cells was a Ham's F12 + GlutaMAX^{™}-I culture medium (Gibco, Cat#31765035) containing 10% fetal bovine serum and 200 µg/mL hygromycin B. HEK293-Hu4-1BB cells were HEK293 cells overexpressing human 4-1BB protein. The culture medium for the cells was a DMEM culture medium (Gibco, Cat#11965092) containing 10% fetal bovine serum and 100 µg/mL hygromycin B. After being thawed, the cells were passaged to adjust their state. The experimental culture medium was a sterile PBS (phosphate-buffered saline, pH 7.40) containing 2% fetal bovine serum. The NUGC4-hi18.2, CHO-K1-CD16A V158, or HEK293-Hu4-1BB cells were washed with the experimental culture medium twice and seeded into a 96-well plate at 1 × 10⁵ cells/well, and test samples at different concentrations were added. The cells were incubated at 4 °C for 1 h and then washed with the experimental culture medium twice. Subsequently, the Alexa Fluor 647-mouse anti-human (IgG, Fcy fragment specific) antibody (Jackson, Cat#209-605-098) was added. After two washes, fluorescence signal values were read using a flow cytometer. The results are shown in FIGs. 12A to 12C and Tables 13 to 15.

The FACS assay results show that the related CLDN18.2/4-1BB multifunctional antibodies exhibited strong abilities to bind to CLDN18.2 on the surface of NUGC44-hi18.2 cells, and that their binding abilities were comparable to those of the CLDN18.2 mAb 1903 and the control antibody TJ-CD4B and stronger than that of the control antibody IMAB362. The related CLDN18.2/4-1BB multifunctional antibodies exhibited good abilities to bind to CD16A V176 on the surface of CHO-K1-CD16AV176 cells, and the abilities were stronger than that of the control antibody IMAB362. The related CLDN18.2/4-1BB multifunctional antibodies exhibited strong abilities to bind to 4-1BB on the surface of HEK293-Hu4-1BB cells, and the abilities were comparable to that of the 4-1BB mAb C5_V2 and slightly stronger than that of TJ-CD4B.

**Table 13. The results of the binding of the CLDN18.2/4-1BB multifunctional antibodies to NUGC4-hi18.2 cells**

| Antibody name | EC₅₀ (nM) | Maximal fluorescence value |
|---|---|---|
| 1903 x C5_V2 | 8.62 | 79142 |
| 1903 x C5_V2 x 34H3 | 11.67 | 77184 |
| 1903 | 8.60 | 91300 |
| IMAB362 | 17.82 | 45926 |
| TJ-CD4B | 16.23 | 89349 |
| IgG1 | - | 107 |

**Table 14. The results of the binding of the CLDN18.2/4-1BB multifunctional antibodies to CHO-K1-CD16AV176 cells**

| Antibody name | EC₅₀ (nM) | Maximal fluorescence value |
|---|---|---|
| 1903 x C5_V2 | 2.13 | 3388 |
| 1903 x C5_V2 x 34H3 | 1.69 | 4797 |
| IMAB362 | 14.77 | 3389 |
| TJ-CD4B | - | 113 |
| 34H | 9.47 | 4357 |
| IgG1 | - | 1586 |

**Table 15. The results of the binding of the CLDN18.2/4-1BB multifunctional antibodies to HEK293-Hu4-1BB cells**

| Antibody name | EC₅₀ (nM) | Maximal fluorescence value |
|---|---|---|
| 1903 x C5_V2 | 0.44 | 11098 |
| 1903 x C5_V2 x 34H3 | 0.55 | 10592 |
| TJ-CD4B | 2.45 | 11898 |
| C5_V2 | 0.50 | 11208 |
| IgG1 | - | 106 |

| | | |
|---|---|---|
| (Note: "-" indicates that the measurement was beyond the limit of detection) | | |

### Example 11. Experiment of CLDN18.2/4-1BB Multifunctional Antibodies Inducing Antibody-Mediated Cell Killing (ADCC) In Vitro

The antibody-mediated ADCC activity of NK cells against CLDN18.2-expressing target cells was assessed using a lactate dehydrogenase (LDH) assay. NUGC4-hi18.2 cells were obtained by stably transfecting NUGC4 cells with a gene expressing human CLDN18.2 protein. The culture medium for the cells was an RPMI1640 culture medium (Gibco, Cat#10491A-01) containing 10% inactivated fetal bovine serum and 10 µg/mL puromycin. SNU601 cells were purchased from Nanjing Cobioer Biosciences (CBP60507). The culture medium for the cells was an RPMI1640 culture medium (Gibco, Cat#10491A-01) containing 10% fetal bovine serum. The cryopreserved PBMCs were isolated from fresh human blood, thawed, then cultured in an RPMI1640 culture medium (Gibco, Cat#10491A-01) containing 10% fetal bovine serum, and incubated overnight at 37 °C. The next day, the different target cells were digested and resuspended in a phenol red-free RPMI1640 culture medium (Gibco, Cat#11835-030) containing 2% fetal bovine serum, and the density was adjusted to 2 × 10⁵ cells/mL; subsequently, the cells were seeded into a 96-well plate at 50 µL/well, and 10× serially diluted test antibodies were added at 10 µL. The plate was incubated in a 5% CO₂ incubator at 37 °C for 0.5 h.

PBMCs were collected and resuspended in a phenol red-free RPMI1640 culture medium containing 2% fetal bovine serum. An appropriate effector-to-target cell ratio was used depending on the target cells, and the cell density was adjusted. The cells were seeded into the above assay plate at 40 µL/well, and the plate was incubated in a 5% CO₂ incubator at 37 °C for 4 h. The cell culture plate was taken out and centrifuged at 400 g for 5 min, and the cell culture supernatants were collected and assayed for LDH levels using a CytoTox 96^{®} Non-Radioactive Cytotoxicity Assay kit (Promega, G1780). The instructions of the reagent were referred to for specific operations.

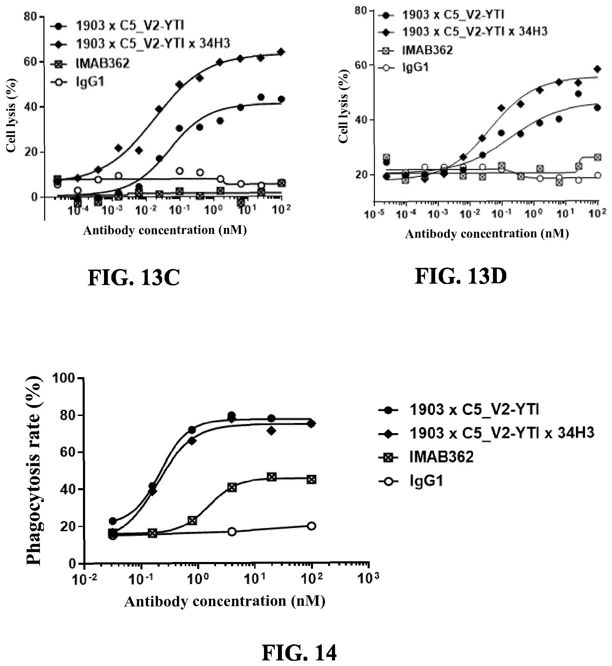

The results are shown in FIGs. 13A to 13D and Table 16 and show that the related CLDN18.2/4-1BB multifunctional antibodies exhibited good ADCC activity against target cells with different expression levels of CLDN18.2 and outperformed the control antibody IMAB362. SC190061(176F) is the PBMC donor number of the CD16A-F176 variant, and S2001102(176V) is the PBMC donor number of the CD16A-V176 variant.

**Table 16. The results of the CLDN18.2/4-1BB multifunctional antibody-induced killing of CLDN18.2-expressing target cells by NK cells**

| Target cell | NUGC4-hi18.2 | | | | SNU601 | | | |
|---|---|---|---|---|---|---|---|---|
| PBMC donor (CD16A variant) | SC190061 (F176) | | S2001102 (V176) | | SC190061 (F176) | | S2001102 (V176) | |
| Antibody name | EC₅₀ (nM) | Maximum killing rate (%) | EC₅₀ (nM) | Maximum killing rate (%) | EC₅₀ (nM) | Maximum killing rate (%) | EC₅₀ (nM) | Maximum killing rate (%) |
| 1903 x C5_V2-YTI | 0.015 | 80.25 | 0.012 | 70.18 | 0.050 | 44.01 | 0.189 | 49.25 |
| 1903 x C5_V2-YTI x 34H3 | 0.002 | 93.25 | 0.008 | 70.69 | 0.018 | 64.07 | 0.044 | 53.61 |
| IMAB362 | 0.218 | 72.99 | 0.094 | 70.18 | - | 5.88 | - | 25.96 |
| IgG1 | - | 10.70 | - | 21.76 | - | 11.39 | - | 22.68 |

| | | | | | | | | |
|---|---|---|---|---|---|---|---|---|
| (Note: "-" indicates that the measurement was beyond the limit of detection) | | | | | | | | |

### Example 12. Experiment of CLDN18.2/4-1BB Multifunctional Antibodies Inducing Antibody-Mediated Cell Phagocytosis (ADCP) In Vitro

PBMCs were isolated from fresh human blood, and CD14⁺ monocytes were then sorted using human CD14 microbeads (Miltenyi Biotec, 130-050-201). These CD14⁺ monocytes were cultured in a macrophage differentiation culture medium-an RPMI1640 culture medium (Gibco, Cat#10491A-01) containing 50 ng/mL recombinant human macrophage colony-stimulating factor (rhM-CSF, PeproTech, Cat#300-25) and 10% fetal bovine serum. After 6 days of differentiation, the macrophages became adhesive and had tentacles. The macrophages were trypsinized for 5 min, scraped gently with a scraper, and resuspended in an RPMI1640 culture medium containing 10% fetal bovine serum, and the density was adjusted to 4 × 10⁵ cells/mL. Subsequently, the cells were seeded into a 96-well plate at 100 µL/well and incubated overnight at 37 °C. The next day, NUGC4-hi18.2 cells were labeled with Cell Trace Far Red (Invitrogen, C34564) at 37 °C for 15 min, washed with PBS twice, and then added to the wells with macrophages at 50 µL/well in a macrophage-to-NUGC4-hi18.2 cell ratio of 1:5, and 4× serially diluted test antibodies were added at 50 µL. The target cells were subjected to a 4-hour phagocytosis process. After the phagocytosis process, the cells were washed with PBS three times and then stained with FITC anti-human/mouse CD11b (Tonbo, 35-0112-M100) in a certain ratio for 30 min. After two washes with PBS, analysis was performed by flow cytometry. Phagocytosis was measured by assessing the percentage of Far Red+/CD11b⁺ double positive cells after CD11b positive cell gating.

The results are shown in FIG. 14 and Table 17 and show that the related CLDN18.2/4-1BB multifunctional antibodies exhibited good ADCP function and outperformed the control antibody IMAB362.

**Table 17. The results of the CLDN18.2/4-1BB multifunctional antibody-induced phagocytosis of tumor cells by macrophages**

| Antibody name | EC₅₀ (nM) | Maximum phagocytosis rate (%) |
|---|---|---|
| 1903 x C5_V2-YTI | 0.22 | 79.6 |
| 1903 x C5_V2-YTI x 34H3 | 0.20 | 77.9 |
| IMAB362 | 1.57 | 46.1 |
| IgG1 | - | 19.8 |

| | | |
|---|---|---|
| (Note: "-" indicates that the measurement was beyond the limit of detection) | | |

### Example 13. CLDN18.2-Dependent 4-1BB/NF-κB Luciferase Reporter Gene Assay of CLDN18.2/4-1BB Multifunctional Antibodies

The CLDN18.2-dependent activation of 4-1BB/NF-xB signaling was measured using an NF-κB luciferase reporter gene assay as described in Example 8.

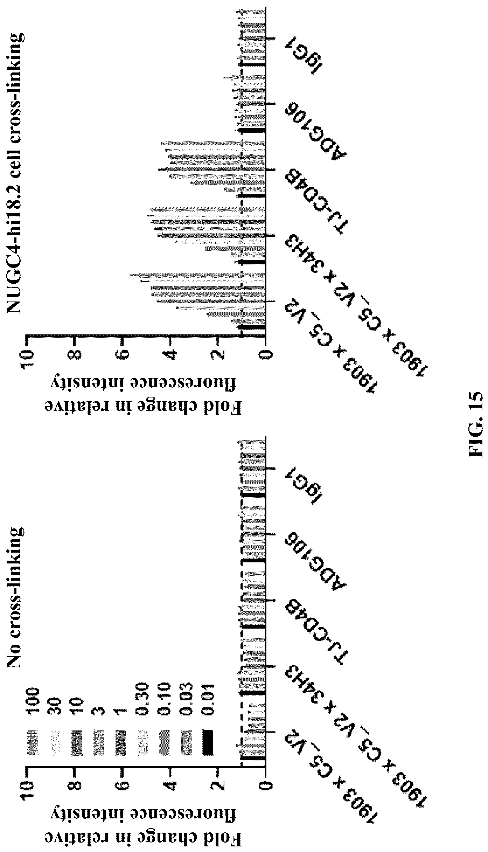

The results are shown in FIG. 15 and Table 18 and show that the related CLDN18.2/4-1BB multifunctional antibodies did not exhibit background activation and that their CLDN18.2-dependent agonistic activity on the 4-1BB/NF-xB signaling pathway was comparable to that of TJ-CD4B.

**Table 18. The results of the CLDN18.2-dependent 4-1BB/NF-κB luciferase reporter gene assay of the CLDN18.2/4-1BB multifunctional antibodies**

| Antibody name | No cross-linking | CLDN18.2 cross-linking | |
|---|---|---|---|
| | Fold change in Emax | EC₅₀ (nM) | Fold change in Emax |
| 1903 x C5_V2 | 1.07 | 0.16 | 5.28 |
| 1903 x C5_V2 x 34H3 | 1.10 | 0.13 | 4.79 |
| TJ-CD4B | 1.06 | 0.07 | 4.19 |
| ADG106 | 1.13 | - | 1.42 |
| IgGl | 1.17 | - | 1.15 |

| | | | |
|---|---|---|---|
| (Note: "-" indicates that the measurement was beyond the limit of detection) | | | |

### Example 14. T Lymphocyte Activation Assay

Freshly isolated and purified PBMCs were resuspended in an RPMI1640 culture medium (Gibco, Cat#10491A-01) containing 10% fetal bovine serum, and the density was adjusted to 1 × 10⁶ cells/mL. NUGC4-hi18.2 cells were obtained by stably transfecting NUGC4 cells with a gene expressing human CLDN18.2 protein. The culture medium for the cells was an RPMI1640 culture medium (Gibco, Cat#10491A-01) containing 10% inactivated fetal bovine serum and 10 µg/mL puromycin. After being thawed, the cells were passaged to adjust their state, and the density was adjusted to 1.25 × 10⁶ cells/mL. A 96-well plate was coated with 0.25 µg/mL anti-CD3 antibody OKT3 (Invitrogen, Cat#16-0037-85) at 100 µL/well, incubated at 37 °C for 2 h, and then washed with PBS to remove the antibody residue. Subsequently, 100 µL of PBMCs (1 × 10⁵ cells/well) and 80 µL of culture medium or NUGC4-hi18.2 cells (1 × 10⁵ cells/well) were added to each well of the OKT3-coated 96-well plate, and 10× test samples at different concentrations were added at 20 µL/well. The plate was incubated in a 5% CO₂ incubator at 37 °C for 3 days. The cell culture plate was taken out and centrifuged at 400 g for 5 min, and the cell culture supernatants were collected and assayed for IL-2 levels using a human IL-2 assay kit (Cisbio, Cat#62HIL02PEG). The instructions of the reagent were referred to for specific operations.

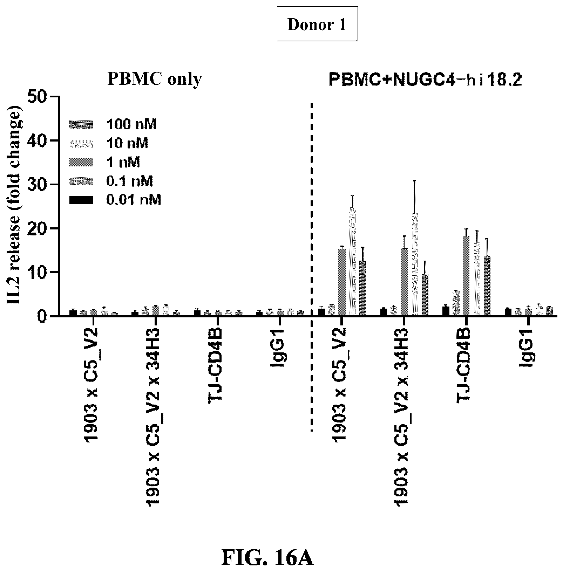

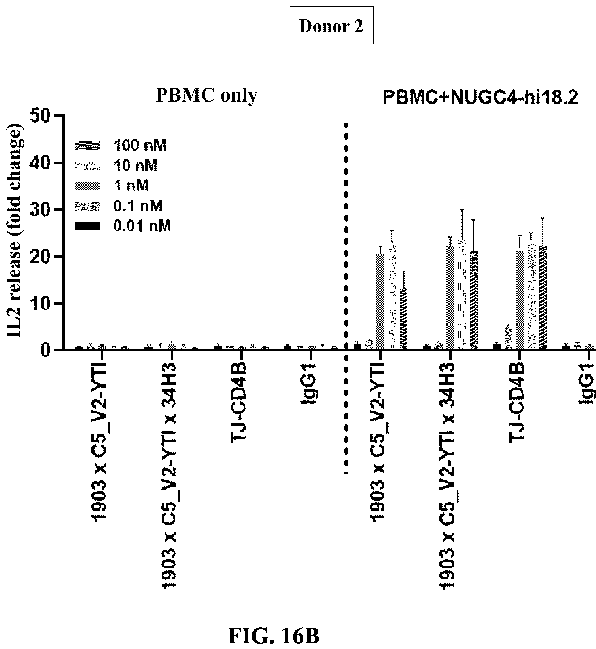

The results are shown in FIGs. 16A and 16B and show that in the two PBMC donors, after CLDN18.2 cross-linking, the related CLDN18.2/4-1BB multifunctional antibodies both activated IL-2 secretion and were comparable to the control antibody TJ-CD4B.

### Example 15. Experiment of NK Cells Killing T Cells

PBMCs were isolated from fresh human blood, and T cells were then sorted using an EasySep^{™} Human T Cell Enrichment Kit (Stemcell, Cat#17951). A T75 culture flask (Corning, Cat#430641) was coated with 0.25 µg/mL anti-CD3 antibody OKT3 (Invitrogen, Cat#16-0037-85), incubated at 37 °C for 2 h, and then washed with PBS to remove the antibody residue. Freshly isolated T cells were resuspended in an RPMI1640 culture medium (Gibco, Cat#10491A-01) containing 10% fetal bovine serum and seeded into the OKT3-coated T75 culture flask, and the density was adjusted to 2 × 10⁶ cells/mL. After 48 h of incubation, the activated T cells were labeled with CellTrace Far Red (Invitrogen, C34564) at 37 °C for 15 min, washed with PBS twice, and then resuspended in a phenol red-free RPMI1640 culture medium (Gibco, Cat#11835-030) containing 2% fetal bovine serum, and the density was adjusted to 2 × 10⁵ cells/mL. Subsequently, the cells were seeded into a 96-well plate at 50 µL/well, and 10× serially diluted test antibodies were added at 10 µL. The plate was incubated in a 5% CO₂ incubator at 37 °C for 0.5 h.

Freshly thawed PBMCs from the same donor were collected and resuspended in a phenol red-free RPMI1640 culture medium containing 2% fetal bovine serum, and the cell density was adjusted in an effector-to-target cell ratio of PBMCs to activated T cells of 25:1. The fresh PBMCs were seeded into the above assay plate at 40 µL/well, and the plate was incubated in a 5% CO₂ incubator at 37 °C for 6 h. After the incubation, the cell culture plate was taken out, and PI (Absin, abs9358) was added in a certain ratio, followed by 10 min of staining. After two washes with PBS, analysis was performed by flow cytometry. The killing of activated T cells by NK cells among PBMCs was measured by assessing the percentage of PI⁺/Far Red⁺ double positive cells after Cell Trace Far Red positive gating.

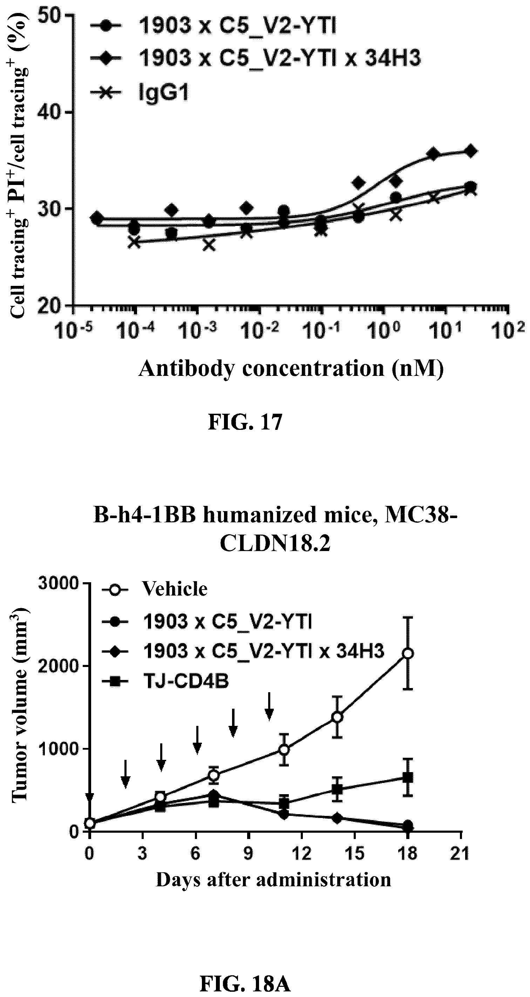

The results are shown in FIG. 17 and show that the related CLDN18.2 x 4-1BB bispecific antibody 1903 x C5_V2-YTI did not induce observable killing of T cells by NK cells and was comparable to the isotype control IgG1, and that the related CLDN18.2 x 4-1BB x CD16A multispecific antibody 1903 x C5_V2-YTI x 34H3 only exhibited induction of slight killing of T cells by NK cells.

### Example 16. Pharmacodynamic, Pharmacokinetic, and Safety Evaluations

### 1. In vivo pharmacodynamic study of CLDN18.2/4-1BB multifunctional antibodies in mouse model of colon cancer MC38-hCLDN18.2

MC38-hCLDN18.2 cells (provided by Biocytogen) were inoculated subcutaneously into B-h4-1BB humanized mice (provided by Biocytogen) at 2 × 10⁵ cells/100 µL/mouse. When tumors grew to about 102 mm³, 24 mice were selected according to tumor volume and randomized into 4 groups of 6: a vehicle group, a 1903 x C5_V2-YTI (0.26 mg/kg) group, a 1903 x C5_V2-YTI x 34H3 (0.3 mg/kg) group, and a TJ-CD4B (0.3 mg/kg) group. The mice were dosed once every two days. During dosing and observation periods, tumor volume was measured twice weekly, and the measurements were recorded.

Tumor volume (TV) was calculated by the formula TV = 1/2 × a × b², where a and b represent the long and short diameters of the measured tumor, respectively. Relative tumor proliferation rate T/C% = (T - T0)/(C - C0) × 100%; tumor growth inhibition rate TGI% = 1 - T/C%. CR% (the proportion of mice with complete tumor regression) = the number of mice with complete tumor regression (<102 mm3)/the number of mice in the group.

The results are shown in FIG. 18A and Table 19 and show that the related CLDN18.2/4-1BB multifunctional antibodies exhibited good antitumor activity and were superior to the control antibody TJ-CD4B.

**Table 19. The antitumor effects of different CLDN18.2/4-1BB multifunctional antibodies on mouse xenograft tumors**

| Group | Administration regimen (dose) | TGI (%) | p value | CR% (<102 mm³) |
|---|---|---|---|---|
| G1 | Vehicle (-) | - | - | - |
| G2 | 1903 x C5_V2-YTI (0.26 mg/kg) | 102.8 | 0.0006 | 83.3 |
| G3 | 1903 x C5_V2-YTI x 34H3 (0.3 mg/kg) | 101.0 | 0.0007 | 66.7 |
| G4 | TJ-CD4B (0.3 mg/kg) | 72.9 | 0.0118 | 16.7 |

### 2. Toxicity test of CLDN18.2/4-1BB multifunctional antibodies in mouse model of colon cancer MC38-hCLDN18.2

CLDN18.2/4-1BB multifunctional antibody cells (provided by Biocytogen) were inoculated subcutaneously into B-h4-1BB humanized mice (provided by Biocytogen) at 2 × 10⁵ cells/ 100 µL/mouse. When tumors grew to about 102 mm³, 24 mice were selected according to tumor volume and randomized into 4 groups of 6: a vehicle group, a 1903 x C5_V2-YTI (0.26 mg/kg) group, a 1903 x C5V2-YTI x 34H3 (0.3 mg/kg) group, and a TJ-CD4B (0.3 mg/kg) group. The mice were dosed once every two days. During dosing and observation periods, the mice were weighed twice weekly, and the measurements were recorded. On day 17, blood was collected and assayed for AST and ALT.

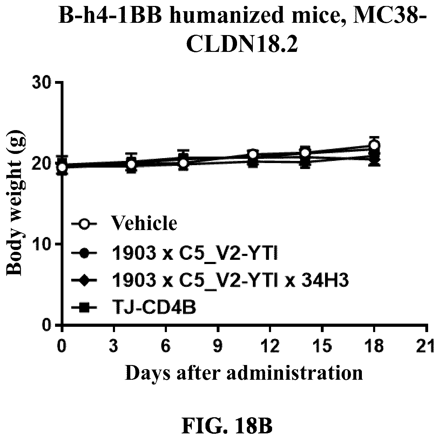

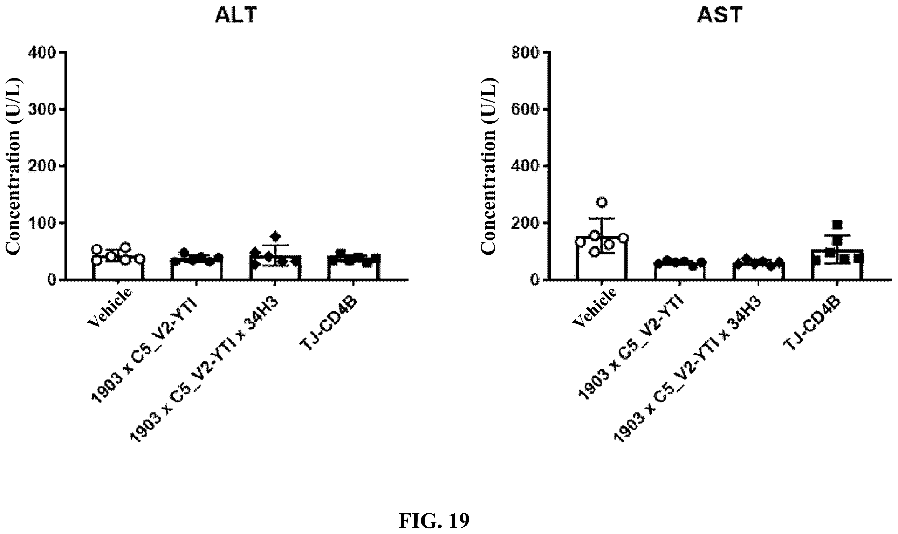

The results are shown in FIGs. 18B and 19. The results show that the weight of the mice was stable during the administration period, suggesting that the related CLDN18.2/4-1BB multifunctional antibodies have no significant toxic or side effects. The day-17 ALT/AST results show that the related CLDN18.2/4-1BB multifunctional antibodies did not exhibit significant hepatotoxicity at effective antitumor doses.

### 3. Single-dose pharmacokinetic study of CLDN18.2/4-1BB multifunctional antibody in mouse model of colon cancer MC38-hCLDN18.2

MC38-hCLDN18.2 cells were inoculated subcutaneously into B-h4-1BB humanized mice (provided by Biocytogen) at 5 × 10⁵ cells/100 µL/mouse. When tumors grew to about 110 mm³, 6 mice were selected according to tumor volume and randomized into 2 groups of 3: a 1903 (10 mg/kg) group and a 1903 x C5_V2 (12 mg/kg) group. The mice were dosed once by intravenous injection.

From the above laboratory mice, blood was collected at the following time points: 0.25 h, 6 h, 24 h, 72 h, 144 h, 240 h, 336 h, 408 h, and 504 h post-dose. The plasma concentration was determined using an ELISA assay. The bottom of a plate was coated with goat anti-human IgG, Fc (Rockland, 609-101-017), and the plate was incubated at 4 °C for 14-18 h. After three washes with 300 µL of PBS, a blocking reagent containing 3% BSA was added, and the plate was incubated at room temperature for 1 h. After three washes with 300 µL of PBS, the test serum samples were added, and the plate was incubated at room temperature for 2 h. After three washes with 300 µL of PBS, 50 µL of goat anti-human IgG-HRP (BETHYL, A80-304P) solution was added to each well. The plate was sealed with a plate-sealing film and incubated at room temperature for 1 h. The secondary antibody detection concentration was 1:20,000, and the diluent was 1% BSA-PBS + 0.5% mouse serum.

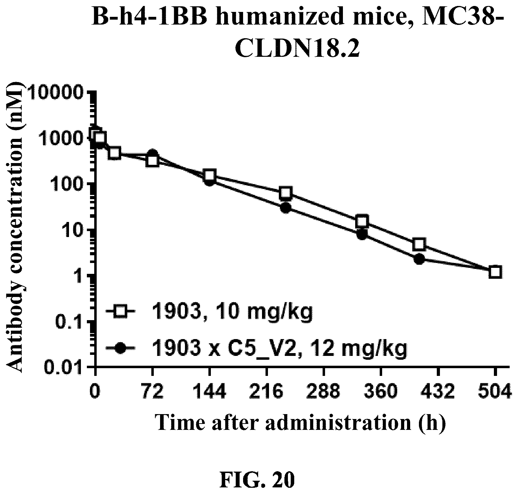

The results are shown in FIG. 20 and Table 20. The related CLDN18.2/4-1BB multifunctional antibody exhibited similar pharmacokinetic parameters to the mAb, based mainly on the CLDN18.2-end-mediated antibody distribution. The multifunctional antibody exhibited a similar PK profile to the equimolar dose of the CLDN18.2 parent mAb 1903, indicating that the mouse plasma concentration of the related CLDN18.2/4-1BB multifunctional antibody was not affected by the 4-1BB end; therefore, the multifunctional antibody exhibited the typical pharmacokinetic profile of a CLDN18.2-targeting antibody.

**Table 20. Pharmacokinetic parameters of the CLDN18.2/4-1BB multifunctional antibody in h4-1BB transgenic mice**

| | | |
|---|---|---|
| Antibody name | 1903 | 1903 x C5_V2 |
| Dose (mg/kg) | 10 | 12 |
| T_{1/2} (h) | 45.54 ± 4.93 | 53.84 ± 4.89 |
| Cₘₐₓ (nM) | 1280.74±331.96 | 1449.35±90.78 |
| Cₘₐₓ (µg/mL) | 184.43 ± 47.80 | 208.71 ± 13.07 |
| AUCall (h*µg/mL) | 10360.70 ± 130.71 | 10996.91 ± 927.20 |
| Cl_F_obs(mL/h/kg) | 0.96 ± 0.01 | 1.00 ± 0.02 |
| Cl_F_obs(mL/day/kg) | 23.12 ± 0.27 | 23.92 ± 0.56 |

| | | |
|---|---|---|
| (mean ± standard deviation) | | |

## Claims

1. A 4-1BB-binding protein, comprising an immunoglobulin single variable domain, wherein the immunoglobulin single variable domain comprises:
a CDR1, a CDR2, and a CDR3 from the amino acid sequence set forth in any one of SEQ ID NOs: 10 and 18-21, wherein the CDR1, CDR2, and CDR3 are defined according to the Kabat, IMGT, Chothia, AbM, or Contact numbering scheme;
preferably, the amino acid sequences of the CDR1, CDR2, and CDR3 of the immunoglobulin single variable domain are set forth in SEQ ID NOs: 11, 12, and 13 or SEQ ID NOs: 11, 12, and 22, respectively.

2. The 4-1BB-binding protein according to claim 1, wherein the immunoglobulin single variable domain is engineered by humanization, affinity maturation, T cell epitope removal, reduction of antibody deamidation, and/or reduction of antibody isomerization; preferably, heavy chain framework regions of a human germline template used in the engineering by humanization are IGHV3-64*04, IGHV3-23*03, and/or IGHV3-74*01.

3. The 4-1BB-binding protein according to claim 1 or 2, wherein the amino acid sequence of the immunoglobulin single variable domain is set forth in any one of SEQ ID NOs: 10 and 18-21 or has at least 80% or at least 90% sequence identity thereto;
preferably, the 4-1BB-binding protein is an anti-4-1BB nanobody or VHH.

4. The 4-1BB-binding protein according to any one of claims 1 to 3, further comprising an Fc region of an immunoglobulin, wherein
preferably, the Fc region is an Fc region of human IgG1, human IgG2, or human IgG4.

5. The 4-1BB-binding protein according to any one of claims 1 to 4, comprising the amino acid sequence set forth in SEQ ID NO: 21 or an amino acid sequence having at least 80% or at least 90% identity thereto.

6. A CLDN18.2/4-1BB-binding protein, comprising:
a first antigen-binding domain that specifically binds to 4-1BB, and
a second antigen-binding domain that specifically binds to CLDN18.2,
wherein the first antigen-binding domain comprises an immunoglobulin single variable domain, and the immunoglobulin single variable domain comprises:
a CDR1, a CDR2, and a CDR3 from the amino acid sequence set forth in any one of SEQ ID NOs: 10 and 18-21, wherein the CDR1, CDR2, and CDR3 are defined according to the Kabat, IMGT, Chothia, AbM, or Contact numbering scheme;
preferably, the amino acid sequences of the CDR1, CDR2, and CDR3 of the immunoglobulin single variable domain are set forth in SEQ ID NOs: 11, 12, and 13 or SEQ ID NOs: 11, 12, and 22, respectively.

7. The CLDN18.2/4-1BB-binding protein according to claim 6, wherein the immunoglobulin single variable domain is engineered by humanization, affinity maturation, T cell epitope removal, reduction of antibody deamidation, and/or reduction of antibody isomerization;
preferably, heavy chain framework regions of a human germline template used in the engineering by humanization are IGHV3-64*04, IGHV3-23*03, and/or IGHV3-74*01.

8. The CLDN18.2/4-1BB-binding protein according to claim 6 or 7, wherein the amino acid sequence of the immunoglobulin single variable domain is set forth in any one of SEQ ID NOs: 10 and 18-21 or has at least 80% or at least 90% sequence identity thereto.

9. The CLDN18.2/4-1BB-binding protein according to any one of claims 6 to 8, wherein the second antigen-binding domain comprises: a heavy chain variable region (VH) and a light chain variable region (VL);
the VH comprises a HCDR1, a HCDR2, and a HCDR3 from the amino acid sequence set forth in SEQ ID NO: 63;
the VL comprises a LCDR1, a LCDR2, and a LCDR3 from the amino acid sequence set forth in SEQ ID NO: 64;
the HCDRs and LCDRs are defined according to the Kabat, IMGT, Chothia, AbM, or Contact numbering scheme;
preferably, the VH comprises a HCDR1, a HCDR2, and a HCDR3 set forth in SEQ ID NOs: 57, 58, and 59, and the VL comprises a LCDR1, a LCDR2, and a LCDR3 set forth in SEQ ID NOs: 60, 61, and 62.

10. The CLDN18.2/4-1BB-binding protein according to claim 9, wherein the VH comprises the amino acid sequence set forth in SEQ ID NO: 63 or an amino acid sequence having at least 80% or at least 90% identity thereto;
the VL comprises the amino acid sequence set forth in SEQ ID NO: 64 or an amino acid sequence having at least 80% or at least 90% identity thereto.

11. A CLDN18.2/4-1BB-binding protein, comprising:
a first antigen-binding domain that specifically binds to 4-1BB, and
a second antigen-binding domain that specifically binds to CLDN18.2,
wherein when the second antigen-binding domain does not bind to CLDN18.2, the first antigen-binding domain that specifically binds to 4-1BB does not activate 4-1BB signaling; and the CLDN18.2/4-1BB-binding protein has an enhanced effector function;
preferably, the CLDN18.2/4-1BB-binding protein comprises an Fc region for enhancing an effector function of the CLDN18.2/4-1BB-binding protein; and/or the CLDN18.2/4-1BB-binding protein comprises a third antigen-binding domain that specifically binds to CD16A;
preferably, the effector function is antibody-dependent cell-mediated cytotoxicity (ADCC), antibody-dependent cellular phagocytosis (ADCP), and/or complement-dependent cytotoxicity (CDC);
preferably, the first antigen-binding domain and the second antigen-binding domain are the first antigen-binding domain and the second antigen-binding domain defined in any one of claims 6-10.

12. A CLDN18.2/4-1BB-binding protein, comprising:
a first antigen-binding domain that specifically binds to 4-1BB,
a second antigen-binding domain that specifically binds to CLDN18.2, and
a third antigen-binding domain that specifically binds to CD16A,
wherein preferably, when the second antigen-binding domain that specifically binds to CLDN18.2 does not bind to CLDN18.2, the first antigen-binding domain that specifically binds to 4-1BB is not capable of activating 4-1BB signaling;
preferably, the first antigen-binding domain and the second antigen-binding domain are the first antigen-binding domain and the second antigen-binding domain defined in any one of claims 6-10.

13. The CLDN18.2/4-1BB-binding protein according to claim 12, wherein the third antigen-binding domain comprises an immunoglobulin single variable domain, and the immunoglobulin single variable domain comprises:
a CDR1, a CDR2, and a CDR3 from the amino acid sequence set forth in any one of SEQ ID NOs: 23, 24, and 35-43,
wherein the CDR1, CDR2, and CDR3 are defined according to the Kabat, IMGT, Chothia, AbM, or Contact numbering scheme;
preferably, the amino acid sequences of the CDR1, CDR2, and CDR3 of the immunoglobulin single variable domain are set forth in SEQ ID NOs: 25, 26, and 27, or SEQ ID NOs: 28, 29, and 30, or SEQ ID NOs: 28, 44, and 30, respectively.

14. The CLDN18.2/4-1BB-binding protein according to claim 13, wherein the immunoglobulin single variable domain in the third antigen-binding domain is engineered by humanization, affinity maturation, T cell epitope removal, reduction of antibody deamidation, and/or reduction of antibody isomerization;
preferably, heavy chain framework regions of a human germline template used in the engineering by humanization are derived from IGHV3-23*04 or IGHV3-20*04.

15. The CLDN18.2/4-1BB-binding protein according to any one of claims 12 to 14, wherein the amino acid sequence of the immunoglobulin single variable domain in the third antigen-binding domain is set forth in any one of SEQ ID NOs: 23, 24, and 35-43 or has at least 80% or at least 90% sequence identity thereto.

16. The CLDN18.2/4-1BB-binding protein according to any one of claims 6 to 15, further comprising an Fc region of an immunoglobulin, wherein
preferably, the Fc region is an Fc region of human IgG1, human IgG2, or human IgG4;
more preferably, the Fc region is an Fc region with an enhanced effector.

17. The CLDN18.2/4-1BB-binding protein according to claim 16, wherein when the Fc is the Fc region of human IgG1, the Fc region comprises any one of or any combination of amino acid mutations selected from the group consisting of: 239D; 239E; 239K, 241A;
262A; 264D; 264L; 264A; 264S; 265A; 265S; 265V; 296A; 301A; 332E; 239D/332E; 239D/330S/332E; 239D/330L/332E; 298A/333A/334A; 247I/339D; 2471/339Q; 280H/290S; 280H/290S/298D; 280H/290S/298V; 243L/292P/300L; 243L/292P/300L/396L; 243L/292P/300L/305I/396L; 236A/239D/332E; 326A/333A; 326W/333S; 290E/298G/299A; 290N/298G/299A; 290E/298G/299A/326E; and 290N/298G/299A/326E; the mutations are defined according to the EU numbering scheme;
preferably, the Fc region comprises any one of or any combination of amino acid mutations selected from the group consisting of: S239D; S239E; S239K, F241A; V262A; V264D; V264L; V264A; V264S; D265A; D265S; D265V; F296A; Y296A; R301A; I332E; S239D/I332E; S239D/A330S/I332E; S239D/A330L/I332E; S298A/D333A/K334A; P2471/A339D; P247I/A339Q; D280H/K290S; D280H/K290S/S298D; D280H/K290S/S298V; F243L/R292P/Y300L; F243L/R292P/Y300L/P396L; F243L/R292P/Y300L/V305I/P396L; G236A/S239D/I332E; K326A/E333A; K326W/E333S; K290E/S298G/T299A; K290N/S298G/T299A; K290E/S298G/T299A/K326E; and K290N/S298G/T299A/K326E.

18. The CLDN18.2/4-1BB-binding protein according to any one of claims 6 to 17, wherein the binding protein further comprises a linker;
preferably, the amino acid sequence of the linker is represented by (GₘSₙ)ₕ or (GGNGT)ₕ or (YGNGT)ₕ or (EPKSS)ₕ, wherein m and n are each independently selected from the group consisting of an integer of 1-8, and h is independently selected from the group consisting of an integer of 1-20;
more preferably, the linker is a linker of (G₄S)₂ or (G₄S)₃.

19. The CLDN18.2/4-1BB-binding protein according to any one of claims 6 to 18, wherein the second antigen-binding domain comprises a heavy chain and a light chain;
the amino acid sequence of the heavy chain is set forth in SEQ ID NO: 45 or has at least 80% or at least 90% sequence identity thereto;
the amino acid sequence of the light chain is set forth in SEQ ID NO: 46 or has at least 80% or at least 90% sequence identity thereto.

20. The CLDN18.2/4-1BB-binding protein according to any one of claims 6 to 19, comprising a first polypeptide chain and a second polypeptide chain, wherein the first polypeptide chain and the second polypeptide chain are selected from the group consisting of:
(1) the amino acid sequence of the first polypeptide chain is set forth in any one of SEQ ID NOs: 47-49 or has at least 80% or at least 90% sequence identity thereto, and
the amino acid sequence of the second polypeptide chain is set forth in SEQ ID NO: 46 or has at least 80% or at least 90% sequence identity thereto; and
(2) the amino acid sequence of the first polypeptide chain is set forth in any one of SEQ ID NOs: 50, 52, and 53 or has at least 80% or at least 90% sequence identity thereto, and the amino acid sequence of the second polypeptide chain is set forth in SEQ ID NO: 51 or 54 or has at least 80% or at least 90% sequence identity thereto.

21. A CD16A-binding protein, comprising an immunoglobulin single variable domain, wherein the immunoglobulin single variable domain comprises:
a CDR1, a CDR2, and a CDR3 from the amino acid sequence set forth in any one of SEQ ID NOs: 23, 24, and 35-43,
wherein the CDR1, CDR2, and CDR3 are defined according to the Kabat, IMGT, Chothia, AbM, or Contact numbering scheme;
preferably, the amino acid sequences of the CDR1, CDR2, and CDR3 of the immunoglobulin single variable domain are set forth in SEQ ID NOs: 25, 26, and 27, or SEQ ID NOs: 28, 29, and 30, or SEQ ID NOs: 28, 44, and 30, respectively.

22. The CD16A-binding protein according to claim 21, wherein the immunoglobulin single variable domain is engineered by humanization, affinity maturation, T cell epitope removal, reduction of antibody deamidation, and/or reduction of antibody isomerization; preferably, heavy chain framework regions of a human germline template used in the engineering by humanization are derived from IGHV3-23*04 or IGHV3-20*04.

23. The CD16A-binding protein according to claim 21 or 22, wherein the amino acid sequence of the immunoglobulin single variable domain is set forth in any one of SEQ ID NOs: 23, 24, and 35-43 or has at least 80% or at least 90% sequence identity thereto; preferably, the CD16A-binding protein is an anti-CD16A nanobody or VHH.

24. The CD16A-binding protein according to any one of claims 21 to 23, further comprising an Fc region of a human immunoglobulin, wherein
preferably, the Fc region is an Fc region of human IgG1, human IgG2, or human IgG4.

25. The CD16A-binding protein according to any one of claims 21 to 24, wherein the CD16A-binding protein does not specifically bind to CD16B.

26. A polynucleotide, wherein the polynucleotide encodes the CLDN18.2/4-1BB-binding protein according to any one of claims 6 to 20, the 4-1BB-binding protein according to any one of claims 1 to 5, or the CD16A-binding protein according to any one of claims 21 to 25;
preferably, the polynucleotide is a DNA or an RNA.

27. A vector, comprising the polynucleotide according to claim 26.

28. A host cell, comprising or expressing the polynucleotide according to claim 26 or the vector according to claim 27.

29. A method for preparing a CLDN18.2/4-1BB-binding protein, a 4-1BB-binding protein, or a CD16A-binding protein, comprising:
expressing the polynucleotide according to claim 26 or the vector according to claim 27 in the host cell according to claim 28 and isolating an expressed CLDN18.2/4-1BB-binding protein, 4-1BB-binding protein, or CD16A-binding protein from the host cell,
wherein optionally, the method further comprises a step of purifying the CLDN18.2/4-1BB-binding protein, the 4-1BB-binding protein, or the CD16A-binding protein.

30. A pharmaceutical composition, comprising the CLDN18.2/4-1BB-binding protein according to any one of claims 6 to 20, the 4-1BB-binding protein according to any one of claims 1 to 5, the CD16A-binding protein according to any one of claims 21 to 25, and at least one pharmaceutically acceptable excipient, diluent, or carrier.

31. A method for treating a cancer, comprising a step set forth in the following (1) or (2):
(1) administering to a subject in need thereof a therapeutically effective amount of the CLDN18.2/4-1BB-binding protein according to any one of claims 6 to 20 or the pharmaceutical composition according to claim 30, wherein preferably, the cancer is CLDN18.2-positive;
or
(2) administering to a subject in need thereof a therapeutically effective amount of the 4-1BB-binding protein according to any one of claims 1 to 5, the CD16A-binding protein according to any one of claims 21 to 25, the polynucleotide according to claim 26, the vector according to claim 27, or the pharmaceutical composition according to claim 30.

32. The method according to claim 31, wherein the cancer is selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell cancer, and hematological cancer, and any combination thereof.

33. Use set forth in the following (1) or (2):
(1) use of the CLDN18.2/4-1BB-binding protein according to any one of claims 6 to 20, the polynucleotide according to claim 26, or the vector according to claim 27 for the manufacture of a medicament for treating a cancer, wherein preferably, the cancer is CLDN18.2-positive;
or
(2) use of the 4-1BB-binding protein according to any one of claims 1 to 5 or the CD16A-binding protein according to any one of claims 21 to 25, the polynucleotide according to claim 26, or the vector according to claim 27 for the manufacture of a medicament for treating a cancer.

34. The use according to claim 33, wherein the cancer is selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell cancer, and hematological cancer, and any combination thereof.
